(19) Europäisches Patentamt
European Patent Office
Office européen des brevets

(11) Veröffentlichungsnummer: **0 373 574 B1**

(12) # EUROPÄISCHE PATENTSCHRIFT

(45) Veröffentlichungstag der Patentschrift: **14.09.94**

(51) Int. Cl.5: **C09D 11/00**, C09D 11/10, B41M 1/42, G03C 7/00

(21) Anmeldenummer: **89122863.7**

(22) Anmeldetag: **11.12.89**

(54) **Tinten, insbesondere für den Tintenstrahldruck.**

(30) Priorität: **14.12.88 CH 4624/88**
**09.06.89 CH 2174/89**

(43) Veröffentlichungstag der Anmeldung:
**20.06.90 Patentblatt 90/25**

(45) Bekanntmachung des Hinweises auf die Patenterteilung:
**14.09.94 Patentblatt 94/37**

(84) Benannte Vertragsstaaten:
**BE DE ES FR GB IT NL**

(56) Entgegenhaltungen:
**DD-A- 107 897**
**DE-A- 2 157 534**
**DE-B- 1 643 358**
**US-A- 4 192 949**

**DERWENT ACCESSION Nr. 86-247 628, Questel Telesystems (WPIL) DERWENT PUBLICATIONS LTD., London, GB; & JP-A-61 126 186**

(73) Patentinhaber: **CIBA-GEIGY AG**
**Klybeckstrasse 141**
**CH-4002 Basel (CH)**

(72) Erfinder: **Vieira, Eric, Dr.**
**Rue d'Or**
**CH-1700 Fribourg (CH)**
Erfinder: **Laver, Hugh Stephen, Dr.**
**Route de Schiffenen 12**
**CH-1700 Fribourg (CH)**

(74) Vertreter: **Zumstein, Fritz, Dr. et al**
**Patentanwälte,**
**Dr. F. Zumstein,**
**Dipl.-Ing. F. Klingseisen,**
**Bräuhausstrasse 4**
**D-80331 München (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die vorliegende Erfindung betrifft die Lichtstabilisierung von Tinten, insbesondere für Tintenstrahldruck, durch Zusatz von Hydrochinonderivaten als Stabilisator sowie neue Verbindungen und derer Verwendung als Stabilisatoren.

Das Drucken mittels Tintenstrahl ist ein Druckverfahren, das durch elektrische Signale gesteuert werden kann. Dabei wird ein feiner Strahl von Tintentröpfchen durch eine Düse auf das Aufzeichnungsmaterial gespritzt. Die Tinte ist meistens eine wässrige Lösung eines Farbstoffes. Das Aufzeichnungsmaterial soll den Farbstoff der Tinte rasch und dauerhaft aufnehmen. Meist verwendet man hierfür besonders präparierte Papiere oder Plastikfolien, die mit einer farbstoffbindenden Schicht versehen sind. Wegen der Feinheit der Düsen werden Pigmente kaum verwendet, sondern vorwiegend Farbstoffe, die im Medium des Tintenstrahls vollständig gelöst sind. Diese Farbstoffe haben allerdings generell eine geringere Lichtechtheit als die in konventionellen Druckfarben üblichen Farb-Pigmente. Als Folge davon sind die im Tintenstrahldruck hergestellten Aufzeichnungen unter Lichteinwirkung nur beschränkt lagerfähig. Bei längerer Lagerung unter Licht beginnen sie zu verblassen oder sich zu verfärben.

Um dieses Problem zu lösen, wurde beispielsweise in US 4,256,493 vorgeschlagen, der Tinte einen wasserlöslichen UV-Absorber vom Typ sulfonierter Hydroxybenzophenone zuzusetzen. Auch die Metallsalze solcher Verbindungen wurden in JP 4 6277/88 als Lichtschutzadditive für Tinten für den Tintenstrahldruck vorgeschlagen. Solche Benzophenonderivate und deren Salze besitzen jedoch den Nachteil, dass sie mit bestimmten Farbstoffen, vor allem schwarzen Farbstoffen, Verfärbungen verursachen.

Es wurde nun gefunden, dass bestimmte Hydrochinonderivate sich für die Stabilisierung von Tinten, insbesondere von Tinten für den Tintenstrahldruck, besonders eignen.

Dihydroxybenzolderivate als Zusatz für Tinten für den Tintenstrahldruck sind bereits bekannt. So beschreibt beispielsweise die JP-A-57-207659 Dihydroxybenzole, welche gegebenenfalls mit einem weiteren -OH oder $-CH_3$ substituiert sind. Die Gallussäure und 3,5-Dimethoxy-4-hydroxy-benzoesäure sind hier ebenfalls genannt. Aus der JP-A-62-106971 ist der Einsatz von Dialkylhydrochinonen, wie z.B. 2,5-Di-t.-amylhydrochinon, bekannt. Daneben ist hier auch die Verwendung des Natriumsalzes von 2-Hydroxy-4-methoxy-5-sulfobenzophenons und 2,2'-Dihydroxy-4,4'-dimethoxy-5-sulfobenzophenons beschrieben. Ferner sind N-Alkanolaminsalze der m-Digallussäure in der JP-A-58-183769 als Tintenzusätze für den Tintenstrahldruck beschrieben.

Weiterhin sind in der DE-A-3 121 711 Hydroxybenzole beschrieben, die 1, 2 oder 3 Hydroxygruppen tragen und gegebenenfalls durch 1 oder 2 -COOH oder -COO-Alkyl substituiert sind, wie zum Beispiel Tannin, Gallussäure und die Methyl-, Ethyl- oder Propylester der Gallussäure. Die genannten Verbindungen werden in Form von flüssigen Zubereitungen als Beschichtung auf das Trägermaterial gebracht, um anschliessend mit Lösungen von Uebergangsmetallsalzen farbige Komplexe zu liefern. Die Hydroxyverbindungen sind in diesem Fall also aktiv an der Farbentstehung beteiligt und dienen nicht als Stabilisatoren. Das so beschichtete Trägermaterial eignet sich für das Ink-Jet-Druckverfahren.

Es besteht jedoch weiterhin ein Bedarf nach wirksamen Lichtschutzmitteln für Tinten.

Gegenstand vorliegender Erfindung ist daher eine Tinte enthaltend als Stabilisator mindestens eine Verbindung der Formel I

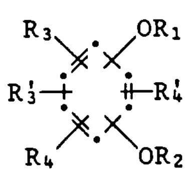 (I),

worin

$R_1$ und $R_2$ unabhängig voneinander $C_1$-$C_4$-Alkyl, welches gegebenenfalls mit einem oder 2 -OH, $-COO^{\ominus}M^{\oplus}$ und/oder $-SO_3{}^{\ominus}M^{\oplus}$ substituiert ist, $C_3$-$C_5$-Alkenyl, $C_3$-$C_5$-Alkinyl,

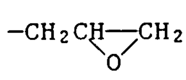,

$-CH_2CH(OH)CH_2-SO_3{}^{\ominus}M^{\oplus}$,

-CO-Alkyl($C_1$-$C_4$) bedeuten, welches gegebenenfalls mit $-COOR_5$ oder $-CO-N(R_5)(R_6)$ substituiert ist, oder,

falls $OR_1$ und $OR_2$ in ortho-Stellung zueinander stehen, $R_1$ und $R_2$ zusammen für $C_1$-$C_6$-Alkylen stehen, wobei $M^{\oplus}$ für $H^{\oplus}$, ein ein-, zwei- oder dreiwertiges Metallkation oder eine Gruppe $(R_{12}')N^{\oplus}(R_{12}'')(R_{13}')(R_{14}')$ steht, worin $R_{12}'$, $R_{12}''$, $R_{13}'$ und
$R_{14}'$ unabhängig voneinander H, $C_1$-$C_1$-Alkyl, welches gegebenenfalls mit 1 bis 3 OH substituiert oder durch ein O unterbrochen ist, Allyl, Cyclopentyl, Cyclohexyl, Phenyl, Benzyl oder Tolyl sind, oder $R_1$ auch für eine Gruppe

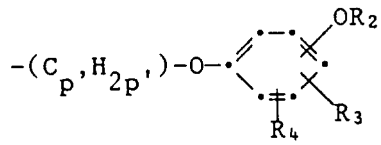

stehen kann, worin p' eine Zahl von 2 bis 6 ist,
$R_5$ und $R_6$ unabhängig voneinander H oder $C_1$-$C_4$-Alkyl, welches gegebenenfalls mit einem OH, $COOR^o$, $COO^{\ominus}M^{\oplus}$, $SO_3{}^{\ominus}M^{\oplus}$ oder $P(O)(OR^o)_2$ oder $-P(O)-(O^{\ominus}M^{\oplus})_2$ substituiert ist, bedeuten,
$R_3'$ und $R_4'$ unabhängig voneinander H, $C_1$-$C_4$-Alkyl, OH oder $C_1$-$C_4$-Alkoxy bedeuten,
$R_3$ und $R_4$ unabhängig voneinander H, Halogen, $-OR_7$, $-COOR^o$, $-COO^{\ominus}M^{\oplus}$,$-OOC-R_5$, $-CO-N(R_5)(R_6)$, $-(R_5)N-CO-R_6$, $-CO-R_5$, $-SO_3{}^{\ominus}M^{\oplus}$, $-SO_2N(R_5)(R_6)$, $-P(OR_5)_3$, $-(O)P(OR^o)_2$, $-(O)P-(O^{\ominus}M^{\oplus})_2$, $C_1$-$C_8$-Alkyl, welches gegebenenfalls mit 1 bis 7 $-OR_5$ oder $-OOC-R_5$, mit 1 oder 2 $-COOR^o$, $-COO^{\ominus}M^{\oplus}$,
$-CO-N(R_5)(R_6)$ oder mit einem oder zwei $-SO_3{}^{\ominus}M^{\oplus}$, $-SO_2N(R_5)(R_6)$,
$-(O)P(OR^o)_2$ oder $-(O)P-(O^{\ominus}M^{\oplus})_2$ substituiert ist oder $C_5$-$C_6$-Cycloalkyl oder Allyl bedeuten, wobei $M^{\oplus}$, $R_5$ und $R_6$ die angegebene Bedeutung haben und $R^o$ ein gegebenenfalls mit einem -OH substituiertes $C_1$-$C_4$-Alkyl ist, und $R_7$ ein gegebenenfalls mit 1 oder 2 -OH substituiertes $C_1$-$C_4$-Alkyl oder -CO-Alkyl($C_1$-$C_4$) bedeutet, oder
$R_3$ und $R_4$ unabhängig voneinander eine der Gruppen der Formeln II-IV bedeuten

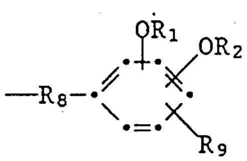 ,

(II)

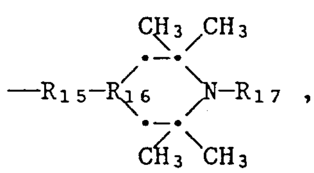 ,

(III)

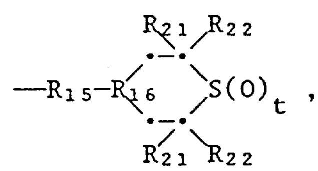 ,

(IV)

wobei
$R_8$ eine direkte Bindung oder Methylen ist, $R_9$ H, $C_1$-$C_8$-Alkyl, $-COO^{\ominus}M^{\oplus}$ oder $-SO_3{}^{\ominus}M^{\oplus}$ ist, worin $M^{\oplus}$ und $R_1$ und $R_2$ die angegebene Bedeutung haben, $R_{15}$ -CO-,

$$-(O)_g-C_pH_{2p}-CO-,$$

$-OOC-C_pH_{2p}-$, $-COO-C_pH_{2p}-$, $-O-C_pH_{2p}-$, $-O-CH_2-CH(OH)-CH_2-$ oder

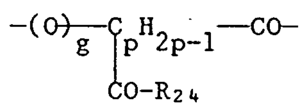

bedeutet, wobei g 0 oder 1 und p 1 bis 6 bedeuten, $R_{24}$ $-OR_5$, $-N(R_5)(R_6)$ oder eine Gruppe

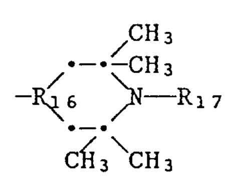

ist,
$R_{16}$ für einen der folgenden Reste steht:
-O-CH< ,

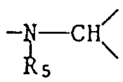 , 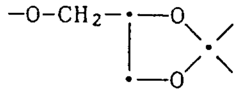 oder 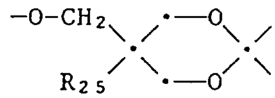 ,

wobei $R_{25}$ H oder $C_1$-$C_4$-Alkyl bedeutet,
$R_{17}$ H, $C_1$-$C_4$-Alkyl, welches gegebenenfalls mit einem -OH substituiert ist, $-CH_2$-CH(OH)-$CH_2$-OH, $C_1$-$C_8$-Alkoxy, -OH, -CO-Alkyl($C_1$-$C_4$), -CO-CH=$CH_2$, Allyl, Benzyl oder eine Gruppe

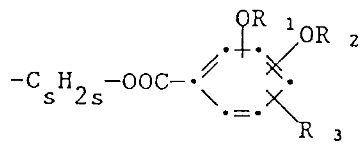

bedeutet, wobei s für die Zahl 2 oder 3 steht,
t für eine Zahl von 0 bis 2 steht, und
$R_{21}$ und $R_{22}$ unabhängig voneinander H, $C_1$-$C_4$-Alkyl oder Phenyl bedeuten.

Hervorzuheben sind Tinten, enthaltend als Stabilisator mindestens eine Verbindung der Formel I'

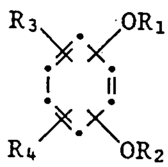 (I'),

worin
$R_1$ und $R_2$ unabhängig voneinander $C_1$-$C_4$-Alkyl, welches gegebenenfalls mit einem oder 2 -OH, $-COO^{\ominus}M^{\oplus}$ und/oder $-SO_3{}^{\ominus}M^{\oplus}$ substituiert ist, $-CH_2CH(OH)$-$CH_2$-$SO_3{}^{\ominus}M^{\oplus}$, -CO-Alkyl($C_1$-$C_4$) sind, $M^{\oplus}$ für $H^{\oplus}$, ein ein-, zwei- oder dreiwertiges Metallkation oder eine Gruppe
$(R_{12}')N^{\oplus}(R_{12}'')(R_{13}')(R_{14}')$ steht,
$R_5$ und $R_6$ unabhängig voneinander H oder $C_1$-$C_4$-Alkyl, welches gegebenenfalls mit einem OH substituiert ist, bedeuten,
$R_3$ und $R_4$ unabhängig voneinander H, Halogen, $-OR_7$, $-COOR^{\circ}$, $-OOC-R_5$, $-COO^{\ominus}M^{\oplus}$, $-CO-N(R_5)(R_6)$, $-(R_5)$-N-CO-$R_6$, $-CO-R_5$, $-SO_3{}^{\ominus}M^{\oplus}$, $-SO_2N(R_5)(R_6)$, $-P(OR_5)_3$, $-(O)P(OR^{\circ})_2$, $-(O)P-(O^{\ominus}M^{\oplus})_2$, $C_1$-$C_8$-Alkyl, welches gegebenenfalls mit 1 bis 7 $-OR_5$ oder $-OOC-R_5$, mit 1 oder 2 $-COOR^{\circ}$, $-COO^{\ominus}M^{\oplus}$, $-CO-N(R_5)(R_6)$ oder mit einem $-SO_3{}^{\ominus}M^{\oplus}$, $-SO_2N(R_5)(R_6)$, $-(O)P(OR^{\circ})_2$ oder $-(O)P-(O^{\ominus}M^{\oplus})_2$ substituiert ist, sind, bedeuten, wobei
$R^{\circ}$ ein gegebenenfalls mit einem -OH substituiertes $C_1$-$C_4$-Alkyl ist, und
$R_7$ ein gegebenenfalls mit 1 oder 2 -OH substituiertes $C_1$-$C_4$-Alkyl bedeutet, oder
$R_3$ und $R_4$ unabhängig voneinander eine Gruppe der Formel II bedeuten

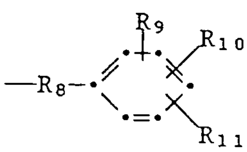 , (II)

wobei
$R_8$ eine direkte Bindung oder Methylen ist, $R_9$ H, $C_1$-$C_8$-Alkyl, $-COO^{\ominus}M^{\oplus}$ oder $-SO_3{}^{\ominus}M^{\oplus}$ ist, worin $M^{\oplus}$ die angegebene Bedeutung hat, und $R_1$ und $R_2$ die angegebene Bedeutung haben.

Ebenfalls bevorzugt sind Tinten, worin $R_1$ und $R_2$ unabhängig voneinander $C_1$-$C_4$-Alkyl, Allyl, $C_2$-$C_4$-Hydroxyalkyl, -$C_1$-$C_4$-Alkyl-$COO^{\ominus}M^{\oplus}$, $-CH_2CH(OH)CH_2-SO_3{}^{\ominus}M^{\oplus}$ oder -CO-Alkyl($C_1$-$C_4$ bedeuten, oder worin $R_1$ eine Gruppe der Formel

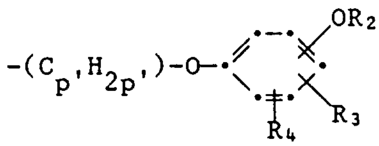

ist, und insbesondere worin $R_1$ und $R_2$ unabhängig voneinander Methyl, Ethyl, Allyl, $-CH_2CH_2-OH$, $-CH_2CH(OH)CH_3$, $CH_2-COO^{\ominus}M^{\oplus}$ oder $-CH_2CH(OH)CH_2(OH)$ bedeuten.

Von Interesse sind Tinten, worin $R_3$ und $R_4$ unabhängig voneinander H, Halogen, $-OR_7$, $-COOR^o$, $-COO^{\ominus}M^{\oplus}$, $-CO-N(R_5)(R_6)$, $-SO_3{}^{\ominus}M^{\oplus}$, $-SO_2N(R_5)(R_6)$, $C_1$-$C_8$-Alkyl, welches gegebenenfalls mit 1 oder 2 $-COOR^o$ oder $-COO^{\ominus}M^{\oplus}$ substituiert ist, oder Allyl bedeuten.

Zu erwähnen sind ferner Tinten, worin $R_3$ und/oder $R_4$ den Rest $-OR_7$ bedeuten, wobei $R_7$ für $C_1$-$C_4$-Alkyl, $C_2$-$C_4$-Hydroxyalkyl oder $-CH_2-CH(OH)CH_2-OH$ steht.

Bevorzugt sind auch jene Tinten, worin $R_1$ und $R_2$ unabhängig voneinander $C_1$-$C_4$-Alkyl oder mit Carboxy substituiertes $C_1$-$C_4$-Alkyl oder $R_1$ und $R_2$ zusammen $C_1$-$C_4$-Alkylen bedeuten, $R_3$ und $R_4$ unabhängig voneinander H, $COOR^o$, $-COO^{\ominus}M^{\oplus}$, $C_1$-$C_4$-Alkoxy, $C_1$-$C_4$-Alkyl-$COOR^o$, $C_1$-$C_4$-Alkyl-$COO^{\ominus}M^{\oplus}$, $C_1$-$C_4$-Alkyl oder $SO_3{}^{\ominus}M^{\oplus}$ und $R_3'$ und $R_4'$ unabhängig voneinander H oder $C_1$-$C_4$-Alkoxy bedeuten, sowie ferner Tinten, worin $R_1$ und $R_2$ $CH_3$, $R_3$ $-COOR^o$ oder $-COO^{\ominus}M^{\oplus}$ und $R_4$ Methoxy ist.

Etwaige Alkylreste als $C_1$-$C_4$-Alkyl bedeuten beispielsweise Methyl, Ethyl, n-Propyl, Isopropyl, n-Butyl, sek-Butyl oder t-Butyl.

Etwaige Alkylreste als $C_1$-$C_8$-Alkyl können zusätzlich zu den genannten Bedeutungen beispielsweise n-Pentyl, t-Amyl, n-Hexyl, n-Heptyl, 2-Ethylhexyl, n-Octyl oder 1,1,3,3-Tetramethylbutyl sein.

Etwaige Reste als $C_1$-$C_4$-Hydroxyalkyl bedeuten beispielsweise Hydroxyethyl, 2-Hydroxyethyl, 1-Hydroxyethyl, 3-Hydroxypropyl, 3-Hydroxybutyl oder 4-Hydroxybutyl.

Etwaige Reste als gegebenenfalls durch 1 bis 3 OH substituiertes $C_1$-$C_8$-Alkyl können zusätzlich zu den Bedeutungen von $C_1$-$C_4$-Hydroxyalkyl beispielsweise 2,3-Dihydroxypropyl, 2,2-Di(hydroxymethyl)-propyl, 6-Hydroxyhexyl, 8-Hydroxyoctyl, 1,2,4-Trihydroxy-but-2-yl, 1,2,6-Trihydroxy-hex-2-yl, 1,2,3-Trihydroxy-prop-2-yl sein.

Etwaige Reste als $C_2$-$C_6$-Alkylen bedeuten beispielsweise Ethylen, Ethyliden, Tri-, Tetra-, Penta- oder Hexamethylen, 1,2-Propylen, 2,2-Propyliden, 2,2-Butyliden, 1,2-Butylen oder 2,2-Dimethyl-1,3-propylen.

Etwaige Reste als $C_1$-$C_4$-Alkylen können zusätzlich zu den vorangehenden Bedeutungen auch Methylen sein.

Etwaige Alkylenreste welche durch 1 oder mehrere -O- oder $-N(R_5)$-unterbrochen sind, bedeuten Reste, worin zwischen 2 Heteroatomen sich mindestens 2 C-Atome befinden.

Etwaige $C_2$-$C_5$-Alkenylreste können beispielsweise Vinyl, Allyl, Methallyl, Propen-1-yl, Buten-1-yl oder Penten-1-yl sein.

$R_3$ und $R_4$ als gegebenenfalls substituiertes $C_2$-$C_5$-Alkenyl können beispielsweise der folgenden Formel entsprechen

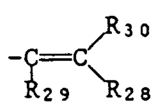

worin $R_{28}$ H oder $CH_3$ ist, $R_{29}$ und $R_{30}$ unabhängig voneinander $-COOR^o$, $-COO^{\ominus}M^{\oplus}$, $-CO-CH_3$, $-CON(R_5)(R_6)$ oder $C{\equiv}N$ bedeuten.

$R_3$ und $R_4$ als Halogen können beispielsweise Cl, Br oder J sein.

Etwaige $C_5$-$C_6$-Cycloalkylreste, resp. $C_5$-$C_7$-Cycloalkylenreste können beispielsweise Cyclopentyl, Cyclopentenyl, Cyclohexyl, Cyclohexenyl oder Cycloheptyl, und bevorzugt Cyclohexyl sein.

Etwaige ein-, zwei- und dreiwertige Metallkationen können z.B. $Na^+$ und $K^+$ und insbesondere $Li^+$ sein, ferner

$Mg^{2+}_{1/2}$, $Ca^{2+}_{1/2}$, $Zn^{2+}_{1/2}$,
$Al^{3+}_{1/3}$, $Fe^{3+}_{1/3}$, $Cu^{2+}_{1/2}$, $Cr^{3+}_{1/3}$, $Mn^{2+}_{1/2}$ und $Ni^{2+}_{1/2}$.

In $M^{\oplus}$ können die Reste $R_{12}'$, $R_{12}''$, $R_{13}'$ und $R_{14}'$ unabhängig voneinander H, $C_1$-$C_4$-Alkyl, welches mit 1 bis 3 OH substituiert sein kann oder gegebenenfalls mit einem O unterbrochen ist, Allyl, Cyclopentyl, Cyclohexyl, Phenyl, Benzyl oder Tolyl sein.

$M^{\oplus}$ als

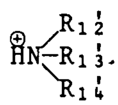

kann beipielsweise $N^{\oplus}H_4$, $HN^{\oplus}(CH_3)_3$, $HN^{\oplus}(CH_2CH_3)_3$, $H_3N^{\oplus}CH_2CH_2OH$, $H_2N^{\oplus}(CH_2CH_2OH)_2$ oder $HN^{\oplus}(CH_2CH_2OH)_3$ bedeuten.

$X^{\ominus}$ kann beispielsweise $F^-$, $Cl^-$, $Br^-$, $J^-$, $R_o$-$SO_3^-$, $C_1$-$C_4$-Alkyl-$OSO_3^-$, $CN^-$, $SCN^-$, $OH^-$, $BF_4^-$, $PF_6^-$, $HCO_3^-$, $H_2PO_3^-$, $H_2PO_4^-$, $R_{25}$-$COO^-$, 1/2 $CO_3^{2-}$, 1/2 $SO_4^{2-}$, 1/2 $HPO_3^{2-}$, 1/2 $HPO_4^{2-}$, 1/2 $R_{27}$-$(COO^-)_2$, 1/3 $PO_4^{3-}$, 1/3 $PO_3^{3-}$ oder 1/3

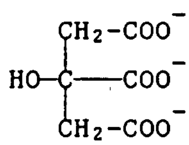

sein.

$R_o$ bedeutet gegebenenfalls mit OH substituiertes $C_1$-$C_4$-Alkyl. $X^{\ominus}$ ist bevorzugt Halogenid ($F^-$, $Cl^-$, $Br^-$, $J^-$), $C_1$-$C_4$-Alkyl-$COO^-$, $C_1$-$C_4$-Alkyl-$OSO_3^-$, $CF_3$-$SO_3^-$, $CH_3$-$SO_3^-$ oder

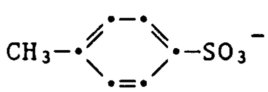.

$X^{\ominus}$ ist besonders bevorzugt $Cl^-$, $CH_3$-$OSO_3^-$, $CH_3CH_2$-$OSO_3^-$, $CH_3SO_3^-$,

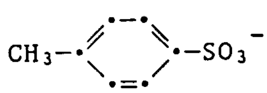

oder $CH_3$-$COO^-$.

Die Verbindungen der Formel I sind zum Teil bekannt und teilweise im Handel erhältlich oder können nach bekannten Methoden hergestellt werden.

Die erfindungsgemässen Tinten zeichnen sich durch unerwartete Qualitätsverbesserung aus. Sie werden bevorzugt im Tintenstrahldruck eingesetzt. Insbesondere ist ihre Beständigkeit gegen Oxidation durch Licht- und Wärmeeinflüsse zu erwahnen. In dieser Hinsicht sind sie entsprechenden Aufzeichnungsmaterialien überlegen, welche Polyhydroxybenzolderivate als Stabilisatoren enthalten. Diese Stabilisatoren sind nicht wirksam genug, um Verfärbungen des Tintenstrahldrucks weitgehend unterdrücken zu können. Bei den erfindungsgemässen Tinten dagegen treten solche Verfärbungen praktisch nicht auf. Die Verbindungen der Formel I können nicht nur in Tintenstrahldrucktinten eingesetzt werden, sondern auch in aller Art von Tinten wie zum Beispiel für Filzstifte, Stempelkissen, Füllfedern, Federschreiber (Pen Plotters), Offsetdruck, Buchdruck, Flexodruck und Tiefdruck, wie auch in Farbbändern für den Punktmatrix- und Schönschreiber-Druck.

Die erfindungsgemässen Tinten enthalten mindestens einen Farbstoff. Hierbei spielt es keine Rolle, welcher Art die Tinte und der in ihr gelöste Farbstoff ist und welche Art von Druckvorrichtung (printer)

verwendet wird.

Bei den heute verwendeten Druckern unterscheidet man solche mit kontinuierlichem Tintenstrahl und "Drop-on-demand"-Drucker, insbesondere bubble-jet-Drucker. Für alle diese apparativen Verfahren lässt sich die erfindungsgemässe Tinte verwenden und zwar zum Bedrucken von Tintenstrahldruckpapieren und -folien.

Die Tinten sind meistens wässrige Tinten, sie können aber auch Lösungen des Farbstoffes in einem organischen Lösungsmittel oder in einem geschmolzenen Wachs sein. Wässrige Tinten enthalten meist noch wasserlösliche Lösungsmittel, wie z.B. Mono-, Di-, Tri- oder höhere Ethylenglykole, Propylenglykol, Butandiol-1,4, oder Ether solcher Glykole, Thiodiglykol, Glycerin und dessen Ether und Ester, Polyglycerin, Mono-, Di- und Triethanolamin, Propanolamin, Dimethylformamid, Dimethylsulfoxid, Dimethylacetamid, N-Methylpyrrolidon, 1,3-Dimethylimidazolidon, Methanol, Ethanol, Isopropanol, n-Propanol, Diacetonalkohol, Aceton, Methyl-ethyl-keton oder Propylencarbonat.

Wässrige Tinten enthalten wasserlösliche Farbstoffe, wie sie auch für das Färben von natürlichen Fasern bekannt sind. Dies können z.B. Monoazo-, Disazo- oder Polyazofarbstoffe, Reaktivfarbstoffe, Triphenylmethanfarbstoffe, Xanthenfarbstoffe oder Phthalocyaninfarbstoffe sein. Beispiele hierfür sind Food Black 2, C.I. Direct Black 19, C.I. Sulphur Black 1, Acid Red 35, Acid Yellow 23 und Kupfer-Phthalocyanine, ferner Direct Black 38, Direct Black 168, Acid Red 249, Direct Red 227, Direct Yellow 86, Acid Blue 9, Direct Blue 86 und Direct Blue 199, Acid Red 14, Acid Red 52, Reactive Red 40, Direct Yellow 107, Direct Black 154 und Acid Red 94.

Die erfindungsgemässen Tinten enthalten vorzugsweise mindestens einen wasserlöslichen Azofarbstoff.

Wässrige Tinten können auch verschiedene Zusätze in kleineren Mengen enthalten, wie z.B. Bindemittel, Tenside, Biocide, Korrosionsinhibitoren, Sequestriermittel, pH-Puffer oder Leitfähigkeitszusätze. Sie können auch weitere wasserlösliche UV-Absorber oder sonstige wasserlösliche Lichtschutzmittel enthalten. Im allgemeinen genügt jedoch die erfindungsgemässe Zugabe eines Stabilisators der Formel I zur Tinte.

Wenn die Tinte eine nicht-wässrige Tinte ist, so stellt sie eine Lösung des Farbstoffes in einem organischen Lösungsmittel oder Lösungsmittelgemisch dar. Beispiele für hierfür verwendete Lösungsmittel sind Alkylcarbitole, Alkylcellosolven, Dialkylformamide, Dialkylacetamide, Alkohole, insbesondere Alkohole mit 1-4 C-Atomen, Aceton, Methyl-ethyl-keton. Diethylketon, Methyl-isobutylketon, Di-isopropylketon, Dibutylketon, Dioxan, Ethylbutyrat, Ethyl-isovalerat, Diethyl-malonat, Diethyl-succinat, Methyl-pelargonat, Butylacetat, Triethylphosphat, Ethylglykol-acetat, Toluol, Xylol, Tetralin, Benzin-Fraktionen. Beispiele für feste Wachse als Lösungsmittel, die als Tinte erst erhitzt werden müssen, sind Stearin- oder Palmitinsäure.

Solche Tinten auf Lösungsmittel-Basis enthalten darin lösliche Farbstoffe wie z.B. Solvent Rot, Solvent Gelb., Solvent Orange, Solvent Blau, Solvent Grün, Solvent Violett, Solvent Braun oder Solvent Schwarz. Auch solche Tinten können noch weitere Additive enthalten, wie sie oben für wässrige Tinten aufgeführt sind.

Die erfindungsgemässen Tinten enthalten vorzugsweise 0,01-50 Gew.-%, insbesondere 0,1-20 Gew.-%, einer Verbindung der Formel I bzw. I',

Ein besonderes Problem bei der Lichtechtheit von Tintenstrahldrucken kann auftreten, wenn eine Tinte über eine andere gespritzt worden ist. Häufig ist die Lichtechtheit der gedruckten Mischfarbe geringer als jene der einzelnen Tinten. Dieses Problem tritt besonders bei Verwendung von Cu-Phthalocyaninfarbstoffen wie Direct Blue 86 und Direct Blue 199 in Kombination mit Azofarbstoffen wie Acid Red 35, Acid Red 249 und Direct Red 227 auf. Durch Verwendung der Stabilisatoren der Formel I lässt sich dieser unerwünschte Effekt weitgehend unterdrücken.

Die Verbindungen der Formel I bzw. I' in den erfindungsgemässen Tinten sind zum Teil neu und daher ebenfalls Gegenstand vorliegender Erfindung.

Die Erfindung betrifft daher auch neue Verbindungen der Formel I°

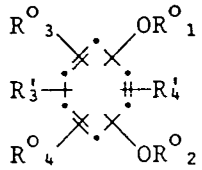

$(I^o)$,

worin

$R^o_1$ und $R^o_2$ unabhängig voneinander $C_1$-$C_4$-Alkyl, welches gegebenenfalls mit einem oder 2 -OH, $-COO^{\ominus}M^{\oplus}$ und/oder $-SO_3{}^{\ominus}M^{\oplus}$ substituiert ist, $C_3$-$C_5$-Alkenyl, $C_3$-$C_5$-Alkinyl,

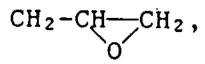

$-CH_2CH(OH)CH_2-SO_3^{\ominus}M^{\oplus}$ oder -CO-Alkyl($C_1$-$C_4$) bedeuten, welches gegebenenfalls mit $-COOR_5$ oder -CO-N($R_5$)($R_6$) substituiert ist, oder, falls $OR^o_1$ und $OR^o_2$ in ortho-Stellung zueinander stehen, $R^o_1$ und $R^o_2$ zusammen für $C_1$-$C_6$-Alkylen stehen, wobei

$M^{\oplus}$ fur $H^{\oplus}$, ein Alkalimetallion oder eine Gruppe

$(R'_{12})N^{\oplus}(R_{12}'')(R_{13}')(R_{14}')$ steht, worin $R_{12}'$, $R_{12}''$, $R_{13}'$ und $R_{14}'$ unabhängig voneinander H, $C_1$-$C_1$-Alkyl, welches gegebenenfalls mit 1 bis 3 OH substituiert oder durch ein O unterbrochen ist, Allyl, Cyclopentyl, Cyclohexyl, Phenyl, Benzyl oder Tolyl sind, oder $R^o_1$ auch für eine Gruppe

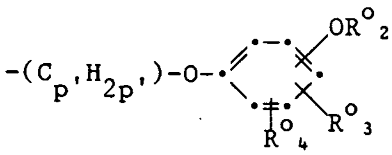

stehen kann, worin p' eine Zahl von 2 bis 6 ist,

$R_5$ und $R_6$ unabhängig voneinander H oder $C_1$-$C_4$-Alkyl, welches gegebenenfalls mit einem OH, $COOR^o$, $COO^{\ominus}M^{\oplus}$, $SO_3^{\ominus}M^{\oplus}$, $P(O)(OR^o)_2$ oder $-P(O)-(O^{\ominus}M^{\oplus})_2$ substituiert ist, bedeuten,

$R_3'$ und $R_4'$ unabhängig voneinander H, $C_1$-$C_4$-Alkyl, OH oder $C_1$-$C_4$-Alkoxy bedeuten,

$R^o_3$ und $R^o_4$ unabhängig voneinander H, Halogen, $-OR_7$, $-COOR^o$, $-COO^{\ominus}M^{\oplus}$, $OOC-R_5$, $-CO-N(R_5)(R_6)$, $-(R_5)-N-CO-R_6$, $-CO-R_5$, $-SO_3^{\ominus}M^{\oplus}$, $-SO_2N(R_5)(R_6)$, $-P(OR_5)_3$, $-(O)P(OR^o)_2$, $-(O)P-(O^{\ominus}M^{\oplus})_2$, $C_1$-$C_8$-Alkyl, welches gegebenenfalls mit 1 bis 7 $-OR_5$ oder $-OOC-R_5$, mit 1 oder 2 $-COOR^o$, $-COO^{\ominus}M^{\oplus}$, $CO-N(R_5)R_6$) oder mit einem $-SO_3^{\ominus}M^{\oplus}$, $-SO_2N(R_5)(R_6)$, $-(O)P(OR^o)_2$ oder $-(O)P-(O^{\ominus}M^{\oplus})_2$ substituiert ist, oder Allyl, $C_5$-$C_6$-Cycloalkyl bedeuten, wobei $M^{\oplus}$, $R_5$ und $R_6$ die angegebene Bedeutung haben und

$R^o$ ein gegebenenfalls mit einem -OH substituiertes $C_1$-$C_4$-Alkyl ist, und $R_7$ ein gegebenenfalls mit 1 oder 2 -OH substituiertes $C_1$-$C_4$-Alkyl oder -CO-Alkyl($C_1$-$C_4$) bedeutet, oder

$R^o_3$ und $R^o_4$ unabhängig voneinander eine der Gruppen der Formeln II-IV bedeuten

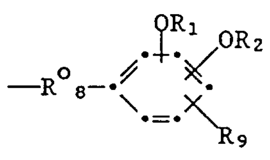 ,

(II°)

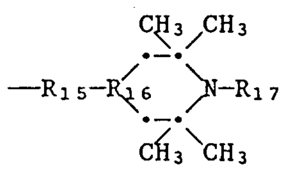 ,

(III)

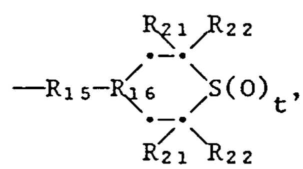

(IV)

wobei

$R^o_8$ eine direkte Bindung, oder Methylen ist, $R_9$ Wasserstoff, $C_1$-$C_8$-Alkyl, $-COO^{\ominus}M^{\oplus}$ oder $-SO_3^{\ominus}M^{\oplus}$ ist,

$R_{15}$ -CO-,

$$-(O)_g-C_pH_{2p}-CO-,$$

$-OOC-C_pH_{2p}-$, $-COO-C_pH_{2p}-$, $-O-C_pH_{2p}-$, $-O-CH_2-CH(OH)-CH_2-$ oder

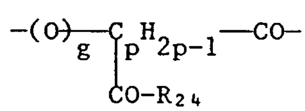

bedeutet, wobei g 0 oder 1 und p 1 bis 6 bedeuten,

$R_{24}$ $-OR_5$, $-N(R_5)(R_6)$ oder eine Gruppe

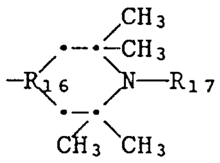

ist,
$R_{16}$ für einen der folgenden Reste steht: -O-CH< ,

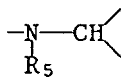 , 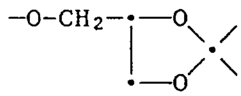 oder 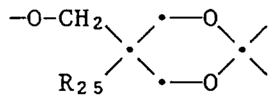 ,

wobei $R_{25}$ H oder $C_1$-$C_4$-Alkyl bedeutet,
$R_{17}$ H, $C_1$-$C_4$-Alkyl, welches gegebenenfalls mit einem -OH substituiert ist, $-CH_2-CH(OH)-CH_2-OH$, $C_1$-$C_8$-Alkoxy, -OH, -CO-Alkyl($C_1$-$C_4$), $-CO-CH=CH_2$, Allyl, Benzyl oder eine Gruppe

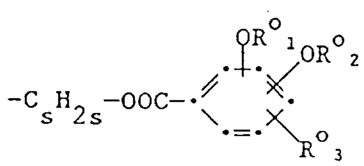

bedeutet, wobei s für die Zahl 2 oder 3 steht,
t für eine Zahl von 0 bis 2 steht, und
$R_{21}$ und $R_{22}$ unabhängig voneinander H, $C_1$-$C_4$-Alkyl oder Phenyl bedeuten,
ausgenommen die folgenden Verbindungen:

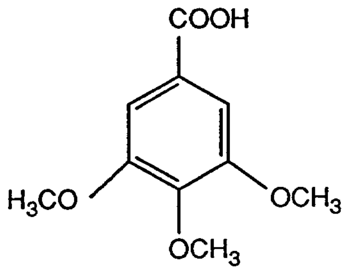 ; 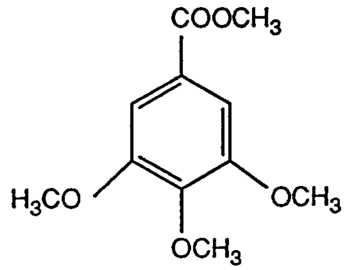 ; 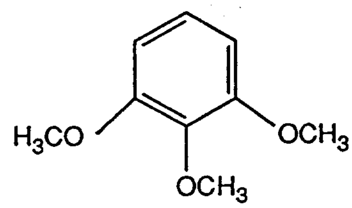 ;

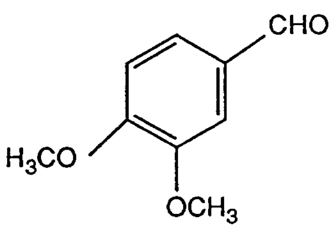 ; 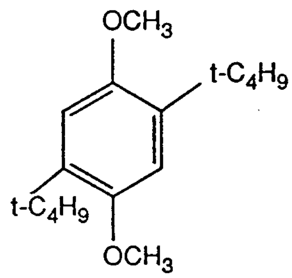 ; 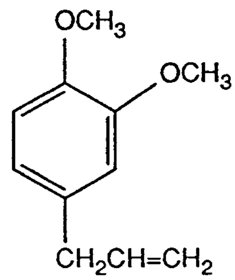 ;

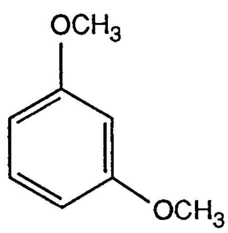 ; 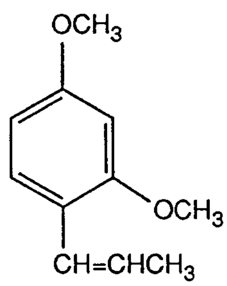 ; 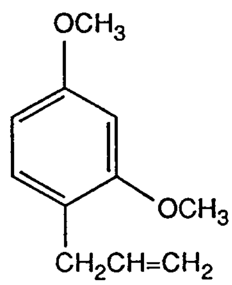 ;

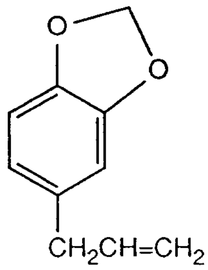 ; 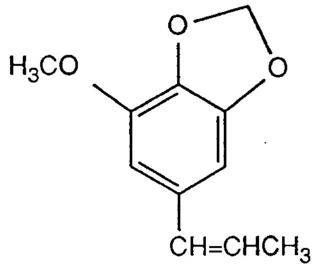 ; 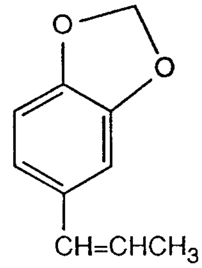 ;

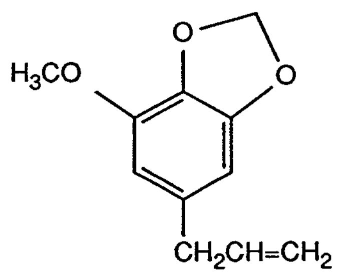 ; 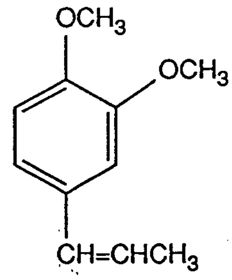 ; 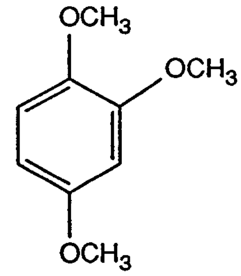 ;

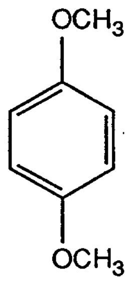 ; 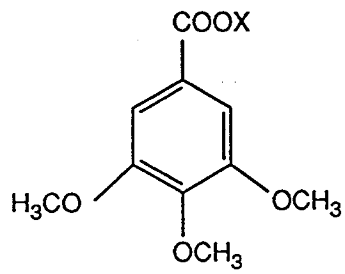 ,

wobei X Na, K, $NH_4$, Ca oder $HN(C_2H_5)_3$ ist;

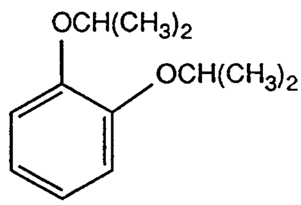 .

Die bereits zuvor unter der Formel I bzw. I' gegebenen beispielhaften Bedeutungsmöglichkeiten für $R^o{}_1$, $R^o{}_2$, $R^o{}_3$ und $R^o{}_4$ gelten hier auch.

Bevorzugt sind Verbindungen der Formel $I^o$, worin $R^o{}_1$ und $R^o{}_2$ unabhängig voneinander $C_1$-$C_4$-Alkyl, welches mit einem -OH substituiert ist, $-CH_2-CH(OH)CH_2-SO_3{}^{\ominus}M^{\oplus}$, $-C_1$-$C_4$-Alkyl-$COO^{\ominus}M^{\oplus}$ oder -CO-Alkyl-($C_1$-$C_4$), welches gegebenenfalls mit $-COOR^0$, $-COO^{\ominus}M^{\oplus}$, $-CO-N(R_5)(R_6)$ oder mit 1 oder 2 OH substituiert ist, bedeuten.

Weiterhin von Interesse sind Verbindungen, worin $R^o{}_3$ und/oder $R^o{}_4$ den Rest $-OR_7$ bedeuten, wobei $R_7$ für $C_1$-$C_4$-Alkyl, $C_2$-$C_4$-Hydroxyalkyl oder $-CH_2-CH(OH)CH_2-OH$ steht.

Bevorzugt sind auch Verbindungen, worin $R^o{}_4$ für Wasserstofff steht und $R^o{}_3$ $-SO_3{}^{\ominus}M^{\oplus}$, $-COOR^0$, $-COO^{\ominus}M^{\oplus}$ oder $-CO-NHR_5$ bedeutet.

Die neuen Verbindungen der Formel $I^o$ können, wie bereits beschrieben, nach an sich bekannnten Methoden hergestellt werden.

Die neuen Verbindungen der Formel $I^o$ werden als Stabilisatoren für organische Materialien, insbesondere gegen deren Schädigung durch Licht, verwendet. Die zu stabilisierenden Materialien können z.B. Oele, Fette, Gelatinen, Wachse, Kosmetika, Farbstoffe oder Polymere sein.

Bevorzugt ist die Verwendung von Verbindungen der Formel $I^o$ als Stabilisatoren für organische Polymere und für farbstoffhaltige Gelatineschichten in photographischen Materialien.

Einen weiteren Gegenstand der Erfindung bilden Zusammensetzungen, welche ein organisches Material und als Stabilisator mindestens eine Verbindung der Formel $I^o$ enthalten.

Beispiele von Polymeren, die mit den erfindungsgemässen Verbindungen der Formel $I^o$ vorteilhaft stabilisiert werden können, sind:

1. Polymere von Mono- und Diolefinen, beispielsweise Polypropylen, Polyisobutylen, Polybuten-1, Polymethylpenten-1, Polyisopren oder Polybutadien sowie Polymerisate von Cycloolefinen wie z.B. von Cyclopenten oder Norbornen; ferner Polyethylefl (das gegebenenfalls vernetzt sein kann), z.B. Polyethylen hoher Dichte (HDPE), Polyethylen niederer Dichte (LDPE), lineares Polyethylen niederer Dichte

(LLDPE).

2. Mischungen der unter 1) genannten Polymeren, z.B. Mischungen von Polypropylen mit Polyisobutylen, Polypropylen mit Polyethylen (z.B. PP/HDPE, PP/LDPE) und Mischungen verschiedener Polyethylentypen (z.B. LDPE/HDPE).

3. Copolymere von Mono- und Diolefinen untereinander oder mit anderen Vinylmonomeren, wie z.B. Ethylen-Propylen-Copolymere, lineares Polyethylen niederer Dichte (LLDPE) und Mischungen desselben mit Polyethylen niederer Dichte (LDPE), Propylen-Buten-1-Copolymere, Propylen-Isobutylen-Copolymere, Ethylen-Buten-1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren-Copolymere, Ethylen-Alkylacrylat-Copolymere, Ethylen-Alkylmethacrylat-Copolymere, Ethylen-Vinylacetat-Copolymere oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen; ferner Mischungen solcher Copolymere untereinander und mit unter 1) genannten Polymeren, z.B. Polypropylen/Ethylen-Propylen-Copolymere, LDPE/Ethylen-Vinylacetat-Copolymere, LDPE/Ethylen-Acrylsäure-Copolymere, LLDPE/Ethylen-Vinylacetat-Copolymere und LLDPE/Ethylen-Acrylsäure-Copolymere.

3a. Kohlenwasserstoffharze (z.B. $C_5$-$C_9$) inklusive hydrierte Modifikationen davon (z.B. Klebrigmacherharze).

4. Polystyrol, Poly-(p-methylstyrol), Poly-(α-methylstyrol).

5. Copolymere von Styrol oder α-Methylstyrol mit Dienen oder Acrylderivaten, wie z.B. Styrol-Butadien, Styrol-Acrylnitril, Styrol-Alkylmethacrylat, Styrol-Butadien-Alkylacrylat, Styrol-Maleinsäureanhydrid, Styrol-Acrylnitril-Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethyle/Propylen-Styrol.

6. Pfropfcopolymere von Styrol oder α-Methylstyrol, wie z.B. Styrol auf Polybutadien, Styrol auf Polybutadien-Styrol- oder Polybutadien-Acrylnitril-Copolymere, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie sie z.B. als sogenannte ABS-, MBS-, ASA- oder AES-Polymere bekannt sind.

7. Halogenhaltige Polymere, wie z.B. Polychloropren, Chlorkautschuk, chloriertes oder chlorsulfoniertes Polyethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.

8. Polymere, die sich von α,β-ungesättigten Säuren und deren Derivaten ableiten, wie Polyacrylate und Polymethacrylate, Polyacrylamide und Polyacrylnitrile.

9. Copolymere der unter 8) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.

10. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin; sowie deren Copolymere mit in Punkt 1 genannten Olefinen.

11. Homo- und Copolymere von cyclischen Ethern, wie Polyalkylenglykole, Polyethylenoxyd, Polypropylenoxyd oder deren Copolymere mit Bisglycidylethern.

12. Polyacetale, wie Polyoxymethylen, sowie solche Polyoxymethylene, die Comonomere, wie z.B. Ethylenoxid, enthalten; Polyacetale, die mit termoplastischen Polyurethanen, Acrylaten oder MBS modifiziert sind.

13. Polyphenylenoxide und -sulfide und deren Mischungen mit Styrolpolymeren oder Polyamiden.

14. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.

15. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, 6/10,

6/9, 6/12, 4/6, Polyamid 11, Polyamid 12, aromatische Polyamide ausgehend von m-Xylol, Diamin und Adipinsäure; Polyamide, hergestellt aus Hexamethylendiamin und Iso- und/oder Terephthalsäure und gegebenenfalls einem Elastomer als Modifikator, z.B. Poly-2,4,4-trimethylhexamethylenterephthalamid, Poly-m-phenylen-isophthalamid. Block-Copolymere der vorstehend genannten Polyamide mit Polyolefinen, Olefin-Copolymeren, Ionomeren oder chemisch gebundenen oder gepfropften Elastomeren; oder mit Polyethern, wie z.B. mit Polyethylenglykol, Polypropylenglykol oder Polytetramethylenglykol. Ferner mit EPDM oder ABS modifizierte Polyamide oder Copolyamide; sowie während der Verarbeitung kondensierte Polyamide ("RIM-Polyamidsysteme").

16. Polyharnstoffe, Polyimide, Polyamid-imide und Polybenzimidazole.

17. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester.

18. Polycarbonate und Polyestercarbonate.

19. Polysulfone, Polyethersulfone und Polyetherketone.

20. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.

21. Trocknende und nicht-trocknende Alkydharze.

22. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.

23. Vernetzbare Acrylharze, die sich von substituierten Acrylsäureestern ableiten, wie z.B. von Epoxyacrylaten, Urethan-acrylaten oder Polyester-acrylaten.

24. Alkydharze, Polyesterharze und Acrylatharze, die mit Melaminharzen, Harnstoffharzen, Polyisocyanaten oder Epoxidharzen vernetzt sind.

25. Vernetzte Epoxidharze, die sich von Polyepoxiden ableiten, z.B. von Bis-glycidylethern oder von cycloaliphatischen Diepoxiden.

26. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose; sowie Kolophoniumharze und Derivate.

27. Mischungen (Polyblends) der vorgenannten Polymeren, wie z.B. PP/EPDM, Polyamid/EPDM oder ABS, PVC/EVA, PVC/ABS, PVC/MBS, PC/ABS, PBTP/ABS, PC/ASA, PC/PBT, PVC/CPE, PVC/Acrylate, POM/thermoplastisches PUR, PC/thermoplastisches PUR, POM/Acrylat, POM/MBS, PPO/HIPS, PPO/PA 6.6 und Copolymere, PA/HDPE, PA/PP, PA/PPO.

28. Natürliche und synthetische organische Stoffe, die reine monomere Verbindungen oder Mischungen von solchen darstellen, beispielsweise Mineralöle, tierische oder pflanzliche Fette, Oele und Wachse, oder Oele, Wachse und Fette auf Basis synthetischer Ester (z.B. Phthalate, Adipate, Phosphate oder Trimellitate), sowie Abmischungen synthetischer Ester mit Mineralölen in beliebigen Gewichtsverhältnissen, wie sie z.B. als Spinnpräparationen Anwendung finden, sowie deren wässrige Emulsionen.

29. Wässrige Emulsionen natürlicher oder synthetischer Kautschuke, wie z.B. Naturkautschuk-Latex oder Latices von carboxylierten Styrol-Butadien-Copolymeren.

Im Hinblick auf ihre Verwendung in Gelatine, ist festzustellen, dass die erfindungsgemässen Stabilisatoren in sämtlichen photographischen Materialien vorliegen können, die einen Farbstoff oder Farbstoffvorläufer, z.B. einen Farbkuppler, in Gelatineschichten aufweisen, wie photographische Silberfarbbleich-, Chromogen- und Transfermaterialien.

Die erfindungsgemässen Stabilisatoren werden den Polymeren zweckmässig in einer Konzentration von 0,01-10 Gew.-%, berechnet auf das zu stabilisierende Material, zugesetzt. Vorzugsweise werden 0,05 bis 5,0 Gew.-%, besonders bevorzugt 0,1 bis 2,0 Gew.-% der Verbindungen der Formel $I^o$, berechnet auf das zu stabilisierende Material, in dieses eingearbeitet.

Die Einarbeitung kann beispielsweise durch Einmischen der erfindungsgemässen Stabilisatoren und gegebenenfalls weiterer Additive nach den in der Technik üblichen Methoden, vor oder während der Formgebung, oder auch durch Aufbringen der gelösten oder dispergierten Verbindungen auf das Polymere, gegebenenfalls unter nachträglichem Verdunsten des Lösungsmittels, erfolgen. Die erfindungsgemässen Stabilisatoren können auch in Form eines Masterbatches, der den Stabilisator beispielsweise in einer Konzentration von 2,5 bis 25 Gew.-% enthält, den zu stabilisierenden Kunststoffen zugesetzt werden.

Die Verbindungen der Formel $I^o$ können auch vor oder während der Polymerisation oder der Vernetzungsreaktion zugefügt werden. Man erhält so direkt stabilisierte Polymere.

Die so stabilisierten Materialien können in verschiedenster Form angewendet werden, z.B. als Folien, Fasern, Bändchen, Formmassen, Profile oder als Bindemittel für Lacke, Klebstoffe oder Kitte.

In der Praxis können die erfindungsgemässen Stabilisatoren zusammen mit anderen Stabilisatoren eingesetzt werden.

Als weitere Additive, mit denen zusammen die erfindungsgemäss verwendeten Stabilisatoren eingesetzt werden können, sind beispielsweise zu nennen:

1. Antioxidantien

1.1. Alkylierte Monophenole, z.B. 2,6-Di-tert.butyl-4-methylphenol, 2-Tert.butyl-4,6-dimethylphenol, 2,6-Di-tert.butyl-4-ethylphenol, 2,6-Di-tert.butyl-4-n-butylphenol, 2,6-Di-tert.butyl-4-i-butylphenol, 2,6-Di-cyclopentyl-4-methylphenol, 2-(α-Methylcyclohexyl)-4,6-dimethylphenol, 2,6-Di-octadecyl-4-methylphenol, 2,4,6-Tri-cyclohexylphenol, 2,6-Di-tert.butyl-4-methoxymethylphenol, 2,6-Di-nonyl-4-methylphenol.

1.2. Alkylierte Hydrochinone, z.B. 2,6-Di-tert.butyl-4-methoxyphenol, 2,5-Di-tert.butyl-hydrochinon, 2,5-Di-tert.amyl-hydrochinon, 2,6-Di-phenyl-4-octadecyloxyphenol.

1.3. Hydroxylierte Thiodiphenylether, z.B. 2,2'-Thio-bis-(6-tert.-butyl-4-methylphenol), 2,2'-Thio-bis-(4-octylphenol), 4,4'-Thio-bis-(6-tert.butyl-3-methylphenol), 4,4'-Thio-bis-(6-tert.butyl-2-methylphenol).

1.4. Alkyliden-Bisphenole, z.B. 2,2'-Methylen-bis-(6-tert.butyl-4-methylphenol), 2,2'-Methylen-bis-(6-tert.butyl-4-ethylphenol), 2,2'-Methylen-bis-[4-methyl-6-(α-methylcyclohexyl)-phenol], 2,2'-Methylen-bis-(4-methyl-6-cyclohexylphenol), 2,2'-Methylen-bis-(6-nonyl-4-methylphenol), 2,2'-Methylen-bis-(4,6-di-tert.butylphenol), 2,2'-Ethyliden-bis-(4,6-di-tert.butylphenol), 2,2'-Ethyliden-bis-(6-tert.butyl-4-isobutylphenol), 2,2'-Methylen-bis-[6-(α-methylbenzyl)-4-nonylphenol], 2,2'-Methylen-bis-[6-(α,α-dimethylbenzyl)-4-nonylphenol], 4,4'-Methylen-bis-(2,6-di-tert.butylphenol), 4,4'-Methylen-bis-(6-tert.butyl-2-methylphenol), 1,1-Bis-(5-tert.butyl-4-hydroxy-2-methylphenyl)-butan, 2,6-Bis-(3-tert.butyl-5-methyl-2-hydroxybenzyl)-4-methylphenol, 1,1,3-Tris-(5-tert.butyl-4-hydroxy-2-methylphenyl)-butan, 1,1-Bis-(5-tert.butyl-4-hydroxy-2-methylphenyl)-3-n-dodecylmercaptobutan, Ethylenglycol-bis-[3,3-bis-(3'-tert.butyl-4'-hydroxyphenyl)-butyrat],Bis-(3-tert.butyl-4-hydroxy-5-methylphenyl)-dicyclopentadien, Bis-[2-(3'-tert.butyl-2'-hydroxy-5'-methylbenzyl)-6-tert.butyl-4-methyl-phenyl]-terephthalat.

1.5 Benzylverbindungen, z.B. 1,3,5-Tris-(3,5-di-tert.butyl-4-hydroxybenzyl)-2,4,6-trimethylbenzol, Bis-(3,5-di-tert.butyl-4-hydroxybenzyl)-sulfid, 3,5-Di-tert.butyl-4-hydroxybenzyl-mercaptoessigsäure-isooctylester, Bis-(4-tert.butyl-3-hydroxy-2,6-dimethylbenzyl)dithiol-terephthalat, 1,3,5-Tris-(3,5-di-tert.butyl-4-hydroxybenzyl)-isocyanurat, 1,3,5-Tris-(4-tert.butyl-3-hydroxy-2,6-dimethylbenzyl)-isocyanurat, 3,5-Di-tert.butyl-4-hydroxybenzyl-phosphonsäure-dioctadecylester, Ca-Salz des 3,5-Di-tert.butyl-4-hydroxybenzyl-phosphonsäure-monoethylester, 1,3,5-Tris-(3,5-dicyclohexyl-4-hydroxybenzyl)isocyanurat.

1.6. Acylaminophenole, z.B. 4-Hydroxy-laurinsäureanilid, 4-Hydroxy-stearinsäureanilid, 2,4-Bis-(octylmercapto)-6-(3,5-di-tert.butyl-4-hydroxyanilino)-s-triazin, N-(3,5-di-tert.butyl-4-hydroxyphenyl)-carbaminsäureoctylester.

1.7. Ester der β-(3,5-Di-tert.butyl-4-hydroxyphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxyethyl)-isocyanurat, N,N'-Bis-(hydroxyethyl)-oxalsäurediamid.

1.8. Ester der β-(5-tert.Butyl-4-hydroxy-3-methylphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid.

1.9. Ester der β-(3,5-Dicyclohexyl-4-hydroxyphenyl)-propionsäure mit einoder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid.

1.10. Amide der β-(3,5-Di-tert.butyl-4-hydroxyphenyl)-propionsäure, wie z.B. N,N'-Bis-(3,5-di-tert.butyl-4-hydroxyphenylpropionyl)-hexamethylendiamin, N,N'-Bis-(3,5-di-tert.butyl-4-hydroxyphenylpropionyl)-trimethylendiamin, N,N'-Bis-(3,5-di-tert.butyl-4-hydroxyphenylpropionyl)-hydrazin.

2. UV-Absorber und Lichtschutzmittel

2.1. 2-(2'-Hydroxyphenyl)-benztriazole, wie z.B. das 5'-Methyl-, 3',5'-Di-tert.butyl-, 5'-tert.Butyl-, 5'-(1,1,3,3-Tetramethylbutyl)-, 5-Chlor-3',5'-di-tert.butyl-, 5-Chlor-3'-tert.butyl-5'-methyl-, 3'-sec.Butyl-5'-tert.butyl, 4'-Octoxy-, 3',5'-Di-tert.amyl-, 3',5'-Bis-(α,α-dimethylbenzyl)-Derivat.

2.2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.

2.3. Ester von gegebenenfalls substituierten Benzoesäuren, wie z.B. 4-tert.Butyl-phenylsalicylat, Phenylsalicylat, Octylphenyl-salicylat, Dibenzoylresorcin, Bis-(4-tert.butylbenzoyl)-resorcin, Benzoylresorcin, 3,5-Di-tert.butyl-4-hydroxybenzoesaure-2,4-di-tert.butylphenylester, 3,5-Di-tert.butyl-4-hydroxybenzoesäurehexadecylester.

2.4. Acrylate, wie z.B. α-Cyan-β,β-diphenylacrylsäure-ethylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxyzimtsäure-methylester,N-(β-Carbomethoxy-β-cyanovinyl)-2-methyl-indolin.

2.5. Nickelverbindungen, wie z.B. Nickelkomplexe des 2,2'-Thio-bis-[4-(1,1,3,3-tetramethylbutyl)-phenols], wie der 1:1- oder der 1:2-Komplex, gegebenenfalls mit zusätzlichen Liganden, wie n-Butylamin, Triethanolamin oder N-Cyclohexyl-diethanolamin, Nickeldibutyldithiocarbamat, Nickelsalze von 4-Hydroxy-3,5-di-tert.butylbenzylphosphonsäure-monoalkylestern, wie vom Methyl- oder Ethylester, Nickelkomplexe von Ketoximen, wie von 2-Hydroxy-4-methyl-phenyl-undecylketoxim, Nickelkomplexe des 1-Phenyl-4-lauroyl-5-hydroxy-pyrazols, gegebenenfalls mit zusätzlichen Liganden.

2.6. Sterisch gehinderte Amine, wie z.B. Bis-(2,2,6,6-tetramethylpiperidyl)-sebacat, Bis-(1,2,2,6,6-pentamethylpiperidyl)-sebacat, n-Butyl-3,5-di-tert.butyl-4-hydroxybenzyl-malonsäure-bis(1,2,2,6,6-pentamethylpiperidyl)-ester,Kondensationsprodukt aus 1-Hydroxyethyl-2,2,6,6-tetramethyl-4-hydroxypiperidin und Bernsteinsäure, Kondensationsprodukt aus N,N'-Bis-(2,2,6,6-Tetramethyl-4-piperidyl)-hexamethylendiamin und 4-tert.Octylamino-2,6-dichlor-1,3,5-s-triazin, Tris-(2,2,6,6-tetramethyl-4-piperidyl)-nitrilotriacetat, Tetrakis-(2,2,6,6-tetramethyl-4-piperidyl)-1,2,3,4-butantetraoat, 1,1'-(1,2-Ethandiyl)-bis-(3,3,5,5-tetramethyl-piperazinon); 1,2,2,6,6-pentamethylpiperidin-4-ol; 2,2,6,6-tetramethylpiperidin-4-ol.

2.7. Oxalsäurediamide, wie z.B. 4,4'-Di-octyloxy-oxanilid, 2,2'-Di-octyloxy-5,5'-di-tert.butyl-oxanilid, 2,2'-Di-dodecyloxy-5,5'di-tert.butyl-oxanilid, 2-Ethoxy-2'-ethyl-oxanilid, N,N'-Bis-(3-dimethylaminopropyl)-oxalamid, 2-Ethoxy-5-tert.butyl-2'-ethyloxanilid und dessen Gemisch mit 2-Ethoxy-2'-ethyl-5,4'-di-tert.butyl-oxanilid, Gemische von o-und p-Methoxy- sowie von o- und p-Ethoxy-di-substituierten Oxaniliden.

2.8. 2-(2-Hydroxyphenyl)-1,3,5-triazine, wie z.B. 2,4,6-Tris(2-hydroxy-4-octyloxyphenyl)-1,3,5-triazin, 2-(2-Hydroxy-4-octyloxyphenyl)-4,6-bis-(2,4-dimethylphenyl)-1,3,5-triazin, 2-(2,4-Dihydroxyphenyl)-4,6-bis(2,4-dimethylphenyl)-1,3,5-triazin, 2,4-Bis(2-hydroxy-4-propyloxyphenyl)-6-(2,4-dimethylphenyl)-1,3,5-triazin, 2-(2-Hydroxy-4-octyloxyphenyl)-4,6-bis(4-methylphenyl)-1,3,5-triazin, 2-(2-Hydroxy-4-dodecyloxyphenyl)-4,6-bis(2,4-dimethylphenyl)-1,3,5-triazin.

3. Metalldesaktivatoren, wie z.B. N,N'-Diphenyloxalsäurediamid, N-Salicylal-N'-salicyloylhydrazin, N,N'-Bis-(salicyloyl)-hydrazin, N,N'-Bis-(3,5-di-tert.butyl-4-hydroxyphenylpropionyl)-hydrazin, 3-Salicyloylamino-1,2,4-triazol, Bis-(benzyliden)-oxalsäuredihydrazid.

4. Phosphite und Phosphonite, wie z.B. Triphenylphosphit, Diphenylalkylphosphite, Phenyldialkylphosphite, Tris-(nonylphenyl)-phosphit, Trilaurylphosphit, Trioctadecylphosphit, Distearyl-pentaerythritdiphosphit, Tris-(2,4-di-tert.butylphenyl)-phosphit, Diisodecylpentaerythrit-diphosphit, Bis-(2,4-di-tert.butylphenyl)-pentaerythritdiphosphit, Tristearyl-sorbit-triphosphit, Tetrakis-(2,4-di-tert.butylphenyl)-4,4'-biphenylen-diphosphonit, 3,9-Bis-(2,4-di-tert.butylphenoxy)-2,4,8,10-tetraoxa-3,9-diphosphaspiro[5.5]undecan.

5. Peroxidzerstörende Verbindungen, wie z.B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrittetrakis-(β-dodecylmercapto)-propionat.

6. Polyamidstabilisatoren, wie z.B. Kupfersalze in Kombination mit Jodiden und/oder Phosphorverbindungen und Salze des zweiwertigen Mangans.

7. Basische Co-Stabilisatoren, wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Stearat, Na-Ricinoleat, K-Palmitat, Antimonbrenzcatechinat oder Zinnbrenzcatechinat.

8. Nukleierungsmittel, wie z.B. 4-tert.Butylbenzoesäure, Adipinsäure, Diphenylessigsäure.

9. Füllstoffe und Verstärkungsmittel, wie z.B. Calciumcarbonat, Silikate, Glasfasern, Asbest, Talk, Kaolin, Glimmer, Bariumsulfat, Metalloxide und -hydroxide, Russ, Graphit.

10. Sonstige Zusätze, wie z.B. Weichmacher, Gleitmittel, Emulgatoren, Pigmente, Optische Aufheller, Flammschutzmittel, Antistatika, Treibmittel.

Die Verbindungen der Formel I können nach aus der Literatur bekannten Verfahren hergestellt werden. Ausgangsmaterialien sind z.B. Polyhydroxybenzole wie Catechol-, Resorcinol-, Hydrochinon und Pyrogallolderivate. Diese können z.B. durch Umsetzung der entsprechenden Phenolate mit $CO_2$ carboxyliert werden, oder man erreicht diese Stufe durch Formylierung und anschliessende Oxidation. Sulfonierung mit Schwefelsäure oder Chlorsulfonsäure erlaubt die Einführung von Sulfonsäuregruppen an den Benzolring. Carboxyalkylgruppen können durch Alkylierung der phenolischen Hydroxylgruppen eingeführt werden, indem man Halogencarbonsäureester wie Methylchloracetat; Aethylbromacetat oder Methyl-3-brompropionat verwendet, ferner mittels olefinischer Alkylierungsmittel wie Methyl- oder Aethyl-(meth)acrylat, Dimethylmaleat oder -fumarat, Maleinsäure- oder Itaconsäureanhydrid usw., oder auch mittels Epoxidverbindungen wie Methyl-2,3-epoxypropionat oder Dimethyl-2,3-epoxysuccinat und Verseifung der Esterfunktion. Die Verwendung von Halogen-, Epoxy- oder Olefin-Alkylsulfonsäurederivaten wie 3-Brom-2-hydroxypropansulfonsäure, 3-Chlorpropansulfonsäure, 2,3-Epoxypropansulfonsäure, Alkylsulfonsäure, Dimethylalkylsulfonsäure oder Sulfonsäurelactone wie 1,3-Propansulfon erlauben die direkte Einführung von Sulfonylgruppen. Allylische Substituenten können über die Claisen-Umlagerung der entsprechenden Allyläther direkt an den Benzolring gebunden werden. An die darin enthaltene Doppelbindung können dann weitere funktionelle Gruppen addiert werden, z.B. durch Sulfonierung, Halogenierung oder Epoxydierung gefolgt von der Umsetzung mit Natriumsulfit oder Trialkylphosphiten, was einer direkten Einführung einer Sulfo- bzw. Phosphorsäuregruppe gleichkommt. Die phenolischen Hydroxylgruppen können mit einer Vielzahl von Alkylierungsmitteln alkyliert werden, wie z.B. Alkyl-, Alkenyl- oder Alkinylhalogeniden, Dialkylsulfaten oder Alkylphosphonsäureestern, also beispielsweise mit Methyliodid, Aethylbromid, Propyl- oder Butylchlorid, Dimethyl- und Diäthylsulfat, Alkylbromid, Dimethylalkylbromid, Propargylbromid usw. Quaternäre Ammoniumgruppen können erhalten werden durch Quaternierung von Aminen durch Verwendung der obengenannten Alkylierungsmittel. Alkoxygruppen können eingeführt werden durch Halogenierung und Substitution des Halogenatoms mit Alkoholat. Mono- und Polycarbonsäurederivate können beispielsweise durch Metallierung der entsprechenden Mono- oder Polyalkoxybenzole mit z.B. Alkyllithium oder Grignardreagens und anschliessender Umsetzung mit $CO_2$ hergestellt werden.

Die folgenden Beispiele erläutern die Erfindung weiter. Prozentangaben beziehen sich auf das Gewicht, wenn nichts anderes angegeben ist.

Beispiel 1: Eine Beschichtungsmasse auf der Basis von Silika/Polyvinylalkohol/Beizmittel wird aus den folgenden Komponenten hergestellt:

14,2 g einer 10%igen Lösung von Polyvinylalkohol (Riedel de Haen GmbH),
0,02 g Di-t-octylphenol-polyethylenoxid,
2,00 g Silika (Syloid ® Typ 244, Grace und Co.)
0,4 g Polyfix® 601 (Beizmittel der Fa. Showa High Polymer Co.) und
11 g Wasser.

Die resultierende Beschichtungsmasse wird mit Hilfe von Ultraschall dispergiert und durch ein Sieb aus Polyesterfasern mit der Maschenweite 24 µm filtriert. Der pH wird durch Zusatz von 2N Natronlauge auf 7,0 eingestellt.

Die Beschichtungsmassen werden mit einer Drahtspirale auf photographisches Papier in einer Dicke von 50 µm aufgetragen. Die nach dem Trocknen mit warmer Luft erhaltene Beschichtung hat eine Trockenmasse von etwa 5,0 $g/m^2$.

Das Aufzeichnungsmaterial wird jeweils mit einer erfindungsgemässen Tinte, welche einen Stabilisator der Formel I enthält, bzw. als Blindprobe ohne Stabilisator ist, bedruckt.

Die Tinten dazu können z.B. wie folgt hergestellt werden: Man löst 6 g eines Stabilisators der Formel I in einer Mischung aus 47 g Diethylenglykol und 47 g Wasser und stellt den pH-Wert dieser Lösung mit einer Base, z.B. Lithiumhydroxid, auf einen Wert von vorzugsweise 7.0 ein. Es werden weitere Lösungen aus je 6 g eines Farbstoffs (C.I. Acid Yellow 23, C.I. Acid Red 249, C.I. Food Black 2) in 47 g Diethylenglykol und 47 g Wasser vorbereitet. Die Lösungen werden durch einen Ultrafilter mit 0,3 µm Porenweite filtriert. Anschliessend werden die erfindungsgemässen Drucktinten erhalten, indem man Stabilisator-Lösungen mit Farbstoff-Lösungen in gleichen Mengen vermischt.

Die erhaltenen Drucktinten bestehen aus:

3 % Farbstoff
3 % Stabilisator der Formel I
47 % Diethylenglykol
47 % Wasser

Die Blindproben werden in gleichen Mengen aus je einer Farbstofflösung mit einer Mischung aus 1 Teil Diethylenglykol und 1 Teil Wasser hergestellt.

Die Tinten werden in die Tintenpatronen der Tintenstrahldurckvorrichtung "Think-jet" (Hewlett-Packard) gefüllt. Es werden bedruckte Proben mit einer Punktdichte von 192 x 96 Punkten pro $Inch^2$ hergestellt.

Nach einer Woche Lagerung, um die Tinten völlig auszutrocknen, wird die Farbdichte (Intensität) der bedruckten Proben mit einem Densitometer (Macbeth TR 924) unter Verwendung eines Status A-Filters bestimmt. Dann werden die Probedrucke in einem Atlas Weather-o-meter mit einer Xenon-Lampe einer

Beleuchtungsstärke von 81 klux hinter einem Filter aus 6 mm dickem Fensterglas bestrahlt. Anschliessend wird erneut die Farbdichte gemessen, um den prozentualen Verlust an Farbdichte zu ermitteln.

Die Ergebnisse sind in der nachfolgenden Tabelle 1 zusammengefasst. Niedrigere Werte bedeuten höhere Lichtechtheit.

Tabelle 1

| PROBE | STABILISATOR | FARBDICHTEVERLUST (%) | | |
|---|---|---|---|---|
| | | Acid Yellow 23 5 kJ/cm² * | Acid Red 249 10 kJ/cm² * | Food Black 2 45 kJ/cm² * |
| 1 | - | 53 | 84 | 31 |
| 2 | 2,3-Dimethoxybenzoesäure | 37 | 73 | 21 |

* Gemessene Menge an Strahlungsenergie im Bereich von 300-800 nm

Beispiel 2: Wie in Beispiel 1 beschrieben, werden weitere Verbindungen der Formel I auf ihre Wirksamkeit als Stabilisatoren für Drucktinten geprüft. Die nachfolgend angegebenen Verbindungen bewirken ebenfalls eine erhebliche Verringerung des Farbdichteverlustes. Es handelt sich dabei um folgende Verbindungen (Säuren und entsprechende Salze):
3,4-Dimethoxybenzoesäure, 3,5-Dimethoxy-4-hydroxybenzoesäure, 1,3-Dimethoxy-2-hydroxybenzol, 2,5-Dimethoxybenzoesäure, 3,4-Dimethoxybenzolsulfonsäure, 3,4,5-Trimethoxyphthalsäure, 4,5-Dimethoxyphthalsäure, 2,3-Bis(carboxymethyloxy)-benzoesäure, 3,4-Methylendioxy-benzoesäure, 2,3-Ethylendioxy-benzoesäure, 2,3-Isopropylidendioxy-benzoesäure, 3,4-Dihydroxybenzoesäuremethylester, 2,5-Dimethoxybenzoesäure, 3,5-Dimethoxybenzoesäure, 2,6-Dimethoxybenzoesäure, 1,3-Dimethoxy-2-hydroxybenzol, 1,2,3-Trimethoxybenzoesäure, 2,4,5-Trimethoxybenzoesäure, 3,4,5-Trimethoxybenzoesäure, 3,4-Dihydroxybenzoesäuremethylester, 1,4-Dihydroxy-2-methylbenzol, 2,5-Dihydroxyphenylessigsäure, 1,4-Bis-(carboxymethyl)-2,5-dihydroxybenzol sowie um die Verbindungen der folgenden Formeln:

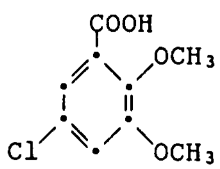

,

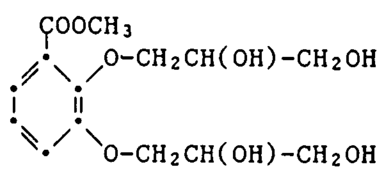

,

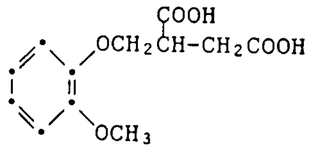

,

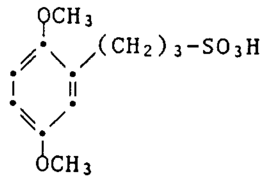

,

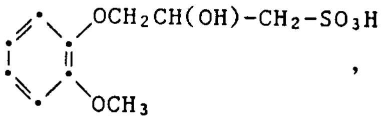

,

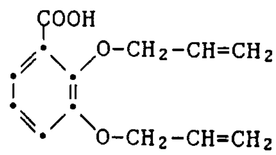

,

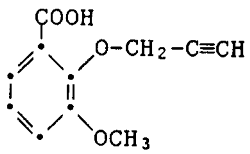

,

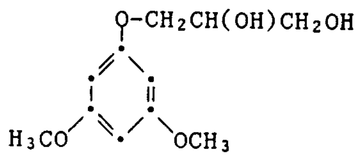

,

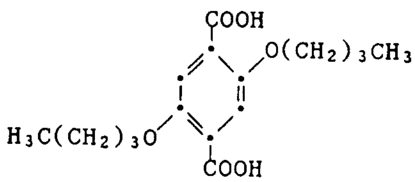

,

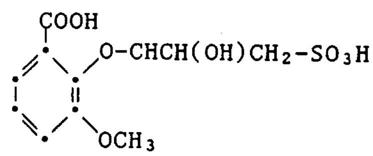

,

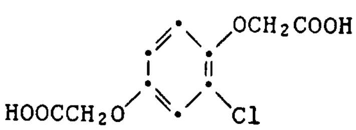

,

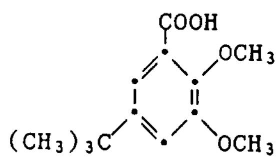

,

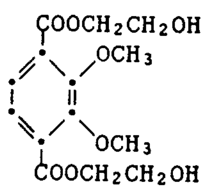

,

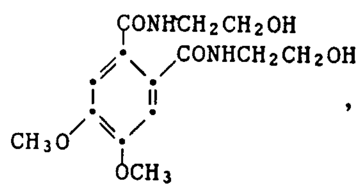

,

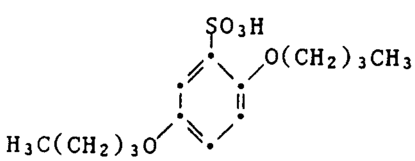

,

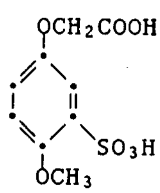

,

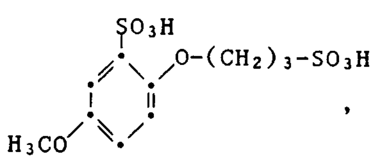

,

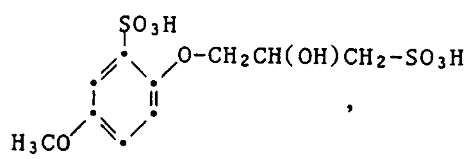

,

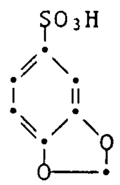 , 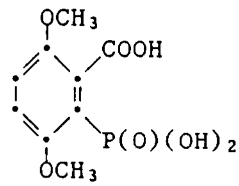 , 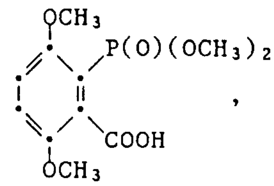 ,

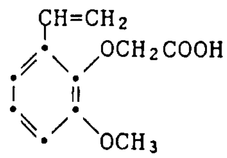 , 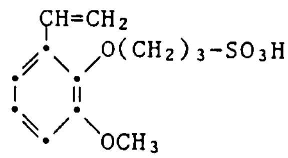 ,

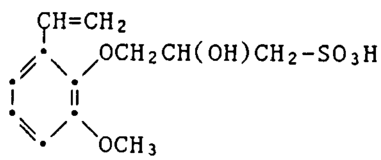 , 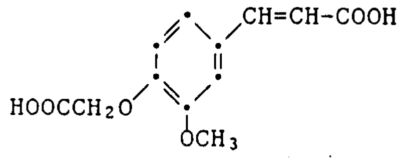 ,

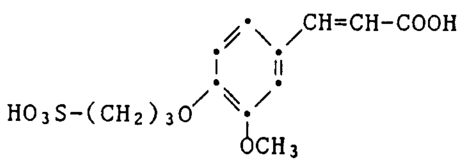 ,

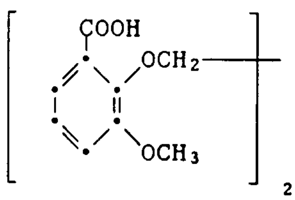 , 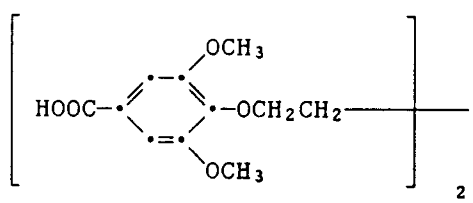 ,

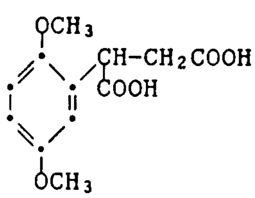 , 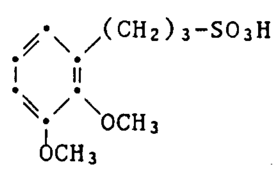 ,

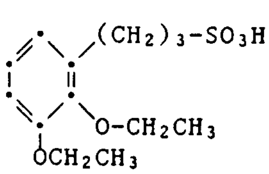 , 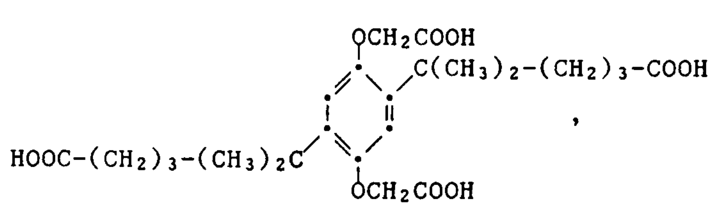 ,

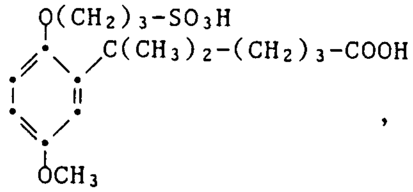 ,

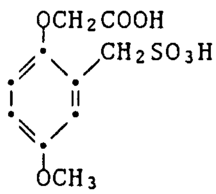 ,

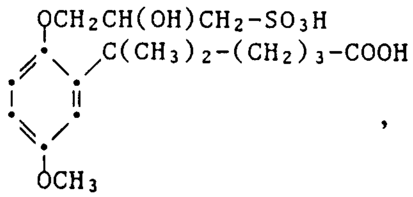 ,

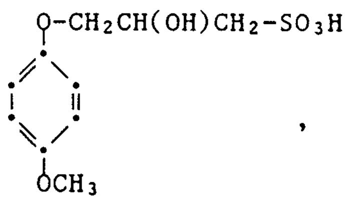 ,

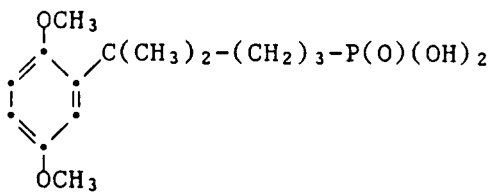 ,

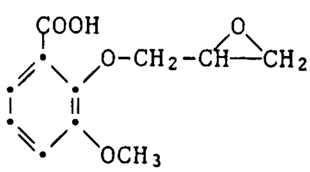 ,

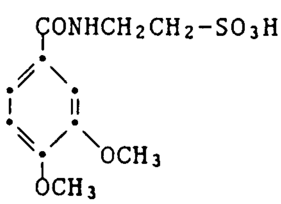 ,

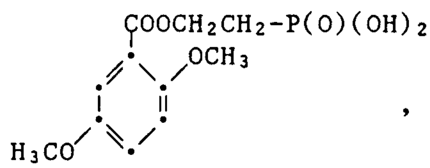 ,

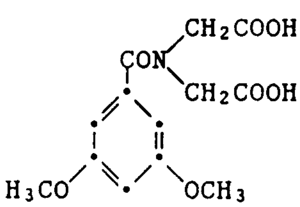 ,

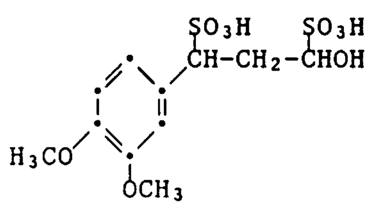 ,

Beispiel 3: Tintenstrahldrucktinten werden aus je 3 g eines Farbstoffes (C.I. Acid Yellow 23, C.I. Acid Red 35, C.I. Direct Red 227, C.I. Acid Red 249, C.I. Food Black 2), 10 g eines Stabilisators der Formel I, 43.5 g Diäthylenglykol und 43.5 g Wasser hergestellt. Die Lösungen werden durch einen Ultrafilter mit 0.3 µm Porenweite filtriert und in Tintenpatronen der Tintenstrahldruckvorrichtung "Quiet-Jet" (Hewlett Packard) gefüllt. Es werden gedruckte Proben mit den erfindungsgemässen Tinten auf "Paint-Jet" Tintenstrahldruckpapier (Hewlett Packard) mit einer Punktdichte von 192 x 96 Punkten pro $Inch^2$ hergestellt. Nach Lagerung von einer Woche sind die Tinten völlig trocken. Analog Beispiel 1 misst man die Farbdichten der Tinten. Die Ergebnisse sind in Tabelle 2 zusammengefasst. Niedrigere Werte bedeuten nach wie vor eine höhere Lichtechtheit.

Tabelle 2

| Probe | Stabilisator | Farbdichteverlust (%) Acid Yellow 23 10 kJ/cm² | Acid Red 35 10 kJ/cm² | Direct Red 227 10 kJ/cm² | Acid Red 249 10 kJ/cm² | Food Black 2 60 kJ/cm² |
|---|---|---|---|---|---|---|
| 1 | - | 40 | 53 | 72 | 64 | 69 |
| 2 | 2,3-Dimethoxybenzosäure, [Lithiumsalz] | 21 | 40 | 65 | 45 | 68 |
| 3 | 3,4-Dimethoxybenzosäure, [Lithiumsalz] | 20 | 43 | 63 | 53 | 67 |
| 4 | 2,6-Dimethoxybenzosäure, [Lithiumsalz] | 21 | 42 | 59 | 52 | 66 |
| 5 | 3,4,5-Trimethoxybenzosäure, [Lithiumsalz] | 22 | 42 | 60 | 43 | 63 |

Beispiel 4: Fluoreszierende Tinten werden aus je 1 g eines Farbstoffes (Cyanosin, Na-Fluorescein, Rhodamine 6G), 8 g Wasser, 2 g N,N-Dimethylimidazolidon, 1 g Glycerin und 2 g eines Stabilisators der Formel I hergestellt. In der Blindprobe werden zusätzlich 2 g Wasser anstelle des Stabilisators verwendet. Die erfindungsgemässen Tinten werden in ausgewaschene und getrocknete Filzstiftschreiber (0.7 mm; für Hewlett Packard Pen Plotter) der Firma Faber Castell gefüllt. Farbige Prüfmuster werden auf Hewlett-Packard "non-glossy" Plotterpapier mit einer Schreibgeschwindigkeit von 20 cm/sek. und einem Linienabstand von 12 Linien pro cm erzeugt. Nach Trocknung während einer Woche werden die Farbdichten der Muster gemessen wie in Beispiel 1. Die Resultate sind in Tabelle 3 zusammengefasst.

Tabelle 3

| Probe | Stabilisator | Farbdichteverlust nach 5 $kJ/cm^2$ (%) | | |
|---|---|---|---|---|
| | | Na-Fluorescein | Cyanosin | Rhodamin 6G |
| 1 | - | 70 | 93 | 77 |
| 2 | 3,4,5-Trimethoxybenzosäure Lithiumsalz | 47 | 84 | 77 |
| 3 | 2,3-Dimethoxybenzosäure Lithiumsalz | 45 | 88 | 63 |

Beispiel 5: Nichtfluoreszierende Pen Plotter-Tinten werden aus je 1 g eines Farbstoffes (C.I. Acid Red 35, C.I. Acid Red 249), 9 g Wasser, 1 g Glycerin und 2 g eines Stabilisators der Formel I hergestellt. Diese Tinten werden analog Beispiel 4 in Filzstiftschreiber gefüllt. Damit stellt man Probedrucke auf Plotter-Papier her. Die Farbdichten der Proben werden wie in Beispiel 1 gemessen. Die Resultate sind in Tabelle 4 zusammengefasst.

Tabelle 4

| Probe | Stabilisator | Farbdichteverlust nach 5 $kJ/cm^2$ (%) | |
|---|---|---|---|
| | | Acid Red 35 | Acid Red 249 |
| 1 | - | 27 | 62 |
| 2 | 3,4,5-Trimethoxybenzoesäure Lithiumsalz | 16 | 29 |
| 3 | 2,3-Dimethoxybenzoesäure Lithiumsalz | 17 | 21 |

**Patentansprüche**
**Patentansprüche für folgende Vertragsstaaten : BE, DE, FR, GB, IT, NL**

**1.** Tinte enthaltend als Stabilisator mindestens eine Verbindung der Formel I

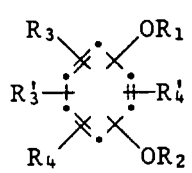

(I),

worin
$R_1$ und $R_2$ unabhängig voneinander $C_1$-$C_4$-Alkyl, welches gegebenenfalls mit einem oder 2 -OH, $-COO^{\ominus}M^{\oplus}$ und/oder $-SO_3^{\ominus}M^{\oplus}$ substituiert ist, $C_3$-$C_5$-Alkenyl, $C_3$-$C_5$-Alkinyl,

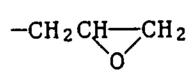

,

$-CH_2CH(OH)CH_2-SO_3^{\ominus}M^{\oplus}$, -CO-Alkyl($C_1$-$C_4$) bedeuten, welches gegebenenfalls mit $-COOR_5$ oder $-CO-N(R_5)(R_6)$ substituiert ist, oder, falls $OR_1$ und $OR_2$ in ortho-Stellung zueinander stehen, $R_1$ und $R_2$ zusammen für $C_1$-$C_6$-Alkylen stehen, wobei $M^{\oplus}$ für $H^{\oplus}$, ein ein-, zwei- oder dreiwertiges Metallkation oder eine Gruppe $(R_{12}')N^{\oplus}(R_{12}'')(R_{13}')(R_{14}')$ steht, worin $R_{12}'$, $R_{12}''$, $R_{13}'$ und $R_{14}'$ unabhängig voneinander H, $C_1$-$C_1$-Alkyl, welches gegebenenfalls mit 1 bis 3 OH substituiert oder durch ein O unterbrochen ist, Allyl, Cyclopentyl, Cyclohexyl, Phenyl, Benzyl oder Tolyl sind, oder $R_1$ auch für eine Gruppe

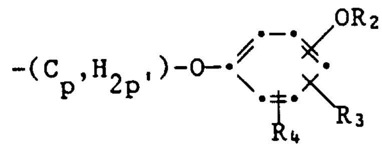

stehen kann, worin p' eine Zahl von 2 bis 6 ist,

$R_5$ und $R_6$ unabhängig voneinander H oder $C_1$-$C_4$-Alkyl, welches gegebenenfalls mit einem OH, $COOR^o$, $COO^{\ominus}M^{\oplus}$, $SO_3{}^{\ominus}M^{\oplus}$ oder $P(O)(OR^o)_2$ oder $-P(O)-(O^{\ominus}M^{\oplus})_2$ substituiert ist, bedeuten,

$R_3$' und $R_4$' unabhängig voneinander H, $C_1$-$C_4$-Alkyl, OH oder $C_1$-$C_4$-Alkoxy bedeuten,

$R_3$ und $R_4$ unabhängig voneinander H, Halogen, $-OR_7$, $-COOR^o$, $-COO^{\ominus}M^{\oplus}$,$-OOC-R_5$, $-CO-N(R_5)(R_6)$, $-(R_5)N-CO-R_6$, $-CO-R_5$, $-SO_3{}^{\ominus}M^{\oplus}$, $-SO_2N(R_5)(R_6)$, $-P(OR_5)_3$, $-(O)P(OR^o)_2$, $-(O)P-(O^{\ominus}M^{\oplus})_2$, $C_1$-$C_8$-Alkyl, welches gegebenenfalls mit 1 bis 7 $-OR_5$ oder $-OOC-R_5$, mit 1 oder 2 $-COOR^o$, $-COO^{\ominus}M^{\oplus}$, $-CO-N(R_5)(R_6)$ oder mit einem oder zwei $-SO_3{}^{\ominus}M^{\oplus}$, $-SO_2N(R_5)(R_6)$, $-(O)P(OR^o)_2$ oder $-(O)P-(O^{\ominus}M^{\oplus})_2$ substituiert ist oder $C_5$-$C_6$-Cycloalkyl oder Allyl bedeuten, wobei $M^{\oplus}$, $R_5$ und $R_6$ die angegebene Bedeutung haben und $R^o$ ein gegebenenfalls mit einem -OH substituiertes $C_1$-$C_4$-Alkyl ist, und $R_7$ ein gegebenenfalls mit 1 oder 2 -OH substituiertes $C_1$-$C_4$-Alkyl oder -CO-Alkyl-($C_1$-$C_4$) bedeutet, oder

$R_3$ und $R_4$ unabhängig voneinander eine der Gruppen der Formeln II-IV bedeuten

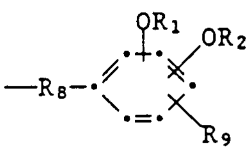 , 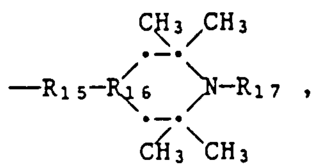 , 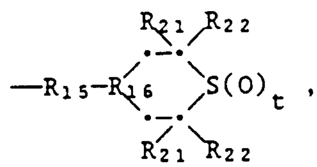 ,

(II) (III) (IV)

wobei

$R_8$ eine direkte Bindung oder Methylen ist, $R_9$ H, $C_1$-$C_8$-Alkyl, $-COO^{\ominus}M^{\oplus}$ oder $-SO_3{}^{\ominus}M^{\oplus}$ ist, worin $M^{\oplus}$ und $R_1$ und $R_2$ die angegebene Bedeutung haben, $R_{15}$ -CO-,

$$-(O)_g-C_pH_{2p}-CO-,$$

$-OOC-C_pH_{2p}-$, $-COO-C_pH_{2p}-$, $-O-C_pH_{2p}-$, $-O-CH_2-CH(OH)-CH_2-$ oder

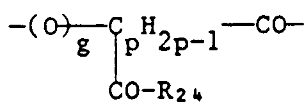

bedeutet, wobei g 0 oder 1 und p 1 bis 6 bedeuten, $R_{24}$ $-OR_5$, $-N(R_5)(R_6)$ oder eine Gruppe

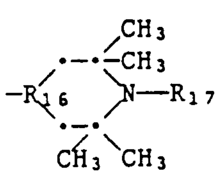

ist,

$R_{16}$ für einen der folgenden Reste steht:

-O-CH< ,

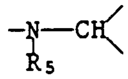 , 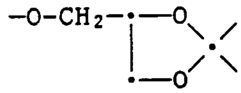 oder 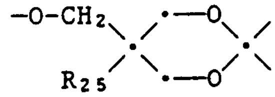 ,

wobei $R_{25}$ H oder $C_1$-$C_4$-Alkyl bedeutet,
$R_{17}$ H, $C_1$-$C_4$-Alkyl, welches gegebenenfalls mit einem -OH substituiert ist, $-CH_2-CH(OH)-CH_2-OH$, $C_1$-$C_4$-Alkoxy, -OH, -CO-Alkyl($C_1$-$C_4$), $-COCH=CH_2$, Allyl, Benzyl oder eine Gruppe

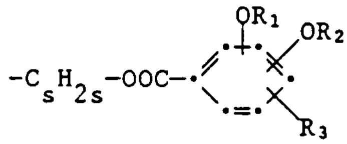

bedeutet, wobei s für die Zahl 2 oder 3 steht,
t für eine Zahl von 0 bis 2 steht, und
$R_{21}$ und $R_{22}$ unabhängig voneinander H, $C_1$-$C_4$-Alkyl oder Phenyl bedeuten.

**2.** Tinte gemäss Anspruch 1, enthaltend als Stabilisator mindestens eine Verbindung der Formel I'

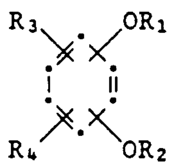 (I'),

worin
$R_1$ und $R_2$ unabhängig voneinander $C_1$-$C_4$-Alkyl, welches gegebenenfalls mit einem oder 2 -OH, $-COO^{\ominus}M^{\oplus}$ und/oder $-SO_3^{\ominus}M^{\oplus}$ substituiert ist, $-CH_2CH(OH)-CH_2-SO_3^{\ominus}M^{\oplus}$, -CO-Alkyl($C_1$-$C_4$) sind, $M^{\oplus}$ für $H^{\oplus}$, ein ein-, zwei- oder dreiwertiges Metallkation oder eine Gruppe
$(R_{12}')N^{\oplus}(R_{12}'')(R_{13}')(R_{14}')$ steht,
$R_5$ und $R_6$ unabhängig voneinander H oder $C_1$-$C_4$-Alkyl, welches gegebenenfalls mit einem OH substituiert ist, bedeuten,
$R_3$ und $R_4$ unabhängig voneinander H, Halogen, $-OR_7$, $-COOR^{o}$, $-OOC-R_5$,
$-COO^{\ominus}M^{\oplus}$, $-CO-N(R_5)(R_6)$, $-(R_5)N-CO-R_6$, $-CO-R_5$, $-SO_3^{\ominus}M^{\oplus}$, $-SO_2N(R_5)(R_6)$,
$-P(OR_5)_3$, $-(O)P{(OR^{o})_2}$, $-(O)P-(O^{\ominus}M^{\oplus})_2$, $C_1$-$C_8$-Alkyl, welches gegebenenfalls mit 1 bis 7 $-OR_5$ oder $-OOC-R_5$, mit 1 oder 2 $-COOR^{o}$, $-COO^{\ominus}M^{\oplus}$,
$-CO-N(R_5)(R_6)$ oder mit einem $-SO_3^{\ominus}M^{\oplus}$, $-SO_2N(R_5)(R_6)$, $-(O)P{(OR^{o})_2}$ oder $-(O)P-(O^{\ominus}M^{\oplus})_2$ substituiert ist, sind, bedeuten, wobei
$R^{o}$ ein gegebenenfalls mit einem -OH substituiertes $C_1$-$C_4$-Alkyl ist, und $R_7$ ein gegebenenfalls mit 1 oder 2 -OH substituiertes $C_1$-$C_4$-Alkyl bedeutet, oder
$R_3$ und $R_4$ unabhängig voneinander eine Gruppe der Formel II bedeuten

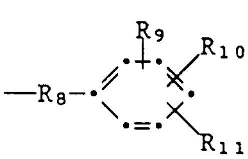 , (II)

wobei
$R_8$ eine direkte Bindung oder Methylen ist, $R_9$ H, $C_1$-$C_8$-Alkyl, $-COO^{\ominus}M^{\oplus}$ oder $-SO_3^{\ominus}M^{\oplus}$ ist, worin $M^{\oplus}$ die angegebene Bedeutung hat, und $R_1$ und $R_2$ die angegebene Bedeutung haben.

3. Tinte gemäss Anspruch 1, worin $R_1$ und $R_2$ unabhängig voneinander $C_1$-$C_4$-Alkyl, Allyl, $C_2$-$C_4$-Hydroxyalkyl, -$C_1$-$C_4$-Alkyl-$COO^{\ominus}M^{\oplus}$, -$CH_2CH(OH)CH_2$-$SO_3^{\ominus}M^{\oplus}$ oder -CO-Alkyl($C_1$-$C_4$) bedeuten, oder worin $R_1$ eine Gruppe der Formel

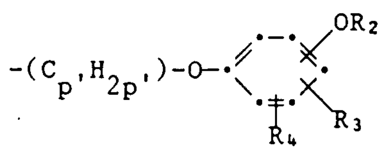

ist.

4. Tinte gemäss Anspruch 3, worin $R_1$ und $R_2$ unabhängig voneinander Methyl, Ethyl, Allyl, -$CH_2CH_2$-OH, -$CH_2$-$COO^{\ominus}M^{\oplus}$, -$CH_2CH(OH)CH_3$ oder -$CH_2CH(OH)CH_2(OH)$ bedeuten.

5. Tinte gemäss Anspruch 1, worin $R_3$ und $R_4$ unabhängig voneinander H, Halogen, -$OR_7$, -$COOR^o$, -$COO^{\ominus}M^{\oplus}$, CO-N($R_5$)($R_6$), -$SO_3^{\ominus}M^{\oplus}$, -$SO_2N(R_5)(R_6)$, $C_1$-$C_8$-Alkyl, welches gegebenenfalls mit 1 oder 2 -$COOR^0$ oder -$COO^{\ominus}M^{\oplus}$, substituiert ist, oder Allyl bedeuten.

6. Tinte gemäss Anspruch 1, worin $R_3$ und/oder $R_4$ den Rest -$OR_7$ bedeuten, wobei $R_7$ für $C_1$-$C_4$-Alkyl, $C_2$-$C_4$-Hydroxyalkyl oder -$CH_2$-$CH(OH)CH_2$-OH steht.

7. Tinte gemäss Anspruch 1, worin $R_1$ und $R_2$ unabhängig voneinander $C_1$-$C_4$-Alkyl oder mit Carboxy substituiertes $C_1$-$C_4$-Alkyl oder $R_1$ und $R_2$ zusammen $C_1$-$C_4$-Alkylen bedeuten, $R_3$ und $R_4$ unabhängig voneinander H, $COO^{\ominus}M^{\oplus}$, -$COOR^0$, $C_1$-$C_4$-Alkoxy, $C_1$-$C_4$-Alkyl-$COO^{\ominus}M^{\oplus}$, $C_1$-$C_4$-Alkyl-$COOR^0$, $C_1$-$C_4$-Alkyl oder -$SO_3^{\ominus}M^{\oplus}$ und $R_3$' und $R_4$' unabhängig voneinander H oder $C_1$-$C_4$-Alkoxy bedeuten.

8. Tinte gemäss Anspruch 1, worin $R_1$ und $R_2$ $CH_3$, $R_3$ -$COO^{\ominus}M^{\oplus}$ oder -$COOR^0$ und $R_4$ Methoxy ist.

9. Verbindung der Formel $I^o$

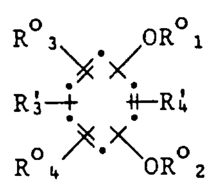

($I^o$),

worin
$R^o_1$ und $R^o_2$ unabhängig voneinander $C_1$-$C_4$-Alkyl, welches gegebenenfalls mit einem oder 2 -OH, -$COO^{\ominus}M^{\oplus}$ und/oder -$SO_3^{\ominus}M^{\oplus}$ substituiert ist, $C_3$-$C_5$-Alkenyl, $C_3$-$C_5$-Alkinyl,

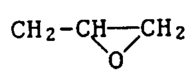

,

-$CH_2CH(OH)CH_2$-$SO_3^{\ominus}M^{\oplus}$ oder -Co-Alkyl($C_1$-$C_4$) bedeuten, welches gegebenenfalls mit -$COOR_5$ oder -CO-N($R_5$)($R_6$) substituiert ist, oder, falls $OR^o_1$ und $OR^o_2$ in ortho-Stellung zueinander stehen, $R^o_1$ und $R^o_2$ zusammen für $C_1$-$C_6$-Alkylen stehen, wobei
$M^{\oplus}$ für $H^{\oplus}$, ein Alkalimetallion oder eine Gruppe
($R'_{12}$)$N^{\oplus}$($R_{12}$'')($R_{13}$')($R_{14}$') steht, worin $R_{12}$', $R_{12}$'', $R_{13}$' und
$R_{14}$' unabhängig voneinander H, $C_1$-$C_1$-Alkyl, welches gegebenenfalls mit 1 bis 3 OH substituiert oder durch ein O unterbrochen ist, Allyl, Cyclopentyl, Cyclohexyl, Phenyl, Benzyl oder Tolyl sind, oder $R^o_1$ auch für eine Gruppe

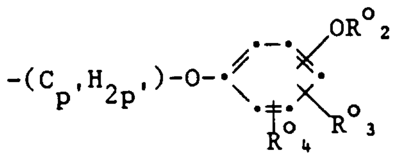

stehen kann, worin p' eine Zahl von 2 bis 6 ist,
$R_5$ und $R_6$ unabhängig voneinander H oder $C_1$-$C_4$-Alkyl, welches gegebenenfalls mit einem OH, $COOR^o$, $COO^{\ominus}M^{\oplus}$, $SO_3{}^{\ominus}M^{\oplus}$, $P(O)(OR^o)_2$ oder $-P(O)-(O^{\ominus}M^{\oplus})_2$ substituiert ist, bedeuten,
$R_3$' und $R_4$' unabhängig voneinander H, $C_1$-$C_4$-Alkyl, OH oder $C_1$-$C_4$-Alkoxy bedeuten,
$R^o{}_3$ und $R^o{}_4$ unabhängig voneinander H, Halogen, $-OR_7$, $-COOR^o$, $-COO^{\ominus}M^{\oplus}$, $OOC-R_5$, $-CO-N(R_5)(R_6)$, $-(R_5)N-CO-R_6$, $-CO-R_5$, $-SO_3{}^{\ominus}M^{\oplus}$, $-SO_2N(R_5)(R_6)$, $-P(OR_5)_3$, $-(O)P(OR^o)_2$, $-(O)P-(O^{\ominus}M^{\oplus})_2$, $C_1$-$C_8$-Alkyl, welches gegebenenfalls mit 1 bis 7 $-OR_5$ oder $-OOC-R_5$, mit 1 oder 2 $-COOR^o$, $-COO^{\ominus}M^{\oplus}$, $CO-N(R_5)R_6)$ oder mit einem $-SO_3{}^{\ominus}M^{\oplus}$, $-SO_2N(R_5)(R_6)$, $-(O)P(OR^o)_2$ oder $-(O)P-(O^{\ominus}M^{\oplus})_2$ substituiert ist, oder Allyl, $C_5$-$C_6$-Cycloalkyl bedeuten, wobei $M^{\oplus}$, $R_5$ und $R_6$ die angegebene Bedeutung haben und
$R^o$ ein gegebenenfalls mit einem -OH substituiertes $C_1$-$C_4$-Alkyl ist, und $R_7$ ein gegebenenfalls mit 1 oder 2 -OH substituiertes $C_1$-$C_4$-Alkyl oder -CO-Alkyl($C_1$-$C_4$) bedeutet, oder
$R^o{}_3$ und $R^o{}_4$ unabhängig voneinander eine der Gruppen der Formeln II-IV bedeuten

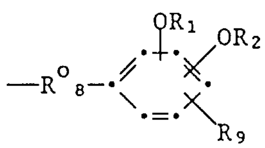 , 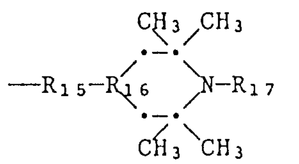 , 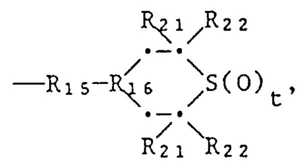

(II°) (III) (IV)

wobei
$R^o{}_8$ eine direkte Bindung, oder Methylen ist, $R_9$ Wasserstoff, $C_1$-$C_8$-Alkyl, $-COO^{\ominus}M^{\oplus}$ oder $-SO_3{}^{\ominus}M^{\oplus}$ ist,
$R_{15}$ -CO-,

$$-(O)_g-C_pH_{2p}-CO-,$$

$-OOC-C_pH_{2p}-$, $-COO-C_pH_{2p}-$, $-O-C_pH_{2p}-$, $-O-CH_2-CH(OH)-CH_2-$ oder

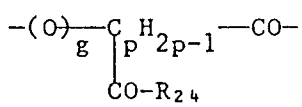

bedeutet, wobei g 0 oder 1 und p 1 bis 6 bedeuten, $R_{24}$ $-OR_5$, $-N(R_5)(R_6)$ oder eine Gruppe

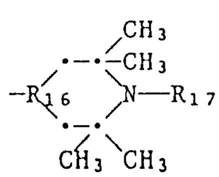

ist,
$R_{16}$ für einen der folgenden Reste steht:
-O-CH< ,

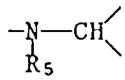 , 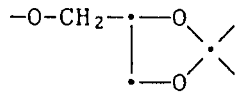 oder 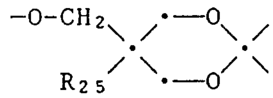 ,

wobei $R_{25}$ H oder $C_1$-$C_4$-Alkyl bedeutet,
$R_{17}$ H, $C_1$-$C_4$-Alkyl, welches gegebenenfalls mit einem -OH substituiert ist, $-CH_2-CH(OH)-CH_2-OH$, $C_1$-$C_8$-Alkoxy, -OH, -CO-Alkyl($C_1$-$C_4$), $-CO-CH=CH_2$, Allyl, Benzyl oder eine Gruppe

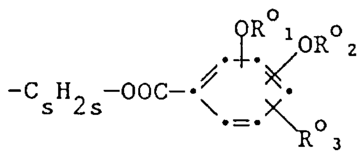

bedeutet, wobei s für die Zahl 2 oder 3 steht,
t für eine Zahl von 0 bis 2 steht, und
$R_{21}$ und $R_{22}$ unabhängig voneinander H, $C_1$-$C_4$-Alkyl oder Phenyl bedeuten,
ausgenommen die folgenden Verbindungen:

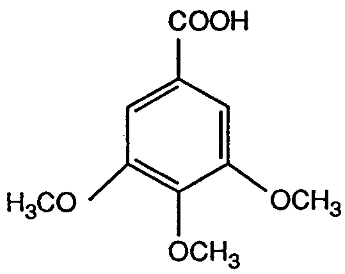 ; 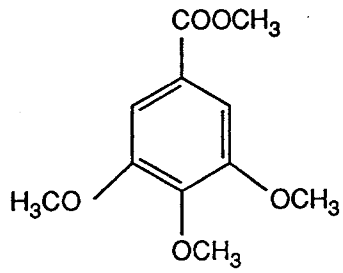 ; 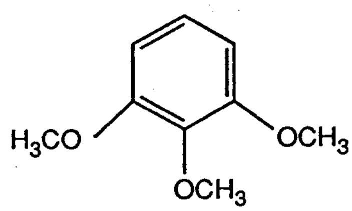 ;

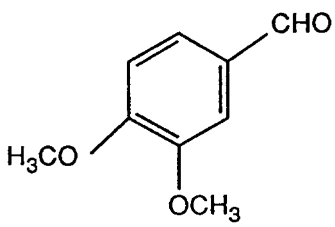 ; 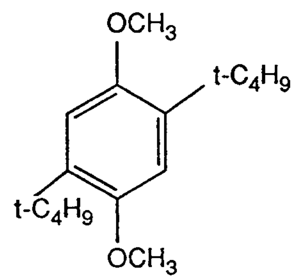 ; 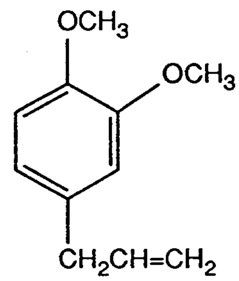 ;

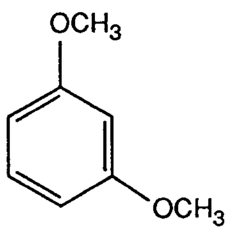 ; 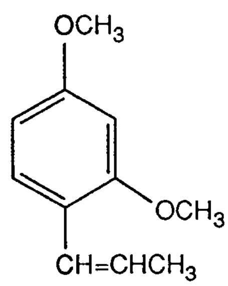 ; 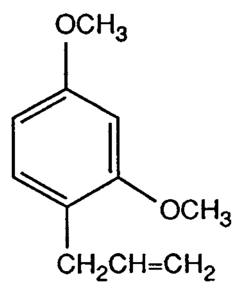 ;

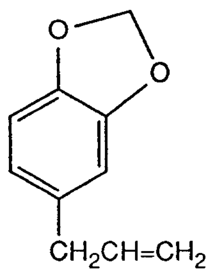 ; 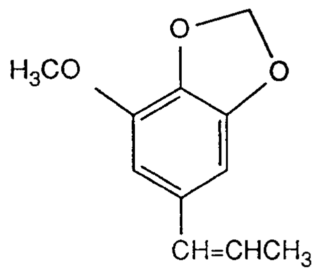 ; 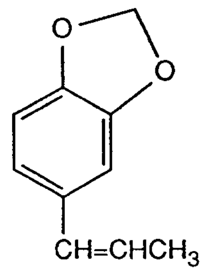 ;

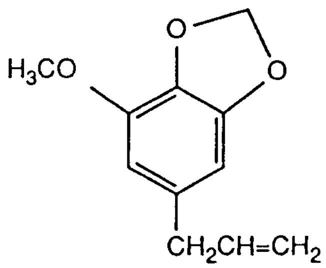 ; 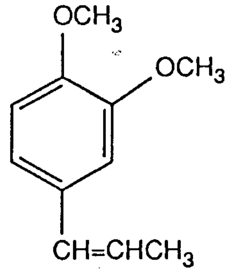 ; 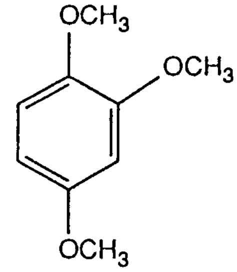 ;

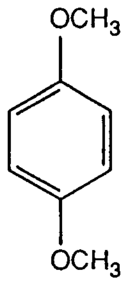 ; 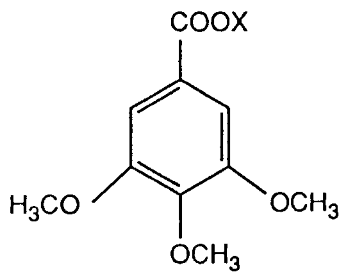 ,

wobei X Na, K, $NH_4$, Ca oder $HN(C_2H_5)_3$ ist;

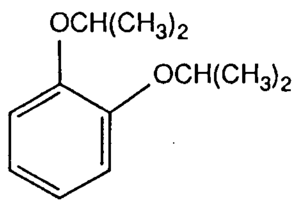 .

**10.** Verbindung gemäss Anspruch 9, worin $R^o_1$ und $R^o_2$ unabhängig voneinander $C_1$-$C_4$-Alkyl, welches mit einem -OH substituiert ist, $-CH_2-CH(OH)CH_2-SO_3^{\ominus}M^{\oplus}$, $-C_1$-$C_4$-Alkyl-$COO^{\ominus}M^{\oplus}$ oder -CO-Alkyl($C_1$-$C_4$), welches gegebenenfalls mit $-COOR^0$, $-COO^{\ominus}M^{\oplus}$, $-CO-N(R_5)(R_6)$ oder mit 1 oder 2 OH substituiert ist, bedeuten.

**11.** Verbindung gemäss Anspruch 9, worin $R^o_3$ und/oder $R^o_4$ den Rest $-OR_7$ bedeuten, wobei $R_7$ für $C_1$-$C_4$-Alkyl, $C_2$-$C_4$-Hydroxyalkyl oder $-CH_2-CH(OH)CH_2-OH$.

**12.** Verbindung gemäss Anspruch 9, worin $R^o_4$ Wasserstoff ist und $R^o_3$ $-SO_3^{\ominus}M^{\oplus}$, $-COOR^0$, $-COO^{\ominus}M^{\oplus}$ oder $-CO-NHR_5$ bedeutet.

**13.** Verwendung von Verbindungen der Formel I gemäss Anspruch 13 als Stabilisatoren für Tinten.

**14.** Verwendung gemäss Anspruch 14, worin die Tinte für den Tintenstrahldruck verwendet wird.

**15.** Verwendung gemäss Anspruch 14, worin die Tinte für den Tintenstrahldruck verwendet wird und zusätzlich mindestens einen wasserlöslichen Azofarbstoff enthält.

**16.** Verwendung gemäss Anspruch 13 als Stabilisator für Gelatineschichten photographischer Materialien, die einen Farbstoff oder Farbstoffvorläufer enthalten.

**Patentansprüche für folgenden Vertragsstaat : ES**

**1.** Tinte enthaltend als Stabilisator mindestens eine Verbindung der Formel I

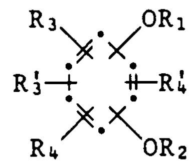

(I),

worin

$R_1$ und $R_2$ unabhängig voneinander $C_1$-$C_4$-Alkyl, welches gegebenenfalls mit einem oder 2 -OH, $-COO^{\ominus}M^{\oplus}$ und/oder $-SO_3{}^{\ominus}M^{\oplus}$ substituiert ist, $C_3$-$C_5$-Alkenyl, $C_3$-$C_5$-Alkinyl,

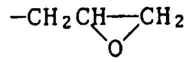

$-CH_2CH(OH)CH_2-SO_3{}^{\ominus}M^{\oplus}$,
-CO-Alkyl($C_1$-$C_4$) bedeuten, welches gegebenenfalls mit $-COOR_5$ oder $-CO-N(R_5)(R_6)$ substituiert ist, oder, falls $OR_1$ und $OR_2$ in ortho-Stellung zueinander stehen, $R_1$ und $R_2$ zusammen für $C_1$-$C_6$-Alkylen stehen, wobei $M^{\oplus}$ für $H^{\oplus}$, ein ein-, zwei- oder dreiwertiges Metallkation oder eine Gruppe $(R_{12}')N^{\oplus}(R_{12}'')(R_{13}')(R_{14}')$ steht, worin $R_{12}'$, $R_{12}''$, $R_{13}'$ und $R_{14}'$ unabhängig voneinander H, $C_1$-$C_1$-Alkyl, welches gegebenenfalls mit 1 bis 3 OH substituiert oder durch ein O unterbrochen ist, Allyl, Cyclopentyl, Cyclohexyl, Phenyl, Benzyl oder Tolyl sind, oder $R_1$ auch für eine Gruppe

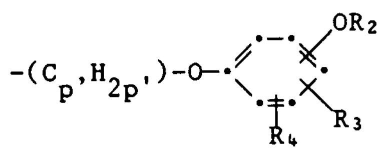

stehen kann, worin p' eine Zahl von 2 bis 6 ist,
$R_5$ und $R_6$ unabhängig voneinander H oder $C_1$-$C_4$-Alkyl, welches gegebenenfalls mit einem OH, $COOR^{\circ}$, $COO^{\ominus}M^{\oplus}$, $SO_3{}^{\ominus}M^{\oplus}$ oder $P(O)(OR^{\circ})_2$ oder $-P(O)-(O^{\ominus}M^{\oplus})_2$ substituiert ist, bedeuten,
$R_3'$ und $R_4'$ unabhängig voneinander H, $C_1$-$C_4$-Alkyl, OH oder $C_1$-$C_4$-Alkoxy bedeuten,
$R_3$ und $R_4$ unabhängig voneinander H, Halogen, $-OR_7$, $-COOR^{\circ}$, $-COO^{\ominus}M^{\oplus}$, $-OOC-R_5$, $-CO-N(R_5)(R_6)$, $-(R_5)N-CO-R_6$, $-CO-R_5$, $-SO_3{}^{\ominus}M^{\oplus}$, $-SO_2N(R_5)(R_6)$, $-P(OR_5)_3$, $-(O)P(OR^{\circ})_2$, $-(O)P-(O^{\ominus}M^{\oplus})_2$, $C_1$-$C_8$-Alkyl, welches gegebenenfalls mit 1 bis 7 $-OR_5$ oder $-OOC-R_5$, mit 1 oder 2 $-COOR^{\circ}$, $-COO^{\ominus}M^{\oplus}$, $-CO-N(R_5)(R_6)$ oder mit einem oder zwei $-SO_3{}^{\ominus}M^{\oplus}$, $-SO_2N(R_5)(R_6)$, $-(O)P(OR^{\circ})_2$ oder $-(O)P-(O^{\ominus}M^{\oplus})_2$ substituiert ist oder $C_5$-$C_6$-Cycloalkyl oder Allyl bedeuten, wobei $M^{\oplus}$, $R_5$ und $R_6$ die angegebene Bedeutung haben und $R^{\circ}$ ein gegebenenfalls mit einem -OH substituiertes $C_1$-$C_4$-Alkyl ist, und $R_7$ ein gegebenenfalls mit 1 oder 2 -OH substituiertes $C_1$-$C_4$-Alkyl oder -CO-Alkyl-($C_1$-$C_4$) bedeutet, oder
$R_3$ und $R_4$ unabhängig voneinander eine der Gruppen der Formeln II-IV bedeuten

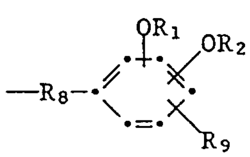 , 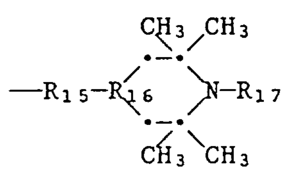 , 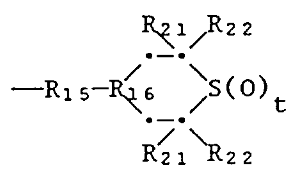 ,

(II) (III) (IV)

wobei

$R_8$ eine direkte Bindung oder Methylen ist, $R_9$ H, $C_1$-$C_8$-Alkyl, $-COO^{\ominus}M^{\oplus}$ oder $-SO_3^{\ominus}M^{\oplus}$ ist, worin $M^{\oplus}$ und $R_1$ und $R_2$ die angegebene Bedeutung haben, $R_{15}$ -CO-,

$$-(O)_g-C_pH_{2p}-CO-,$$

$-OOC-C_pH_{2p}-$, $-COO-C_pH_{2p}-$, $-O-C_pH_{2p}-$, $-O-CH_2-CH(OH)-CH_2-$ oder

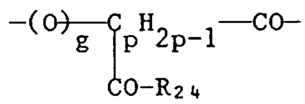

bedeutet, wobei g 0 oder 1 und p 1 bis 6 bedeuten, $R_{24}$ $-OR_5$, $-N(R_5)(R_6)$ oder eine Gruppe

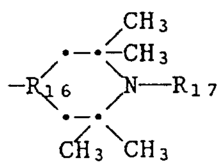

ist,
$R_{16}$ für einen der folgenden Reste steht:
-O-CH< ,

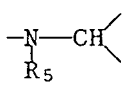 , 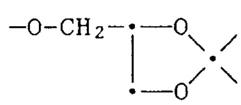 oder 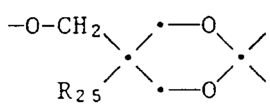 ,

wobei $R_{25}$ H oder $C_1$-$C_4$-Alkyl bedeutet,
$R_{17}$ H, $C_1$-$C_4$-Alkyl, welches gegebenenfalls mit einem -OH substituiert ist, $-CH_2-CH(OH)-CH_2-OH$, $C_1$-$C_8$-Alkoxy, -OH, -CO-Alkyl($C_1$-$C_4$), $-CO-CH=CH_2$, Allyl, Benzyl oder eine Gruppe

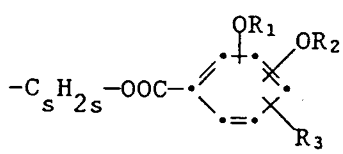

bedeutet, wobei s für die Zahl 2 oder 3 steht,
t für eine Zahl von 0 bis 2 steht, und
$R_{21}$ und $R_{22}$ unabhängig voneinander H, $C_1$-$C_4$-Alkyl oder Phenyl bedeuten.

**2.** Tinte gemäss Anspruch 1, enthaltend als Stabilisator mindestens eine Verbindung der Formel I'

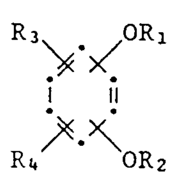 (I'),

worin
$R_1$ und $R_2$ unabhängig voneinander $C_1$-$C_4$-Alkyl, welches gegebenenfalls mit einem oder 2 -OH, $-COO^{\ominus}M^{\oplus}$ und/oder $-SO_3^{\ominus}M^{\oplus}$ substituiert ist, $-CH_2CH(OH)-CH_2-SO_3^{\ominus}M^{\oplus}$, -CO-Alkyl($C_1$-$C_4$) sind, $M^{\oplus}$ für

$H^{\oplus}$, ein ein-, zwei- oder dreiwertiges Metallkation oder eine Gruppe $(R_{12}')N^{\oplus}(R_{12}'')(R_{13}')(R_{14}')$ steht,
$R_5$ und $R_6$ unabhängig voneinander H oder $C_1$-$C_4$-Alkyl, welches gegebenenfalls mit einem OH substituiert ist, bedeuten,
$R_3$ und $R_4$ unabhängig voneinander H, Halogen, $-OR_7$, $-COOR^o$, $-OOC-R_5$, $-COO^{\ominus}M^{\oplus}$, $-CO-N(R_5)(R_6)$, $-(R_5)N-CO-R_6$, $-CO-R_5$, $-SO_3{}^{\ominus}M^{\oplus}$, $-SO_2N(R_5)(R_6)$, $-P(OR_5)_3$, $-(O)P\{OR^o)_2$, $-(O)P-(O^{\ominus}M^{\oplus})_2$, $C_1$-$C_8$-Alkyl, welches gegebenenfalls mit 1 bis 7 $-OR_5$ oder $-OOC-R_5$, mit 1 oder 2 $-COOR^o$, $-COO^{\ominus}M^{\oplus}$, $-CO-N(R_5)(R_6)$ oder mit einem $-SO_3{}^{\ominus}M^{\oplus}$, $-SO_2N(R_5)(R_6)$, $-(O)P\{OR^o)_2$ oder $-(O)P-(O^{\ominus}M^{\oplus})_2$ substituiert ist, sind, bedeuten, wobei
$R^o$ ein gegebenenfalls mit einem -OH substituiertes $C_1$-$C_4$-Alkyl ist, und $R_7$ ein gegebenenfalls mit 1 oder 2 -OH substituiertes $C_1$-$C_4$-Alkyl bedeutet, oder
$R_3$ und $R_4$ unabhängig voneinander eine Gruppe der Formel II bedeuten

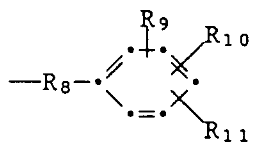 , (II)

wobei
$R_8$ eine direkte Bindung oder Methylen ist, $R_9$ H, $C_1$-$C_8$-Alkyl, $-COO^{\ominus}M^{\oplus}$ oder $-SO_3{}^{\ominus}M^{\oplus}$ ist, worin $M^{\oplus}$ die angegebene Bedeutung hat, und $R_1$ und $R_2$ die angegebene Bedeutung haben.

**3.** Tinte gemäss Anspruch 1, worin $R_1$ und $R_2$ unabhängig voneinander $C_1$-$C_4$-Alkyl, Allyl, $C_2$-$C_4$-Hydroxyalkyl, $-C_1$-$C_4$-Alkyl-$COO^{\ominus}M^{\oplus}$, $-CH_2CH(OH)CH_2-SO_3{}^{\ominus}M^{\oplus}$ oder -CO-Alkyl($C_1$-$C_4$) bedeuten, oder worin $R_1$ eine Gruppe der Formel

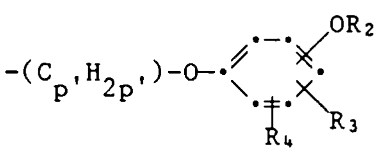

ist.

**4.** Tinte gemäss Anspruch 3, worin $R_1$ und $R_2$ unabhängig voneinander Methyl, Ethyl, Allyl, $-CH_2CH_2-OH$, $-CH_2-COO^{\ominus}M^{\oplus}$, $-CH_2CH(OH)CH_3$ oder $-CH_2CH(OH)CH_2(OH)$ bedeuten.

**5.** Tinte gemäss Anspruch 1, worin $R_3$ und $R_4$ unabhängig voneinander H, Halogen, $-OR_7$, $-COOR^o$, $-COO^{\ominus}M^{\oplus}$, $CO-N(R_5)(R_6)$, $-SO_3{}^{\ominus}M^{\oplus}$, $-SO_2N(R_5)(R_6)$, $C_1$-$C_8$-Alkyl, welches gegebenenfalls mit 1 oder 2 $-COOR^0$ oder $-COO^{\ominus}M^{\oplus}$, substituiert ist, oder Allyl bedeuten.

**6.** Tinte gemäss Anspruch 1, worin $R_3$ und/oder $R_4$ den Rest $-OR_7$ bedeuten, wobei $R_7$ für $C_1$-$C_4$-Alkyl, $C_2$-$C_4$-Hydroxyalkyl oder $-CH_2-CH(OH)CH_2-OH$ steht.

**7.** Tinte gemäss Anspruch 1, worin $R_1$ und $R_2$ unabhängig voneinander $C_1$-$C_4$-Alkyl oder mit Carboxy substituiertes $C_1$-$C_4$-Alkyl oder $R_1$ und $R_2$ zusammen $C_1$-$C_4$-Alkylen bedeuten, $R_3$ und $R_4$ unabhängig voneinander H, $COO^{\ominus}M^{\oplus}$, $-COOR^0$, $C_1$-$C_4$-Alkoxy, $C_1$-$C_4$-Alkyl-$COO^{\ominus}M^{\oplus}$, $C_1$-$C_4$-Alkyl-$COOR^0$, $C_1$-$C_4$-Alkyl oder $-SO_3{}^{\ominus}M^{\oplus}$ und $R_3'$ und $R_4'$ unabhängig voneinander H oder $C_1$-$C_4$-Alkoxy bedeuten.

**8.** Tinte gemäss Anspruch 1, worin $R_1$ und $R_2$ $CH_3$, $R_3$ $-COO^{\ominus}M^{\oplus}$ oder $-COOR^0$ und $R_4$ Methoxy ist.

**9.** Verwendung von Verbindungen der Formel I°

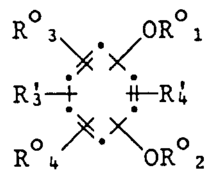

(I°),

worin
$R^o_1$ und $R^o_2$ unabhängig voneinander $C_1$-$C_4$-Alkyl, welches gegebenenfalls mit einem oder 2 -OH, $-COO^{\ominus}M^{\oplus}$ und/oder $-SO_3{}^{\ominus}M^{\oplus}$ substituiert ist, $C_3$-$C_5$-Alkenyl, $C_3$-$C_5$-Alkinyl,

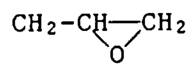

,

$-CH_2CH(OH)CH_2-SO_3{}^{\ominus}M^{\oplus}$ oder -CO-Alkyl($C_1$-$C_4$) bedeuten, welches gegebenenfalls mit $-COOR_5$ oder $-CO-N(R_5)(R_6)$ substituiert ist, oder, falls $OR^o_1$ und $OR^o_2$ in ortho-Stellung zueinander stehen, $R^o_1$ und $R^o_2$ zusammen für $C_1$-$C_6$-Alkylen stehen, wobei
$M^{\oplus}$ für $H^{\oplus}$, ein Alkalimetallion oder eine Gruppe
$(R'_{12})N^{\oplus}(R_{12}'')(R_{13}')(R_{14}')$ steht, worin $R_{12}'$, $R_{12}''$, $R_{13}'$ und
$R_{14}'$ unabhängig voneinander H, $C_1$-$C_1$-Alkyl, welches gegebenenfalls mit 1 bis 3 OH substituiert oder durch ein O unterbrochen ist, Allyl, Cyclopentyl, Cyclohexyl, Phenyl, Benzyl oder Tolyl sind, oder $R^o_1$ auch für eine Gruppe

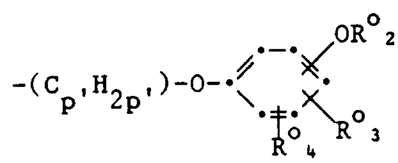

stehen kann, worin p' eine Zahl von 2 bis 6 ist,
$R_5$ und $R_6$ unabhängig voneinander H oder $C_1$-$C_4$-Alkyl, welches gegebenenfalls mit einem OH, $COOR^o$, $COO^{\ominus}M^{\oplus}$, $SO_3{}^{\ominus}M^{\oplus}$, $P(O)(OR^o)_2$ oder
$-P(O)-(O^{\ominus}M^{\oplus})_2$ substituiert ist, bedeuten,
$R_3'$ und $R_4'$ unabhängig voneinander H, $C_1$-$C_4$-Alkyl, OH oder $C_1$-$C_4$-Alkoxy bedeuten,
$R^o_3$ und $R^o_4$ unabhängig voneinander H, Halogen, $-OR_7$, $-COOR^o$, $-COO^{\ominus}M^{\oplus}$, $OOC-R_5$, $-CO-N(R_5)(R_6)$, $-(R_5)N-CO-R_6$, $-CO-R_5$, $-SO_3{}^{\ominus}M^{\oplus}$, $-SO_2N(R_5)(R_6)$, $-P(OR_5)_3$, $-(O)P(OR^o)_2$, $-(O)P-(O^{\ominus}M^{\oplus})_2$, $C_1$-$C_8$-Alkyl, welches gegebenenfalls mit 1 bis 7 $-OR_5$ oder $-OOC-R_5$, mit 1 oder 2 $-COOR^o$, $COO^{\ominus}M^{\oplus}$, $CO-N(R_5)R_6$) oder mit einem $-SO_3{}^{\ominus}M^{\oplus}$, $-SO_2N(R_5)(R_6)$, $-(O)P(OR^o)_2$ oder $-(O)P-(O^{\ominus}M^{\oplus})_2$ substituiert ist, oder Allyl, $C_5$-$C_6$-Cycloalkyl bedeuten, wobei $M^{\oplus}$, $R_5$ und $R_6$ die angegebene Bedeutung haben und
$R^o$ ein gegebenenfalls mit einem -OH substituiertes $C_1$-$C_4$-Alkyl ist, und $R_7$ ein gegebenenfalls mit 1 oder 2 -OH substituiertes $C_1$-$C_4$-Alkyl oder -CO-Alkyl($C_1$-$C_4$) bedeutet, oder
$R^o_3$ und $R^o_4$ unabhängig voneinander eine der Gruppen der Formeln II-IV bedeuten

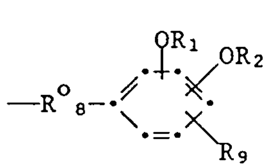

,

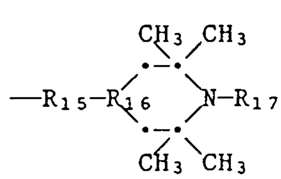

,

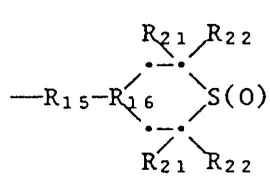

,

(II°) (III) (IV)

wobei

$R^o_8$ eine direkte Bindung, oder Methylen ist, $R_9$ Wasserstoff, $C_1$-$C_8$-Alkyl, $-COO^{\ominus}M^{\oplus}$ oder $-SO_3^{\ominus}M^{\oplus}$ ist, $R_{15}$ -CO-,

$$-(O)_g-C_pH_{2p}-CO-,$$

$-OOC-C_pH_{2p}-$, $-COO-C_pH_{2p}-$, $-O-C_pH_{2p}-$, $-O-CH_2-CH(OH)-CH_2-$ oder

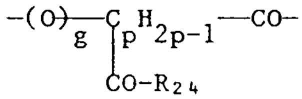

bedeutet, wobei g 0 oder 1 und p 1 bis 6 bedeuten, $R_{24}$ $-OR_5$, $-N(R_5)(R_6)$ oder eine Gruppe

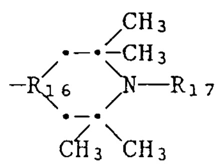

ist,
$R_{16}$ für einen der folgenden Reste steht:
-O-CH< ,

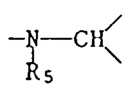 , 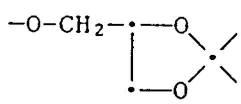 oder 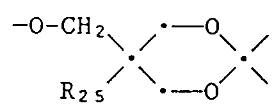 ,

wobei $R_{25}$ H oder $C_1$-$C_4$-Alkyl bedeutet,
$R_{17}$ H, $C_1$-$C_4$-Alkyl, welches gegebenenfalls mit einem -OH substituiert ist, $-CH_2-CH(OH)-CH_2-OH$, $C_1$-$C_8$-Alkoxy, -OH, -CO-Alkyl($C_1$-$C_4$), $-CO-CH=CH_2$, Allyl, Benzyl oder eine Gruppe

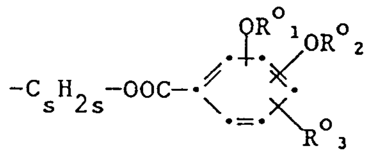

bedeutet, wobei s für die Zahl 2 oder 3 steht,
t für eine Zahl von 0 bis 2 steht, und
$R_{21}$ und $R_{22}$ unabhängig voneinander H, $C_1$-$C_4$-Alkyl oder Phenyl bedeuten,
ausgenommen die folgenden Verbindungen:

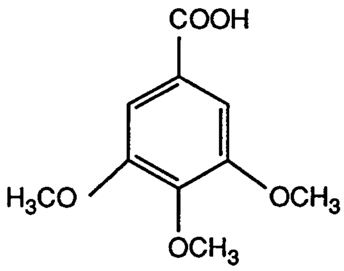 ; 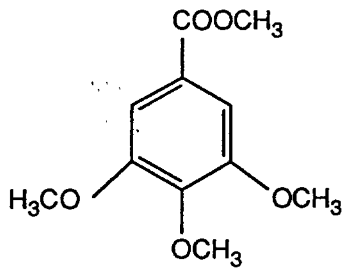 ; 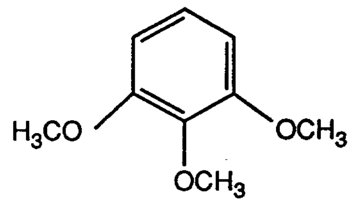 ;

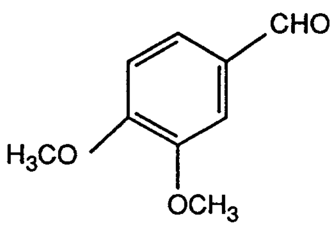 ; 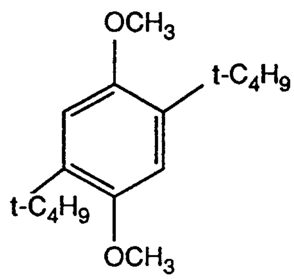 ; 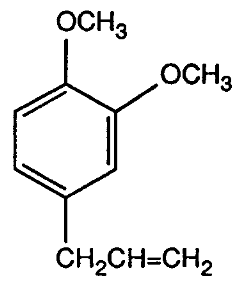 ;

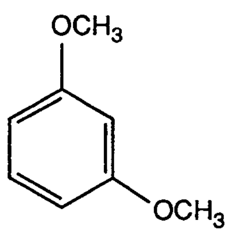 ; 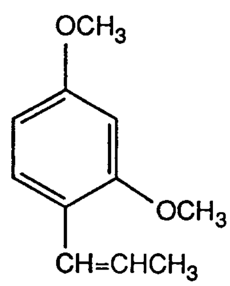 ; 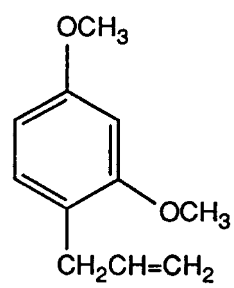 ;

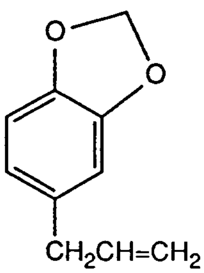 ; 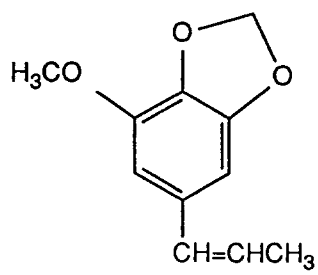 ; 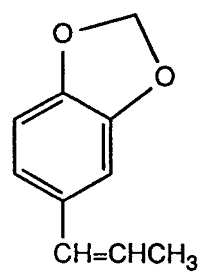 ;

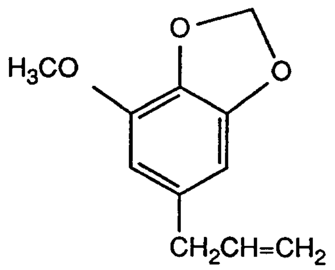 ; 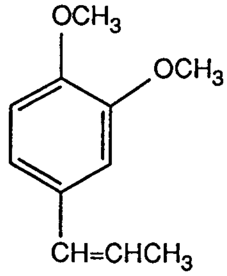 ; 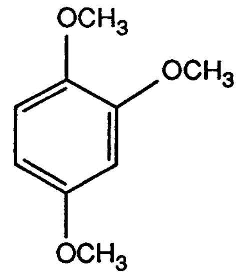 ;

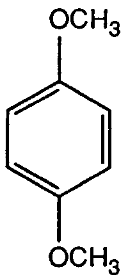 ; 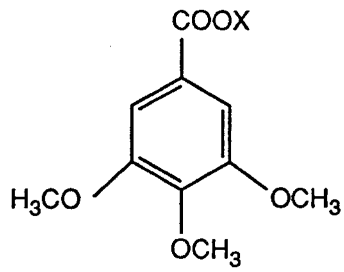 ,

wobei X Na, K, $NH_4$, Ca oder $HN(C_2H_5)_3$ ist;

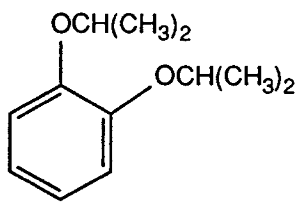 .

als Stabilisatoren für Tinten.

**10.** Verwendung von Verbindungen der Formel I als Stabilisatoren für Tinten.

**11.** Verwendung gemäss Anspruch 10, worin die Tinte für den Tintenstrahldruck verwendet wird.

**12.** Verwendung gemäss Anspruch 10, worin die Tinte für den Tintenstrahldruck verwendet wird und zusätzlich mindestens einen wasserlöslichen Azofarbstoff enthält.

**13.** Verwendung gemäss Anspruch 9 als Stabilisator für Gelatineschichten photographischer Materialien, die einen Farbstoff oder Farbstoffvorläufer enthalten.

**Claims**

**Claims for the following Contracting States : BE, DE, FR, GB, IT, NL**

**1.** An ink containing, as the stabilizer, at least one compound of the formula I

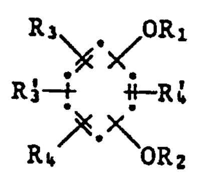 (I),

in which $R_1$ and $R_2$ independently of one another are $C_1$-$C_4$alkyl which is unsubstituted or substituted by one or 2 -OH, $-COO^{\ominus}M^{\oplus}$ and/or $-SO_3{}^{\ominus}M^{\oplus}$, $C_3$-$C_5$alkenyl, $C_3$-$C_5$alkynyl,

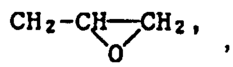

$-CH_2CH(OH)CH_2-SO_3{}^{\ominus}M^{\oplus}$, -CO-alkyl($C_1$-$C_4$) which is unsubstituted or substituted by $-COOR_5$ or -CO-N-($R_5$)($R_6$), or, if $OR_1$ and $OR_2$ are in the ortho-position relative to one another, $R_1$ and $R_2$ together are $C_1$-$C_6$alkylene, $M^{\oplus}$ being $H^{\oplus}$, a monovalent, divalent or trivalent metal cation or a group ($R_{12}$')N($R_{12}$'')-($R_{13}$')($R_{14}$'), where $R_{12}$' $R_{12}$' $R_{13}$' and $R_{14}$' are independently of one another H, $C_1$-$C_4$alkyl, optionally substituted by 1 to 3 OH or by one O, or are allyl, cyclopentyl, cyclohexyl, phenyl, benzyl or tolyl, or else $R_1$ can be a group

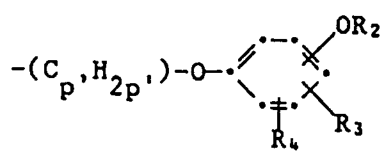

in which p' is a number from 2 to 6, $R_5$ and $R_6$ independently of one another are H or $C_1$-$C_4$alkyl which is unsubstituted or substituted by one OH, $COOR^o$, $-COO^{\ominus}M^{\oplus}$, $SO_3{}^{\ominus}M^{\oplus}$ or $P(O)(OR^o)_2$ or $P(O)(O^{\ominus}M^{\oplus})_2$, $R_3$' and $R_4$' independently of one another are H, $C_1$-$C_4$alkyl, OH or $C_1$-$C_4$alkoxy, $R_3$ and $R_4$ independently of one another are H, halogen, $-OR_7$, $-COOR^o$, $-COO^{\ominus}M^{\oplus}$, $-OOC-R_5$, $-CO-N(R_5)(R_6)$, $-(R_5)N-CO-R_6$, $-CO-R_5$, $-SO_3{}^{\ominus}M^{\oplus}$, $-SO_2N(R_5)(R_6)$, $-P(OR_5)_3$, $-(O)P-(OR^o)_2$, $-(O)-P-(O^{\ominus}M^{\oplus})$,$C_1$-$C_8$alkyl which is unsubstituted or substituted by 1 to 7 $-OR_5$ or $-OOC-R_5$ groups, by 1 or 2 $-COOR^o$, $-COO^{\ominus}M^{\oplus}$, $-CO-N(R_5)(R_6)$ or by one or two $-SO_3{}^{\ominus}M^{\oplus}$, $-SO_2N(R_5)(R_6)$, $-(O)P-(OR^o)_2$, $(O)P(O^{\ominus}M^{\oplus})_2$, or allyl or $C_5$-$C_6$cycloalkyl where $M^{\oplus}$, $R_5$ and $R_6$ are as defined above, $R^o$ being $C_1$-$C_4$alkyl which is unsubstituted or substituted by one -OH, and $R_7$ being $C_1$-$C_4$alkyl or allyl or -CO-alkyl($C_1$-$C_4$) each of which is unsubstituted or substituted by 1 or 2 -OH, or $R_3$ and $R_4$ independently of one another are one of the groups of the formulae II-IV

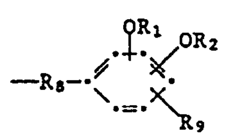 ,

(II)

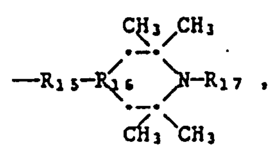

(III)

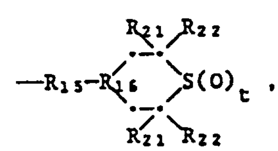

(IV)

in which $R_8$ is a direct bond or methylene, $R_9$ is H, $C_1$-$C_8$alkyl, $-COO^{\ominus}M^{\oplus}$ or $-SO_3{}^{\ominus}M^{\oplus}$ in which $M^{\oplus}$ and $R_1$ and $R_2$ are as defined above, $R_{15}$ is -CO-, $-(O)_g-C_pH_{2p}-CO-$, $-OOC-C_pH_{2p}-$, $COO-C_pH_{2p}-$, $-O-C_pH_{2p}-$, $-O-CH_2-CH(OH)-CH_2-$ or

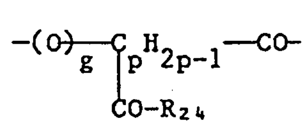

in which g is 0 or 1 and p is 1 to 6, and in which $R_{24}$ is $-OR_5$, $-N(R_5)(R_6)$ or a group

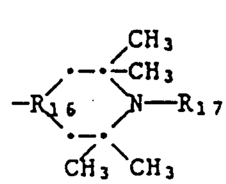

ist,
and $R_{16}$ is one of the following radicals:

-O-CH< ,

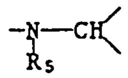 , 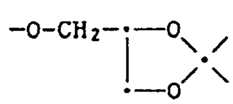 or 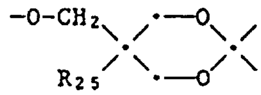 ,

in which $R_{25}$ is H or $C_1$-$C_4$alkyl,
$R_{17}$ is H, $C_1$-$C_4$alkyl which is unsubstituted or substituted by one -OH, or is $-CH_2-CH(OH)-CH_2-OH$, $C_1$-$C_4$alkoxy, -OH, -CO-alkyl($C_1$-$C_4$), $-COCH=CH_2$, allyl, benzyl or a group

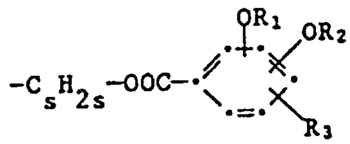

in which s is the number 2 or 3, t is a number from 0 to 2 and $R_{21}$ and $R_{22}$ independently of one another are H, $C_1$-$C_4$alkyl or phenyl.

**2.** An ink according to claim 1 containing, as the stabilizer, at least one compound of the formula I'

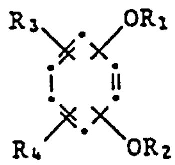 (I'),

in which $R_1$ and $R_2$ independently of one another are $C_1$-$C_4$alkyl which is unsubstituted or substituted by one or 2 -OH, $-COO^{\ominus}M^{\oplus}$ and/or $-SO_3^{\ominus}M^{\oplus}$, or are $-CH_2CH(OH)CH_2-SO_3^{\ominus}M^{\oplus}$ or -CO-alkyl($C_1$-$C_4$), $M^{\oplus}$ being $H^{\oplus}$, a monovalent, divalent or trivalent metal cation or a group $(R_{12})N(R_{12})(R_{13})(R_{14})$, $R_5$ and $R_6$ independently of one another are H or $C_1$-$C_4$alkyl which is unsubstituted or substituted by one OH, $R_3$ and $R_4$ independently of one another are H, halogen, $-OR_7$, $-COOR^{o}$, $-OOC-R_5$, $-COO^{\ominus}M^{\oplus}$, $-CO-N(R_5)-(R_6)$, $-(R_5)N-CO-R_6$, $-CO-R_5$, $-SO_3^{\ominus}M^{\oplus}$, $-SO_2N(R_5)(R_6)$, $-P(OR_5)_3$, $-(O)P-(OR^{o})_2$, $(O)P(O^{\ominus}M^{\oplus})_2$, $C_1$-$C_8$alkyl which is unsubstituted or substituted by 1 to 7 $-OR_5$ or $-OOC-R_5$, by 1 or 2 $-COOR^{o}$, $-COO^{\ominus}M^{\oplus}$ or $-CO-N(R_5)(R_6)$ or by an $-SO_3^{\ominus}M^{\oplus}$, $-SO_2N(R_5)(R_6)$, $(O)P(O^{\ominus}M^{\oplus})_2$ or $-(O)P-(OR^{o})_2$ group, $R^{o}$ being $C_1$-$C_4$alkyl which is unsubstituted or substituted by one -OH, and $R_7$ being $C_1$-$C_4$alkyl which is unsubstituted or substituted by 1 or 2 -OH, $R_3$ and $R_4$ independently of one another are a group of the formula II

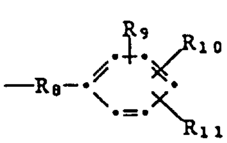 , (II)

in which $R_8$ is a direct bond or methylene, $R_9$ is H, $C_1$-$C_8$alkyl, $-COO^{\ominus}M^{\oplus}$ or $-SO_3^{\ominus}M^{\oplus}$, in which $M^{\oplus}$ is as defined above, and $R_1$ and $R_2$ are as defined above.

**3.** An ink according to claim 1, wherein $R_1$ and $R_2$ independently of one another are $C_1$-$C_4$alkyl, allyl, $C_2$-$C_4$hydroxyalkyl, $-C_1$-$C_4$-alkyl-$COO^{\ominus}M^{\oplus}$, $-CH_2CH(OH)CH_2-SO_3^{\ominus}M^{\oplus}$ or -CO-alkyl($C_1$-$C_4$), or wherein $R_1$ is a group of the formula

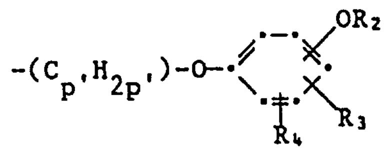

4. An ink according to claim 3, wherein $R_1$ and $R_2$ independently of one another are methyl, ethyl, allyl, $-CH_2CH_2-OH$, $-CH_2COO^{\ominus}M^{\oplus}$,$-CH_2CH(OH)CH_3$ or $-CH_2CH(OH)CH_2(OH)$.

5. An ink according to claim 1, wherein $R_3$ and $R_4$ independently of one another are H, halogen, $-OR_7$, $-COOR^o$, $-COO^{\ominus}M^{\oplus}$, $-CO-N(R_5)(R_6)$, $-SO_3{}^{\ominus}M^{\oplus}$, $-SO_2N(R_5)(R_6)$, $C_1$-$C_8$alkyl which is unsubstituted or substituted by 1 or 2 $-COOR^o$ or $-COO^{\ominus}M^{\oplus}$, or allyl.

6. An ink according to claim 1, wherein $R_3$ and/or $R_4$ are the radical $-OR_7$ in which $R_7$ is $C_1$-$C_4$alkyl, $C_2$-$C_4$hydroxyalkyl or $-CH_2-CH(OH)CH_2-OH$.

7. An ink according to claim 1, wherein $R_1$ and $R_2$ independently of one another are $C_1$-$C_4$alkyl or $C_1$-$C_4$alkyl which is substituted by carboxyl, or $R_1$ and $R_2$ together are $C_1$-$C_4$alkylene, $R_3$ and $R_4$ independently of one another are H, $-COO^{\ominus}M^{\oplus}$, $COOR^o$, $C_1$-$C_4$alkoxy, $C_1$-$C_4$alkyl-$COO^{\ominus}M^{\oplus}$, $C_1$-$C_4$alkyl-$COOR^o$, $C_1$-$C_4$alkyl or $SO_3{}^{\ominus}M^{\oplus}$, and $R_3'$ and $R_4'$ independently of one another are H or $C_1$-$C_4$alkoxy.

8. An ink according to claim 1, wherein $R_1$ and $R_2$ are $CH_3$, $R_3$ is $-COO^{\ominus}M^{\oplus}$ or $-COOR^o$ and $R_4$ is methoxy.

9. A compound of the formula I°

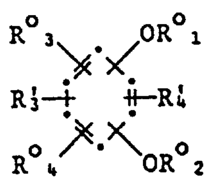

(I°),

in which $R^o_1$ and $R^o_2$ independently of one another are $C_1$-$C_4$alkyl which is unsubstituted or substituted by one or 2 -OH, $-CO^{\ominus}M^{\oplus}$
and/or $-SO_3{}^{\ominus}M^{\oplus}$, $C_3$-$C_5$alkenyl, $C_3$-$C_5$alkynyl,

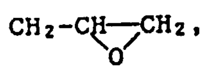

$-CH_2CH(OH)CH_2-SO_3{}^{\ominus}M^{\oplus}$ or -CO-alkyl($C_1$-$C_4$) which is unsubstituted or substituted by $-COOR_5$ or $-CO-N(R_5)(R_6)$ or, if $OR^o_1$ and $OR^o_2$ are in the ortho-position relative to one another, $R^o_1$ and $R^o_2$ together are $C_1$-$C_6$alkylene, $M^{\oplus}$ being $H^{\oplus}$, an alkali metal ion or a group $(R_{12})N^{\oplus}(R_{12})(R_{13})(R_{14})$, where $R_{12}'$, $R_{12}'$, $R_{13}'$ and $R_{14}'$ are independently of one another H, $C_1$-$C_4$alkyl optionally substituted by 1 to 3 OH or by one O or are allyl, cyclopentyl, cyclohexyl, phenyl, benzyl or tolyl, or else $R^o_1$ can be a group

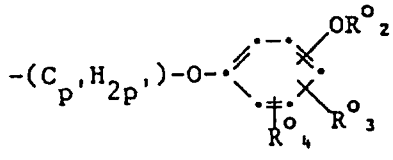

in which p' is a number from 2 to 6, $R_5$ and $R_6$ independently of one another are H or $C_1$-$C_4$alkyl which is unsubstituted or substituted by one OH, $COOR^o$, $-COO^{\ominus}M^{\oplus}$, $SO_3{}^{\ominus}M^{\oplus}$, $P(O)(O^{\ominus}M^{\oplus})_2$ or $P(O)(OR^o)_2$, $R_3'$

and $R_4'$ independently of one another are H, $C_1$-$C_4$alkyl, OH or $C_1$-$C_4$alkoxy, $R^o_3$ and $R^o_4$ independently of one another are H, halogen, $-OR_7$, $-COOR^o$, $-COO^{\ominus}M^{\oplus}$, $-OOC-R_5$, $-CO-N(R_5)(R_6)$, $-(R_5)N-CO-R_6$, $-CO-R_5$, $-SO_3{}^{\ominus}M^{\oplus}$, $-SO_2N(R_5)(R_6)$, $-P(OR_5)_3$, $-(O)P-(OR^o)_2$, $(O)P(O^{\ominus}M^{\oplus})_2$,$C_1$-$C_8$alkyl which is unsubstituted or substituted by 1 to 7 $-OR_5$ or $-OOC-R_5$ groups, by 1 or 2 $-COOR^o$, $-COO^{\ominus}M^{\oplus}$ or $-CO-N(R_5)-(R_6)$ or by one $-SO_3{}^{\ominus}M^{\oplus}$, $-SO_2N(R_5)(R_6)$, $-(O)P-(OR^o)_2$ or $(O)P(O^{\ominus}M^{\oplus})_2$, or are allyl or $C_5$-$C_6$cycloalkyl, where $M^{\oplus}$, $R_5$ and $R_6$ are as defined above, $R^o$ being $C_1$-$C_4$alkyl which is unsubstituted or substituted by one -OH, and $R_7$ being $C_1$-$C_4$alkyl or -CO-alkyl($C_1$-$C_4$) each of which is unsubstituted or substituted by 1 or 2 -OH, or $R^o_3$ and $R^o_4$ independently of one another are one of the groups of the formulae II-IV

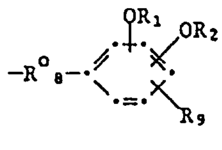 ,

(II°)

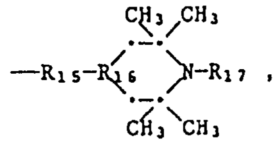 ,

(III)

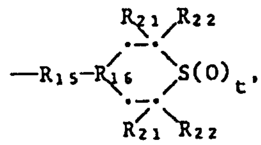 ,

(IV)

in which $R^o_8$ is a direct bond or methylene, $R_9$ is hydrogen, $C_1$-$C_8$-alkyl, $-COO^{\ominus}M^{\oplus}$ or $-SO_3{}^{\ominus}M^{\oplus}$,$R_{15}$ is -CO-,

$$-(O)_g-C_pH_{2p}-CO-,$$

, $-OOC-C_pH_{2p}-$, $-COO-C_pH_{2p}-$, $-O-C_pH_{2p}-$, $-CH_2-CH(OH)-CH_2-$ or

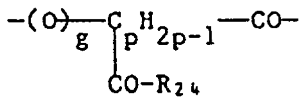 ,

in which g is 0 or 1 and p is 1 to 6, and in which $R_{24}$ is $-OR_5$, $-N(R_5)(R_6)$ or a group

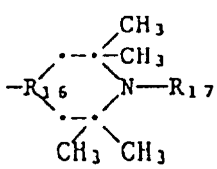

and $R_{16}$ is one of the following radicals:
-O-CH< ,

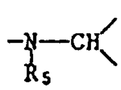 , 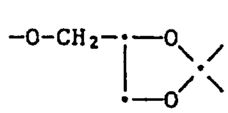 oder 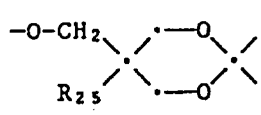 ,

where $R_{25}$ is H or $C_1$-$C_4$alkyl,
$R_{17}$ is H, $C_1$-$C_4$alkyl which is unsubstituted or substituted by one -OH, or is $-CH_2-CH(OH)-CH_2-OH$, $C_1$-$C_8$-alkoxy, -OH, -CO-alkyl($C_1$-$C_4$), $-CO-CH=CH_2$, allyl, benzyl or a group

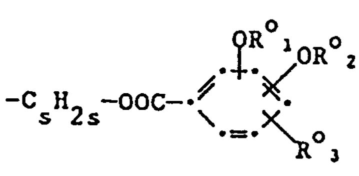

in which s is the number 2 or 3,

t is a number from 0 to 2 and $R_{21}$ and $R_{22}$ independently of one another are $C_1$-$C_4$alkyl or phenyl, with the exception of the following compounds:

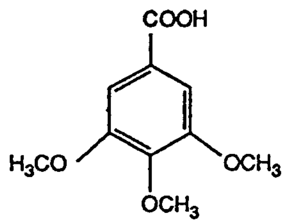 ; 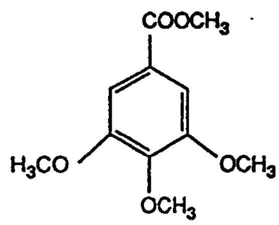 ; 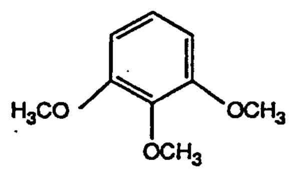 ;

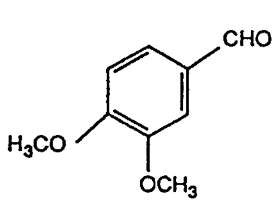 ; 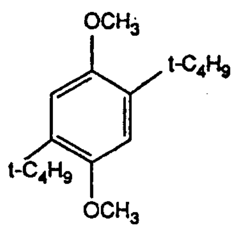 ; 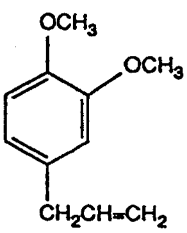 ;

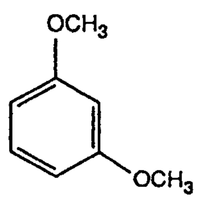 ; 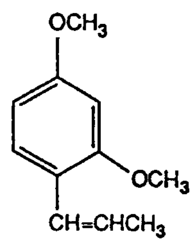 ; 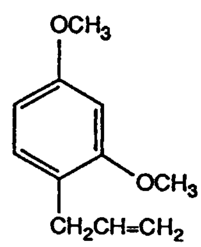 ;

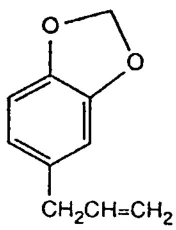 ; 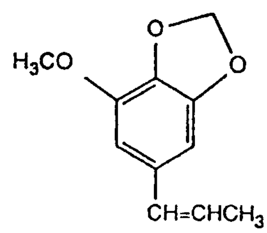 ; 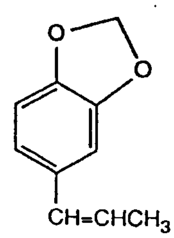 ;

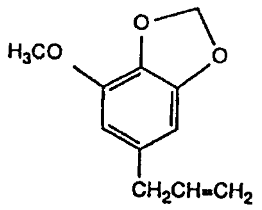 ; 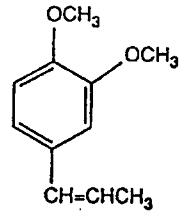 ; 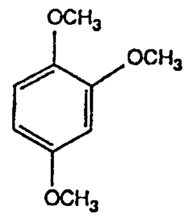 ;

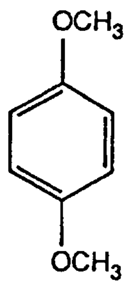 ; 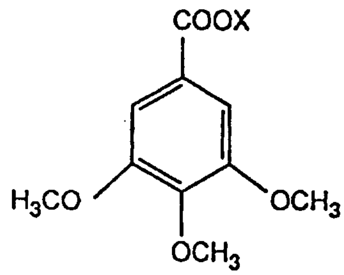 ,

$HN(C_2H_5)_3$ ist;
in which X is Na, K, $NH_4$, Ca or $HN(C_2H_5)_3$;

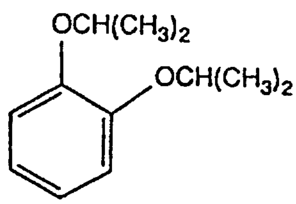 .

**10.** A compound according to claim 9, wherein $R^o_1$ and $R^o_2$ independently of one another are $C_1$-$C_4$alkyl which is substituted by one -OH, or is $-CH_2-CH(OH)CH_2-SO_3^{\ominus}M^{\oplus}$, $-C_1$-$C_4$alkyl-$COO^{\ominus}M^{\oplus}$ or -CO-alkyl-($C_1$-$C_4$) each of which is unsubstituted or substituted by $-COOR^o$, $-COO^{\ominus}M^{\oplus}$ or $-CO-N(R_5)(R_6)$ or by 1 or 2 OH.

**11.** A compound according to claim 9, wherein $R^o_3$ and/or $R^o_4$ are the radical $-OR_7$, $R_7$ being $C_1$-$C_4$alkyl, $C_2$-$C_4$hydroxyalkyl or $-CH_2-CH(OH)CH_2-OH$.

**12.** A compound according to claim 9, wherein $R^o_4$ is hydrogen and $R^o_3$ is $SO_3^{\ominus}M^{\oplus}$, $-COOR^o$, $COO^{\ominus}M^{\oplus}$ or $-CO-NHR_5$.

**13.** The use of a compound of the formula I according to claim 1 as a stabilizer for inks.

**14.** Use according to claim 13, wherein the ink is used for ink jet printing.

**15.** Use according to claim 13, wherein the ink is used for ink jet printing and additionally contains at least one water-soluble azo dye.

**16.** Use according to claim 13 as a stabilizer for gelatine layers of photographic materials containing a dye or dye precursor.

**Claims for the following Contracting State : ES**

**1.** An ink containing, as the stabilizer, at least one compound of the formula I

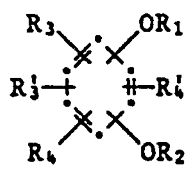

(I),

in which $R_1$ and $R_2$ independently of one another are $C_1$-$C_4$alkyl which is unsubstituted or substituted by one or 2 -OH, $-COO^{\ominus}M^{\oplus}$ and/or $-SO_3^{\ominus}M^{\oplus}$, $C_3$-$C_5$alkenyl, $C_3$-$C_5$alkinyl,

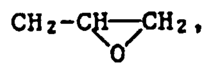
,

$-CH_2CH(OH)CH_2-SO_3^{\ominus}M^{\oplus}$, -CO-alkyl($C_1$-$C_4$) which is unsubstituted or substituted by $-COOR_5$ or -CO-N-($R_5$)($R_6$), or, if $OR_1$ and $OR_2$ are in the ortho-position relative to one another, $R_1$ and $R_2$ together are $C_1$-$C_6$alkylene, $M^{\oplus}$ being $H^{\oplus}$, a monovalent, divalent or trivalent metal cation or a group $(R_{12}')N(R_{12}'')$-$(R_{13}')(R_{14}')$ where $R_{12}'$, $R_{13}'$, $R_{13}'$ and $R_{14}'$ are independently of one another H, $C_1$-$C_4$alkyl, optionally substituted by 1 to 3 OH or by one O, or are allyl, cyclopentyl, cyclohexyl, phenyl, benzyl or tolyl, or else $R_1$ can be a group

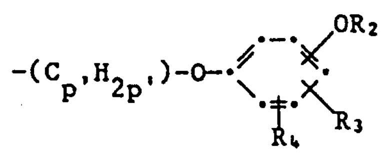

in which p' is a number from 2 to 6, $R_5$ and $R_6$ independently of one another are H or $C_1$-$C_4$alkyl which is unsubstituted or substituted by one OH, $COOR^{\circ}$, $-COO^{\ominus}M^{\oplus}$, $SO_3^{\ominus}M^{\oplus}$ or $P(O)(OR^{\circ})_2$ or $P(O)(O^{\ominus}M^{\oplus})_2$, $R_3'$ and $R_4'$ independently of one another are H, $C_1$-$C_4$alkyl, OH or $C_1$-$C_4$alkoxy, $R_3$ and $R_4$ independently of one another are H, halogen, $-OR_7$, $-COOR^{\circ}$, $-COO^{\ominus}M^{\oplus}$, $-OOC-R_5$, $-CO-N(R_5)(R_6)$, $-(R_5)N-CO-R_6$, $-CO-R_5$, $-SO_3^{\ominus}M^{\oplus}$, $-SO_2N(R_5)(R_6)$, $-P(OR_5)_3$, $-(O)P-(OR^{\circ})_2$, $-(O)-P-(O^{\ominus}M^{\oplus})$,$C_1$-$C_8$alkyl which is unsubstituted or substituted by 1 to 7 $-OR_5$ or $-OOC-R_5$ groups, by 1 or 2 $-COOR^{\circ}$, $-COO^{\ominus}M^{\oplus}$, $-CO-N(R_5)(R_6)$ or by one or two $-SO_3^{\ominus}M^{\oplus}$, $-SO_2N(R_5)(R_6)$, $-(O)P-(OR^{\circ})_2$, $(O)P(O^{\ominus}M^{\oplus})_2$, or allyl or $C_5$-$C_6$cycloalkyl where $M^{\oplus}$, $R_5$ and $R_6$ are as defined above, $R^{\circ}$ being $C_1$-$C_4$alkyl which is unsubstituted or substituted by one -OH, and $R_7$ being $C_1$-$C_4$alkyl or allyl or -CO-alkyl($C_1$-$C_4$) each of which is unsubstituted or substituted by 1 or 2 -OH, or $R_3$ and $R_4$ independently of one another are one of the groups of the formulae II-IV

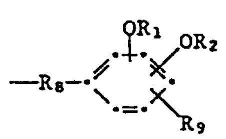
,
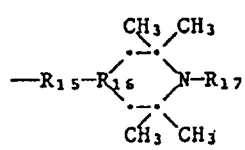
,
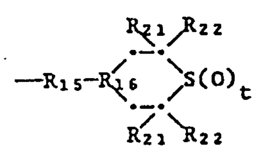
,

(II) (III) (IV)

in which $R_8$ is a direct bond or methylene, $R_9$ is H, $C_1$-$C_8$alkyl, $-COO^{\ominus}M^{\oplus}$ or $-SO_3^{\ominus}M^{\oplus}$ in which $M^{\oplus}$ and $R_1$ and $R_2$ are as defined above, $R_{15}$ is -CO-, $-(O)_g-C_pH_{2p}-CO-$, $-OOC-C_pH_{2p}-$, $-COO-C_pH_{2p}-$, $-O-C_pH_{2p}-$, $-O-CH_2-CH(OH)-CH_2-$ or

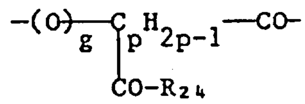

in which g is 0 or 1 and p is 1 to 6, and in which $R_{24}$ is $-OR_5$, $-N(R_5)(R_6)$ or a group

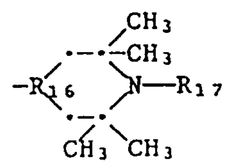

and $R_{16}$ is one of the following radicals:
-O-CH< ,

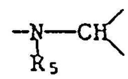 , 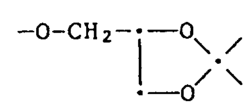 or 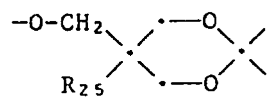 ,

in which $R_{25}$ is H or $C_1$-$C_4$alkyl,
$R_{17}$ is H, $C_1$-$C_4$alkyl which is unsubstituted or substituted by one -OH, or is $-CH_2-CH(OH)-CH_2-OH$, $C_1$-$C_4$alkoxy, -OH, -CO-alkyl($C_1$-$C_4$), $-COCH=CH_2$, allyl, benzyl or a group

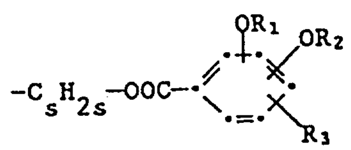 .

in which s is the number 2 or 3, t is a number from 0 to 2 and $R_{21}$ and $R_{22}$ independently of one another are H, $C_1$-$C_4$alkyl or phenyl.

**2.** An ink according to claim 1 containing, as the stabilizer, at least one compound of the formula I'

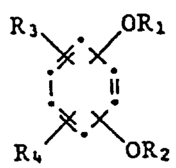 (I'),

in which $R_1$ and $R_2$ independently of one another are $C_1$-$C_4$alkyl which is unsubstituted or substituted by one or 2 -OH, $-COO^{\ominus}M^{\oplus}$ and/or $-SO_3^{\ominus}M^{\oplus}$, or are $-CH_2CH(OH)CH_2-SO_3^{\ominus}M^{\oplus}$ or CO-alkyl($C_1$-$C_4$), $M^{\oplus}$ being $H^{\oplus}$, a monovalent, divalent or trivalent metal cation or a group $(R_{12})N(R_{12})(R_{13})(R_{14})$, $R_5$ and $R_6$ independently of one another are H or $C_1$-$C_4$alkyl which is unsubstituted or substituted by one OH, $R_3$ and $R_4$ independently of one another are H, halogen, $-OR_7$, $-COOR^{o}$, $-OOC-R_5$, $-COO^{\ominus}M^{\oplus}$, $-CO-N(R_5)(R_6)$, $-(R_5)N-CO-R_6$, $-CO-R_5$, $-SO_3^{\ominus}M^{\oplus}$, $-SO_2N(R_5)(R_6)$, $-P(OR_5)_3$, $-(O)P-(OR^{o})_2$, $(O)P(O^{\ominus}M^{\oplus})_2$, $C_1$-$C_8$alkyl which is unsubstituted or substituted by 1 to 7 $-OR_5$ or $-OOC-R_5$, by 1 or 2 $-COOR^{o}$, $-COO^{\ominus}M^{\oplus}$ or $-CO-N(R_5)(R_6)$ or by an $-SO_3^{\ominus}M^{\oplus}$, $-SO_2N(R_5)(R_6)$, $(O)P(O^{\ominus}M^{\oplus})_2$ or $-(O)P-(OR^{o})_2$ group, $R^{o}$ being $C_1$-$C_4$alkyl which is unsubstituted or substituted by one -OH, and $R_7$ being $C_1$-$C_4$alkyl which is unsubstituted or substituted by 1 or 2 -OH, $R_3$ and $R_4$ independently of one another are a group of the formula II

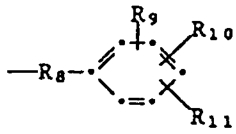, (II)

in which $R_8$ is a direct bond or methylene, $R_9$ is H, $C_1$-$C_8$alkyl, $-COO^{\ominus}M^{\oplus}$ or $-SO_3{}^{\ominus}M^{\oplus}$, in which $M^{\oplus}$ is as defined above, and $R_1$ and $R_2$ are as defined above.

**3.** An ink according to claim 1, wherein $R_1$ and $R_2$ independently of one another are $C_1$-$C_4$alkyl, allyl, $C_2$-$C_4$hydroxyalkyl, $-C_1$-$C_4$-alkyl-$COO^{\ominus}M^{\oplus}$,$-CH_2CH(OH)CH_2$-$SO_3{}^{\ominus}M^{\oplus}$ or -CO-alkyl($C_1$-$C_4$), or wherein $R_1$ is a group of the formula

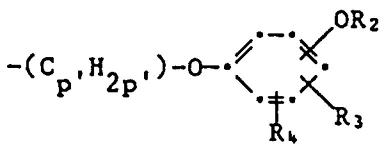

**4.** An ink according to claim 3, wherein $R_1$ and $R_2$ independently of one another are methyl, ethyl, allyl, $-CH_2CH_2$-OH, $-CH_2COO^{\ominus}M^{\oplus}$,$-CH_2CH(OH)CH_3$ or $-CH_2CH(OH)CH_2(OH)$.

**5.** An ink according to claim 1, wherein $R_3$ and $R_4$ independently of one another are H, halogen, $-OR_7$, $-COOR^{o}$, $-COO^{\ominus}M^{\oplus}$, -CO-N($R_5$)($R_6$), $-SO_3{}^{\ominus}M^{\oplus}$, $-SO_2N(R_5)(R_6)$, $C_1$-$C_8$alkyl which is unsubstituted or substituted by 1 or 2 $-COOR^{o}$ or $-COO^{\ominus}M^{\oplus}$, or allyl.

**6.** An ink according to claim 1, wherein $R_3$ and/or $R_4$ are the radical $-OR_7$ in which $R_7$ is $C_1$-$C_4$alkyl, $C_2$-$C_4$hydroxyalkyl or $-CH_2$-$CH(OH)CH_2$-OH.

**7.** An ink according to claim 1, wherein $R_1$ and $R_2$ independently of one another are $C_1$-$C_4$alkyl or $C_1$-$C_4$alkyl which is substituted by carboxyl, or $R_1$ and $R_2$ together are $C_1$-$C_4$alkylene, $R_3$ and $R_4$ independently of one another are H, $-COO^{\ominus}M^{\oplus}$, $COOR^{o}$, $C_1$-$C_4$alkoxy, $C_1$-$C_4$alkyl-$COO^{\ominus}M^{\oplus}$, $C_1$-$C_4$alkyl-$COOR^{o}$, $C_1$-$C_4$alkyl or $SO_3{}^{\ominus}M^{\oplus}$, and $R_3$' and $R_4$' independently of one another are H or $C_1$-$C_4$alkoxy.

**8.** An ink according to claim 1, wherein $R_1$ and $R_2$ are $CH_3$, $R_3$ is $-COO^{\ominus}M^{\oplus}$ or $-COOR^{o}$ and $R_4$ is methoxy.

**9.** Use of a compound of the formula I°

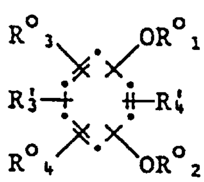 (I°),

in which $R^{o}_1$ and $R^{o}_2$ independently of one another are $C_1$-$C_4$alkyl which is unsubstituted or substituted by one or 2 -OH, $-COO^{\ominus}M^{\oplus}$ and/or $-SO_3{}^{\ominus}M^{\oplus}$, $C_3$-$C_5$alkenyl, $C_3$-$C_5$alkynyl,

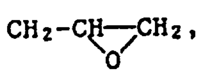 ,

$-CH_2CH(OH)CH_2$-$SO_3{}^{\ominus}M^{\oplus}$ or -CO-alkyl($C_1$-$C_4$) which is unsubstituted or substituted by $-COOR_5$ or -CO-N($R_5$)($R_6$) or, if $OR^{o}_1$ and $OR^{o}_2$ are in the ortho-position relative to one another, $R^{o}_1$ and $R^{o}_2$ together

are $C_1$-$C_6$alkylene, $M^{\oplus}$ being $H^{\oplus}$, an alkali metal ion or a group $(R_{12}')N^{\oplus}(R_{12}'')(R_{13}')(R_{14}')$, where $R_{12}$, $R_{12}$, $R_{13}$ and $R_{14}$ are independently of one another H, $C_1$-$C_4$alkyl, optionally substituted by 1 to 3 OH or by one O, or are allyl, cyclopentyl, cyclohexyl, phenyl, benzyl or tolyl, or else $R^o_1$ can be a group

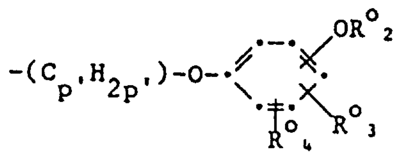

in which p' is a number from 2 to 6, $R_5$ and $R_6$ independently of one another are H or $C_1$-$C_4$alkyl which is unsubstituted or substituted by one OH, $COOR^o$, $-COO^{\ominus}M^{\oplus}$, $-SO_3{}^{\ominus}M^{\oplus}$, $P(O)(O^{\ominus}M^{\oplus})_2$ or $P(O)(OR^o)_2$, $R_3'$ and $R_4'$ independently of one another are H, $C_1$-$C_4$alkyl, OH or $C_1$-$C_4$alkoxy, $R^o_3$ and $R^o_4$ independently of one another are H, halogen, $-OR_7$, $-COOR^o$, $-COO^{\ominus}M^{\oplus}$, $-OOC-R_5$, $-CO-N(R_5)(R_6)$, $-(R_5)N-CO-R_6$, $-CO-R_5$, $-SO_3{}^{\ominus}M^{\oplus}$, $-SO_2N(R_5)(R_6)$, $-P(OR_5)_3$, $-(O)P-(OR^o)_2$, $(O)P(O^{\ominus}M^{\oplus})_2$, $C_1$-$C_8$alkyl which is unsubstituted or substituted by 1 to 7 $-OR_5$ or $-OOC-R_5$ groups, by 1 or 2 $-COOR^o$, $-COO^{\ominus}M^{\oplus}$ or $-CO-N(R_5)(R_6)$ or by one $-SO_3{}^{\ominus}M^{\oplus}$, $-SO_2N(R_5)(R_6)$, $-(O)P-(OR^o)_2$ or $(O)P(O^{\ominus}M^{\oplus})_2$, or are allyl or $C_5$-$C_6$cycloalkyl, where $M^{\oplus}$, $R_5$ and $R_6$ are as defined above, $R^o$ being $C_1$-$C_4$alkyl which is unsubstituted or substituted by one -OH, and $R_7$ being $C_1$-$C_4$alkyl or -CO-alkyl($C_1$-$C_4$) each of which is unsubstituted or substituted by 1 or 2 -OH, or $R^o_3$ and $R^o_4$ independently of one another are one of the groups of the formulae II-IV

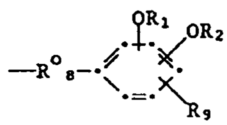 ,

(II°)

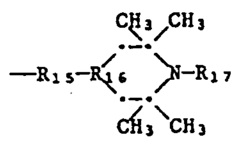 ,

(III)

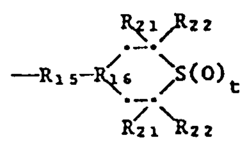

(IV)

in which $R^o_8$ is a direct bond or methylene, $R_9$ is hydrogen, $C_1$-$C_8$-alkyl, $-COO^{\ominus}M^{\oplus}$ or $-SO_3{}^{\ominus}M^{\oplus}$,
$R_{15}$ is -CO-, $-(O)_g-C_pH_{2p}-CO-$, $-OOC-C_pH_{2p}-$, $-COO-C_pH_{2p}-$, $-O-C_pH_{2p}-$, $-CH_2-CH(OH)-CH_2-$ or

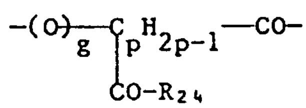

in which g is 0 or 1 and p is 1 to 6, and in which $R_{24}$ is $-OR_5$, $-N(R_5)(R_6)$ or a group

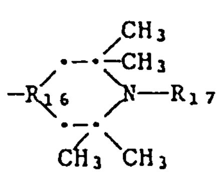

and $R_{16}$ is one of the following radicals:
-O-CH< ,

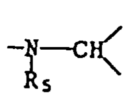 , 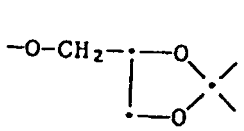 or 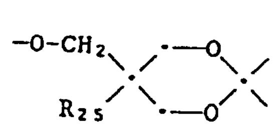 ,

where $R_{25}$ is H or $C_1$-$C_4$alkyl,

$R_{17}$ is H, $C_1$-$C_4$alkyl which is unsubstituted or substituted by one -OH, or is $-CH_2-CH(OH)-CH_2-OH$, $C_1$-$C_8$alkoxy, -OH, -CO-alkyl($C_1$-$C_4$), $-CO-CH=CH_2$, allyl, benzyl or a group

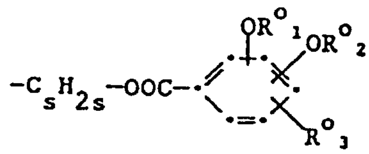

in which s is the number 2 or 3,
t is a number from 0 to 2 and $R_{21}$ and $R_{22}$ independently of one another are $C_1$-$C_4$alkyl or phenyl, with the exception of the following compounds:

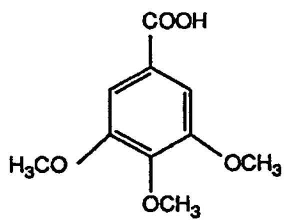
;
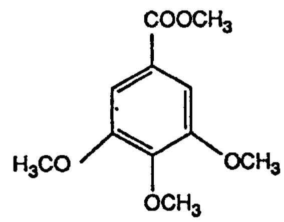
;
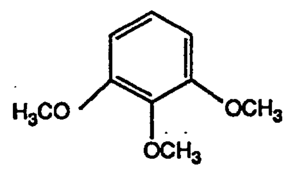
;

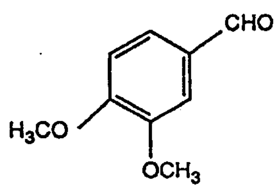
;
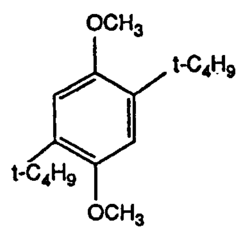
;
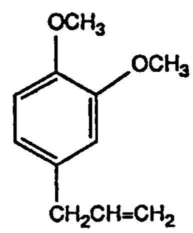
;

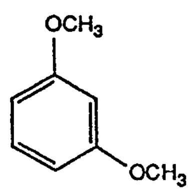
;
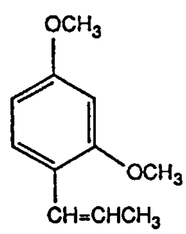
;
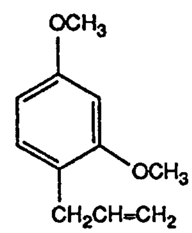
;

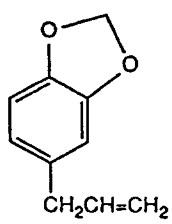
;
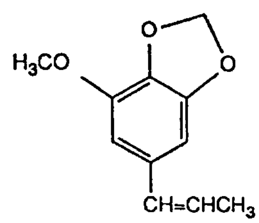
;
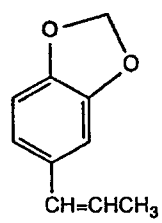
;

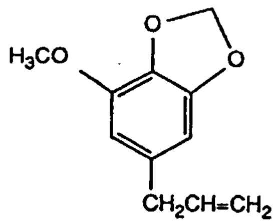 ; 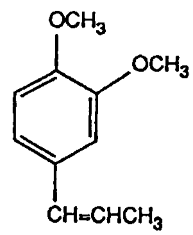 ; 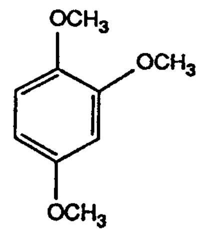 ;

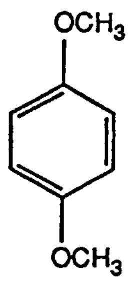 ; 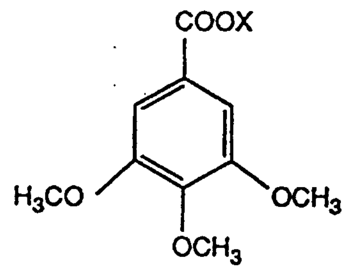 ,

$HN(C_2H_5)_3$ ist,

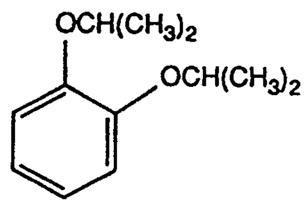

in which X is Na, K, $NH_4$, Ca or $HN(C_2H_5)_3$;
as a stabilizer for inks.

**10.** The use of a compound of the formula I as a stabilizer for inks.

**11.** Use according to claim 10, wherein the ink is used for ink jet printing.

**12.** Use according to claim 10, wherein the ink is used for ink jet printing and additionally contains at least one water-soluble azo dye.

**13.** Use according to claim 9 as a stabilizer for gelatine layers of photographic materials containing a dye or dye precursor.

**Revendications**
**Revendications pour les Etats contractants suivants : BE, DE, FR, GB, IT, NL**

**1.** Encre comportant comme stabilisant au moins un composé de formule I

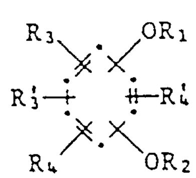 (I),

dans laquelle

$R_1$ et $R_2$, indépendamment l'un de l'autre, représentent un alkyle en $C_1$-$C_4$, qui est éventuellement substitué par 1 ou 2 -OH, $-COO^-M^+$ et/ou $-SO_3^-M^+$, ou ils représentent un alcényle en $C_3$-$C_5$, un alcynyle en $C_3$-$C_5$,

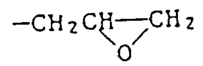,

$-CH_2CH(OH)CH_2$-$SO_3^{\ominus}M^{\oplus}$,
-CO-alkyle en $C_1$-$C_4$, qui est éventuellement substitué avec $-COOR_5$ ou $-CO-N(R_5)(R_6)$, ou, si $OR_1$ et $OR_2$ sont l'un par rapport à l'autre en position ortho, $R_1$ et $R_2$ représentent ensemble un alkylène en $C_1$-$C_6$, où $M^+$ représente $H^+$, un cation métallique mono, bi ou trivalent, ou un groupe $(R_{12}')N^+(R_{12}'')$-$(R_{13}')(R_{14}')$, où $R_{12}'$, $R_{12}''$, $R_{13}'$ et $R_{14}'$, indépendamment l'un de l'autre, représentent H, un alkyle en $C_1$-$C_1$, qui est éventuellement substitué par de 1 à 3 OH, ou qui est interrompu par un O, ou ils représentent l'allyle, le cyclopentyle, le cyclohexyle, le phényle, le benzyle, ou le tolyle, ou $R_1$ peut représenter aussi un groupe

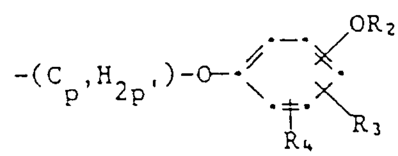

où p' est un nombre de 2 à 6.
$R_5$ et $R_6$, indépendamment l'un de l'autre, représentent H ou un alkyle en $C_1$-$C_4$, qui est éventuellement substitué par un OH, $COOR^o$, $COO^-M^+$, $SO_3^-M^+$ ou $P(O)(OR^o)_2$ ou $-P(O)-(O^-M^+)_2$,
$R'_3$ et $R'_4$, indépendamment l'un de l'autre, représentent H, un alkyle en $C_1$-$C_4$, OH ou un alcoxy en $C_1$-$C_4$,
$R_3$ et $R_4$, indépendamment l'un de l'autre, représentent H, un haloqène, $-OR_7$,
$-COOR^o$, $-COO^-M^+$, $-OOC-R_5$, $-CO-N(R_5)(R_6)$, $-(R_5)N-CO-R_6$, $-CO-R_5$,
$-SO_3^-M^+$, $-SO_2N(R_5)(R_6)$, $-P(OR_5)_3$, $-(O)P-(-OR^o)_2$, $-(O)P-(O^-M^+)_2$, un alkyle en $C_1$-$C_8$ qui est substitué éventuellement par de 1 à 7 $-OR_5$ ou $-OOC-R_5$, avec 1 ou 2 $-COOR^o$, $-COO^-M^+$, $-CO-N(R_5)(R_6)$, ou avec un ou deux $-SO_3^-M^+$,
$-SO_2N(R_5)(R_6)$, $-(O)P-(-OR^o)_2$ ou $-(O)P-(O^-M^+)_2$, ou ils représentent un cycloalkyle en $C_5$-$C_6$ ou l'allyle, où $M^+$, $R_5$ et $R_6$ ont la sionification précédente et $R^o$ représente un alkyle en $C_1$-$C_4$ éventuellement substitué par un -OH, et
$R_7$ représente un alkyle en $C_1$-$C_4$ éventuellement substitué par 1 ou 2 - OH, ou il représente -CO-alkyle en $C_1$-$C_4$ ou
$R_3$ et $R_4$, indépendamment l'un de l'autre, représentent un des groupes des formules II à IV

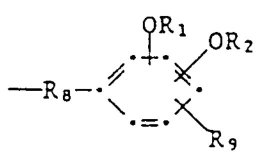 ,
(II)

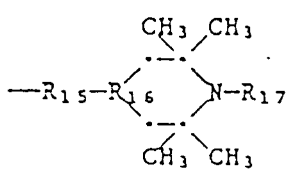 ,
(III)

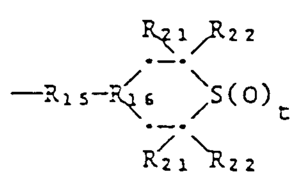 ,
(IV)

où
$R_8$ est une liaison directe ou le méthyléne, $R_9$ représente H, un alkyle en $C_1$-$C_8$, $-COO^-M^+$ ou $-SO_3^-M^+$, où $M^+$ et $R_1$ et $R_2$ ont la signification précédente, $R_{15}$ représente -CO-, $-(-O)_g-C_pH_{2p}-CO-$, $-OOC-C_pH_{2p}-$, $-COO-C_pH_{2p}-$, $-O-C_pH_{2p}-$, $-O-CH_2-CH(OH)-CH_2-$ ou il représente

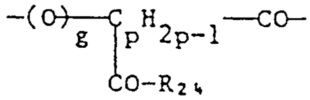
,

où g représente 0 ou 1 et p représente de 1 à 6,
$R_{24}$ représente $-OR_5$, $-N(R_5)(R_6)$ ou un groupe

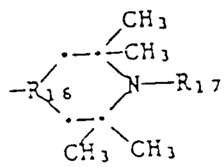

$R_{16}$ représente un des radicaux suivants :
-O-CH< ,

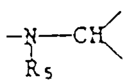 , 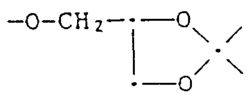 ou 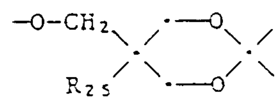 ,

où $R_{25}$ représente H ou un alkyle en $C_1$-$C_4$,
$R_{17}$ représente H, un allcvle en $C_1$-$C_4$ qui est éventuellement substitué par un - OH, ou il représente-$CH_2$-CH(OH)-$CH_2$-OH, un alcoxy en $C_1$-$C_4$, -OH, -CO-alkyle en $C_1$-$C_4$, $-CO-CH=CH_2$, l'allyle, le benzyle ou un groupe

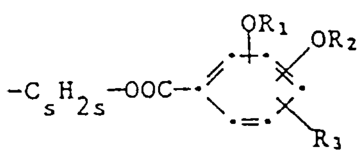

où s représente le nombre 2 ou 3,
t représente un nombre de 0 à 2, et
$R_{21}$ et $R_{22}$, indépendamment l'un de l'autre, représentent H, un alkyle en $C_1$-$C_4$ ou le phényle.

**2.** Encre selon la revendication 1, comportant comme stabilisant au moins un composé de formule I'

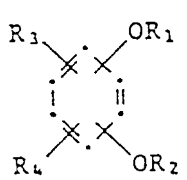

(I'),

où
$R_1$ et $R_2$ indépendamment l'un de l'autre représentent un alkyle en $C_1$-$C_4$, qui est substitué éventuellement par 1 ou 2 -OH, $-COO^-M^+$ et/ou $-SO_3^-M^+$, ou ils représentent $-CH_2CH(OH)-CH_2-SO_3^-M^+$, -CO-alkyle en $C_1$-$C_4$, $M^+$ représente $H^+$, un cation métallique mono- di- ou trivalent, ou un groupe $(R_{12}')N^+(R_{12}'')(R_{13}')(R_{14}')$,
$R_5$ et $R_6$ indépendamment l'un de l'autre représentent H ou un alkyle en $C_1$-$C_4$, qui est substitué éventuellement par un OH,
$R_3$ et $R_4$ indépendamment l'un de l'autre représentent H, un halogène, - $OR_7$, $-COOR^o$, $-OOC-R_5$, $-COO^-M^+$, $-CO-N(R_5)(R_6)$, $-(R_5)N-CO-R_6$, $-CO-R_5$, $-SO_3^-M^+$, $-SO_2N(R_5)(R_6)$, $-P(OR_5)_3$, $-(O)P-(-OR^o)_2$,

$-(O)P-(O^-M^+)_2$, un alkyle en $C_1$-$C_8$ qui éventuellement est substitué par de 1 à 7 $-OR_5$ ou $-OOC-R_5$, avec 1 ou 2 $-COOR^o$, $-COO^-M^+$, -CO-N(R5)($R_6$) ou avec un $-SO_3^-M^+$, $-SO_2N(R_5)(R_6)$, $-(O)P-(-OR^o)_2$, ou $-(O)P-(O^-M^+)_2$ où
$R^o$ représente un alkyle en $C_1$-$C_4$ éventuellement substitué avec un - OH et;
$R_7$ représente un allcyle en $C_1$-$C_4$ éventuellement substitué par 1 ou 2 - OH, ou
$R_3$ et $R_4$ indépendamment l'un de l'autre représentent un groupe de formule II

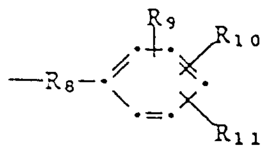 ,

(II)

où
$R_8$ représente une liaison directe ou le méthyléne, $R_9$ représente H, un alkyle en $C_1$-$C_8$, $-COO^-M^+$ ou $-SO_3^-M^+$, où $M^+$ a la signification précédente, et $R_1$ et $R_2$ ont la signification précitée.

**3.** Encre selon la revendication 1, où $R_1$ et $R_2$, indépendamment l'un de l'autre, représentent un alkyle en $C_1$-$C_4$, l'allyle, un hydroxyalkyle en $C_2$-$C_4$, -alkyle en $C_1$-$C_4$-$COO^-M^+$, $-CH_2CH(OH)CH_2-SO_3^-M^+$ ou -CO-alkyle en $C_1$-$C_4$, ou dans laquelle $R_1$ est un groupe de formule

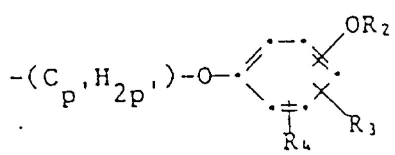

**4.** Encre selon la revendication 3, où $R_1$ et $R_2$, indépendamment l'un de l'autre, représentent le méthyle, l'éthyle, l'allyle, $-CH_2CH_2-OH$, $-CH_2-COO^-M^+$, $-CH_2CH(OH)CH_3$ ou $-CH_2CH(OH)CH_2(OH)$.

**5.** Encre selon la revendication 1, où $R_3$ et R4, indépendamment l'un de l'autre, représentent H, un halogène, $-OR_7$, $-COOR^o$, $-COO^-M^+$, $-CO-N(R_5)(R_6)$, - $SO_3^-M^+$, $-SO_2N(R_5)(R_6)$, un alkyle en $C_1$-$C_8$, qui est éventuellement substitué avec 1 ou 2 $-COOR^o$ ou $-COO^-M^+$, ou ils représentent l'allyle.

**6.** Encre selon la revendication 1, où $R_3$ et/ou $R_4$ représentent le radical - $OR_7$, où $R_7$ représente un alkyle en $C_1$-$C_4$, un hydroxyalkyle en $C_2$-$C_4$ ou $-CH_2-CH(OH)CH_2-OH$.

**7.** Encre selon la revendication 1, où $R_1$ et $R_2$, indépendamment l'un de l'autre, représentent un alkyle en $C_1$-$C_4$ ou un alkyle en $C_1$-$C_4$ substitué par le carboxy, ou $R_1$ et $R_2$ représentent ensemble un alkylène en $C_1$-$C_4$, $R_3$ et $R_4$ indépendamment l'un de l'autre représentent H, , $COO^-M^+$,$-COOR^o$, un alcoxy en $C_1$-$C_4$, alkyle en $C_1$-$C_4$-$COO^-M^+$, alkyle en $C_1$-$C_4$-$COOR^o$, un alkyle en $C_1$-$C_4$ ou $-SO_3^-M^+$ et $R_3'$ et $R_4'$ indépendamment l'un de l'autre représentent H ou un alcoxy en $C_1$-$C_4$.

**8.** Encre selon la revendication 1, où $R_1$ et $R_2$ représentent $CH_3$, $R_3$ représente $-COO^-M^+$ ou $-COOR^o$ et $R_4$ est le méthoxy.

**9.** Composé de formule $I^o$

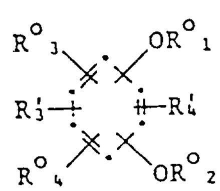

($I^o$),

dans laquelle

$R^o_1$ et $R^o_2$, indépendamment l'un de l'autre, représentent un alkyle en $C_1$-$C_4$ qui est éventuellement substitué par 1 ou 2 -OH, $-COO^-M^+$ et/ou $-SO_3^-M^+$, ou ils représentent un alcényle en $C_3$-$C_5$, un alcynyle en $C_3$-$C_5$,

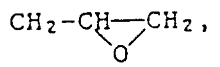

$-CH_2CH(OH)CH_2-SO_3^{\ominus}M^{\oplus}$ ou

-CO-alkyle en $C_1$-$C_4$, qui est éventuellement substitué avec $-COOR_5$ ou - $CO-N(R_5)(R_6)$, ou, si $OR^o_1$ et $OR^o_2$ sont en position ortho l'un par rapport à l'autre, $R^o_1$ et $R^o_2$ ensemble représentent un alkylène en $C_1$-$C_6$, où

$M^+$ représente $H^+$, un ion de métal alcalin, ou un groupe $(R_{12'})N^+(R_{12}'')(R_{13}')(R_{14}')$, où $R_{12}'$, $R_{12}''$, $R_{13}'$ et $R_{14}'$, indépendamment les uns des autres, représentent H, un alkyle en $C_1$-$C_1$, qui est éventuellement substitué avec de 1 à 3 OH, ou qui est interrompu par un O, ou ils représentent l'allyle, le cyclopentyle, le cyclohexyle, le phényle, le benzyle ou le tolyle, ou $R^o_1$ peut aussi représenter un groupe

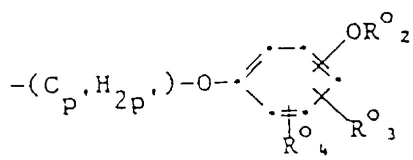

où p' est un nombre de 2 à 6,

$R_5$ et $R_6$, indépendamment l'un de l'autre, représentent H ou un alkyle en $C_1$-$C_4$, qui est éventuellement substitué avec un OH, $COOR^o$, $COO^-M^+$, $SO_3^-M^+$, $P(O)(OR^o)_2$ ou $-P(O)-(O^-M^+)_2$,

$R_3'$ et $R_4'$, indépendamment l'un de l'autre, représentent H, un alkyle en $C_1$-$C_4$, OH ou un alcoxy en $C_1$-$C_4$,

$R^o_3$ et $R^o_4$, indépendamment l'un de l'autre, représentent H, un halogène, $-OR_7$, $-COOR^o$, $-COO^{\ominus}M^{\oplus}$, $OOC-R_5$, $-CO-N(R_5)(R_6)$, $-(R_5)N-CO-R_6$, $-CO-R_5$, $-SO_3^{\ominus}M^{\oplus}$,$-SO_2N(R_5)(R_6)$, $-P(OR_5)_3$, $-(O)P{(OR^o)_2}$, $-(O)-P-(O^{\ominus}M^{\oplus})_2$,

un alkyle en $C_1$-$C_8$, qui est éventuellement substitué avec de 1 à 7 $-OR_5$ ou $-OOC-R_5$, avec 1 ou 2 $-COOR^o$, $-COO^-M^+$, $CO-N(R_5)(R_6)$ ou avec un $-SO_3^-M^+$,

$-SO_2N(R_5)(R_6)$, $-(O)P-(-OR^o)_2$ ou $-(O)P-(O^-M^+)_2$, ou ils représentent l'allyle, un cycloalkyle en $C_5$-$C_6$, où $M^+$, $R_5$ et $R_6$ ont la signification donnée et $R^o$ représente un alkyle en $C_1$-$C_4$, éventuellement substitué avec un -OH, et

$R_7$ est un alkyle en $C_1$-$C_4$, éventuellement substitué avec 1 ou 2 OH, ou il représente -CO-alkyle en $C_1$-$C_4$, ou

$R^o_3$ et $R^o_4$, indépendamment l'un de l'autre, représentent un des groupes des formules II à IV

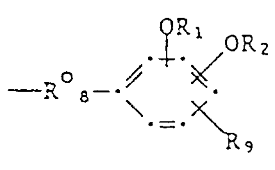

(II°)

,

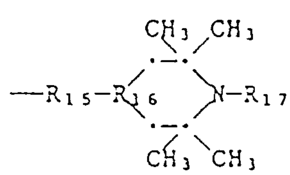

(III)

,

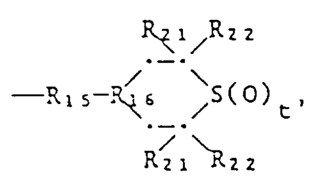

(IV)

où

$R^o_8$ est une liaison directe, ou le méthylène,

$R_9$ représente l'hydrooène: un alkyle en $C_1$-$C_8$, $-COO^-M^+$, ou $-SO_3^-M^+$,

$R_{15}$ représente -CO-,

$-(O)_g-C_pH_{2p}-CO-$,

$-OOC-C_pH_{2p}-$, $-COO-C_pH_{2p}-$, $-O-C_pH_{2p}-$, $-O-CH_2-CH(OH)-CH_2-$ou

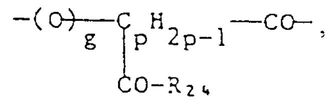

où g représente 0 ou 1 et p représente de 1 à 6,
$R_{24}$ représente $-OR_5$, $-N(R_5)(R_6)$ ou un groupe

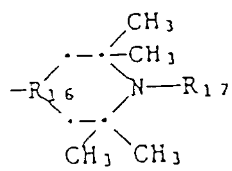

$R_{16}$ représente un des radicaux suivants :
-O-CH< ,

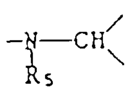 , 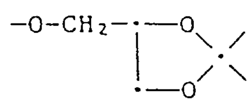 ou 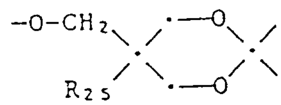 ,

où $R_{25}$ représente H ou un alkyle en $C_1$-$C_4$,
$R_{17}$ représente H, un alkyle en $C_1$-$C_4$ qui est éventuellement substitué avec un - OH, ou il représente $-CH_2-CH(OH)-CH_2-OH$, un alcoxy en $C_1$-$C_8$, -OH, -CO-alkyle en $C_1$-$C_4$, $-CO-CH=CH_2$, l'allyle, le benzyle ou un groupe

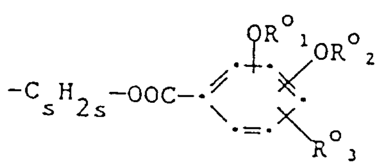

où s représente le nombre 2 ou 3,
t représente un nombre de 0 à 2, et
$R_{21}$ et $R_{22}$, indépendamment l'un de l'autre, représentent H, un alkyle en $C_1$-$C_4$ ou le phényle,
à l'exception des composés suivants :

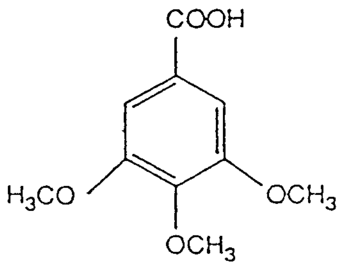 ; 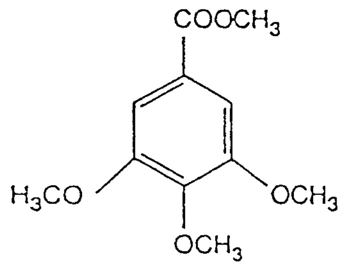 ; 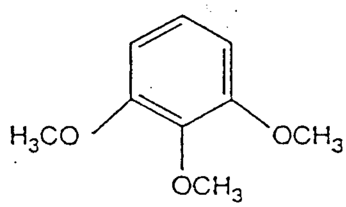 ;

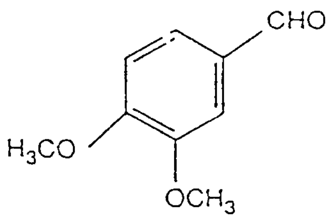 ; 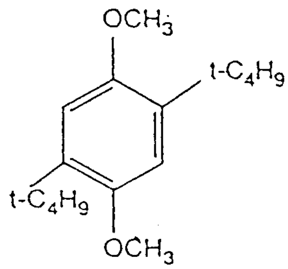 ; 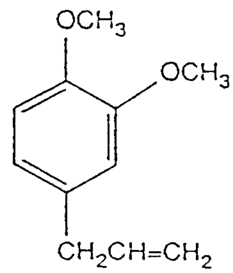 ;

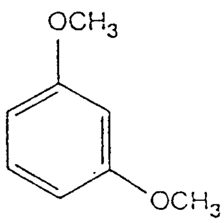 ; 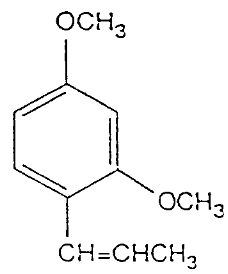 ; 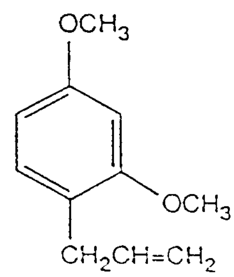 ;

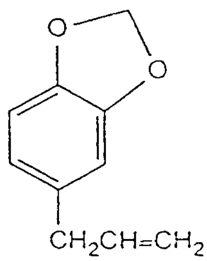 ; 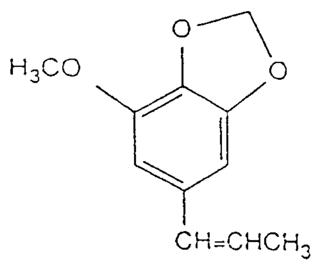 ; 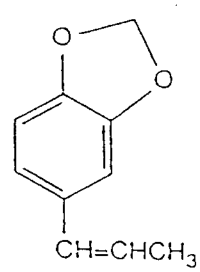 ;

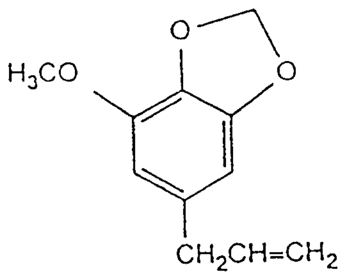 ; 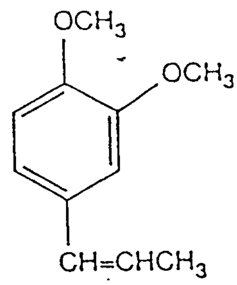 ; 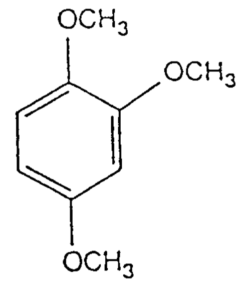 ;

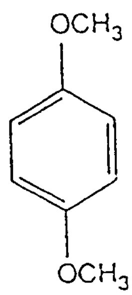 ; 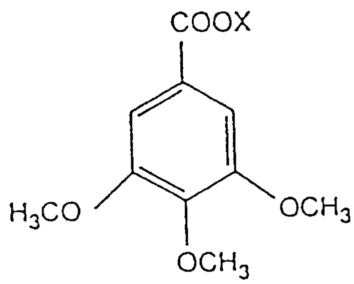 ,

où X est Na, K, $NH_4$,
Ca ou
$HN(C_2H_5)_3$

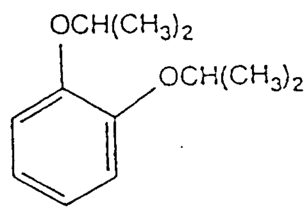 .

**10.** Composé selon la revendication 9, où $R^o_1$ et $R^o_2$, indépendamment l'un de l'autre, représentent un alkyle en $C_1$-$C_4$, qui est substitué avec un - OH, ou ils représentent $-CH_2-CH(OH)CH_2-SO_3^-M^+$, -alkyle en $C_1$-$C_4$-$COO^-M^+$, ou -CO-alkyle en $C_1$-$C_4$, qui est éventuellement substitué par $-COOR^o$, $-COO^-M^+$, $-CO-N(R_5)(R_6)$ ou avec 1 ou 2 OH.

**11.** Composé selon la revendication 9, où $R^o_3$ et/ou $R^o_4$ représentent le radical $-OR_7$, où $R_7$ représente un alkyle en $C_1$-$C_4$, un hydroxyalkyle en $C_2$-$C_4$, ou $-CH_2-CH(OH)CH_2-OH$.

**12.** Composé selon la revendication 9, où $R^o_4$ représente l'hydrogène et $R^o_3$ représente $-SO_3^-M^+$, $-COOR^o$, $-COO^-M^+$ ou $-CO-NHR_5$.

**13.** Utilisation des composés de formule I selon la revendication 13 comme stabilisants pour les encres.

**14.** Utilisation selon la revendication 14, où l'encre est utilisée pour l'impression par jet d'encre.

**15.** Utilisation selon la revendication 14, où l'encre est utilisée pour l'impression par jet d'encre et comporte en outre au moins un colorant azoïque soluble dans l'eau.

**16.** Utilisation selon la revendication 13 comme stabilisant pour les matières pliotographiques à couches de gélatine, qui comportent un colorant ou un précurseur de colorant.

**Revendications pour l'Etat contractant suivant : ES**

1. Encre comportant comme stabilisant au moins un composé de formule I

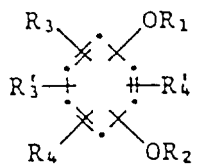

(I),

dans laquelle
$R_1$ et $R_2$, indépendamment l'un de l'autre, représentent un alkyle en $C_1$-$C_4$, qui est éventuellement substitué par 1 ou 2 -OH, $-COO^-M^+$ et/ou $-SO_3{}^-M^+$, ou ils représentent un alcényle en $C_3$-$C_5$, un alcynyle en $C_3$-$C_5$,

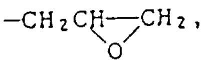

$-CH_2CH(OH)CH_2$-$SO_3{}^{\ominus}M^{\oplus}$,
-CO-alkyle en $C_1$-$C_4$, qui est éventuellement substitué avec $-COOR_5$ ou $-CO-N(R_5)(R_6)$, ou, si $OR_1$ et $OR_2$ sont l'un par rapport à l'autre en position ortho, $R_1$ et $R_2$ représentent ensemble un alkylène en $C_1$-$C_6$, où $M^+$ représente $H^+$, un cation métallique mono, bi ou trivalent, ou un groupe $(R_{12}')N^+(R_{12}'')$-$(R_{13}')(R_{14}')$, où $R_{12}'$, $R_{12}''$, $R_{13}'$ et $R_{14}'$, indépendamment l'un de l'autre, représentent H, un alkyle en $C_1$-$C_1$, qui est éventuellement substitué par de 1 à 3 OH, ou qui est interrompu par un O, ou ils représentent l'allyle, le cyclopentyle, le cyclohexyle, le phényle, le benzyle, ou le tolyle, ou $R_1$ peut représenter aussi un groupe

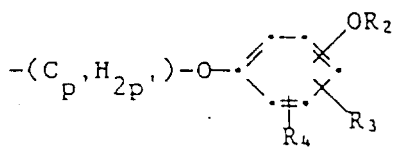

où p' est un nombre de 2 à 6.
$R_5$ et $R_6$, indépendamment l'un de l'autre, représentent H ou un alkyle en $C_1$-$C_4$, qui est éventuellement substitué par un OH, $COOR^o$, $COO^-M^+$, $SO_3{}^-M^+$ ou $P(O)(OR^o)_2$ ou $-P(O)-(O^-M^+)_2$,
$R'_3$ et $R'_4$, indépendamment l'un de l'autre, représentent H, un alkyle en $C_1$-$C_4$, OH ou un alcoxy en $C_1$-$C_4$,
$R_3$ et $R_4$, indépendamment l'un de l'autre, représentent H, un halogène, - $OR_7$, $-COOR^o$, $-COO^-M^+$, $-OOC-R_5$, $-CO-N(R_5)(R_6)$, $-(R_5)N-CO-R_6$, $-CO-R_5$,
$-SO_3{}^-M^+$, $-SO_2N(R_5)(R_6)$, $-P(OR_5)_3$, $-(O)P-(-OR^o)_2$, $-(O)P-(O^-M^+)_2$, un alkyle en $C_1$-$C_8$ qui est substitué éventuellement par de 1 à 7 $-OR_5$ ou $-OOC-R_5$, avec 1 ou 2 $-COOR^o$, $-COO^-M^+$, $-CO-N(R_5)(R_6)$, ou avec un ou deux $-SO_3{}^-M^+$, $-SO_2N(R_5)(R_6)$, $-(O)P-(-OR^o)_2$ ou $-(O)P-(O^-M^+)_2$, ou ils représentent un cycloalkyle en $C_5$-$C_6$ ou l'allyle, où $M^+$, $R_5$ et $R_6$ ont la signification précédente et $R^o$ représente un alkyle en $C_1$-$C_4$ éventuellement substitué par un -OH, et
$R_7$ représente un alkyle en $C_1$-$C_4$ éventuellement substitué par 1 ou 2 - OH, ou il représente -CO-alkyle en $C_1$-$C_4$ ou
$R_3$ et $R_4$, indépendamment l'un de l'autre, représentent un des groupes des formules II à IV

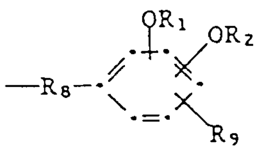 , 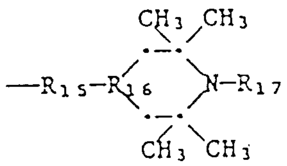 , 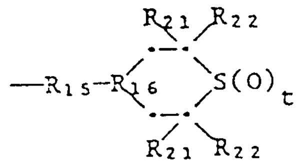 ,

(II) (III) (IV)

où
$R_8$ est une liaison directe ou le méthylène, $R_9$ représente H, un alkyle en $C_1$-$C_8$, $-COO^-M^+$ ou $-SO_3^-M^+$, où $M^+$ et $R_1$ et $R_2$ ont la signifcation précédente, $R_{15}$ représente -CO-, $-(-O)_g-C_pH_{2p}-CO$, $-OOC-C_pH_{2p}-$, $-COO-C_pH_{2p}-$, $-O-C_pH_{2p}-$, $-O-CH_2-CH(OH)-CH_2-$ ou
il représente

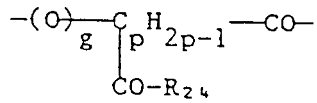 ,

où g représente 0 ou 1 et p représente de 1 à 6,
$R_{24}$ représente $-OR_5$, $-N(R_5)(R_6)$ ou un groupe

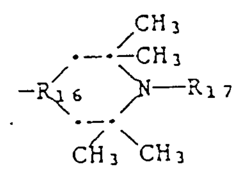

$R_{16}$ représente un des radicaux suivants :
-O-CH< ,

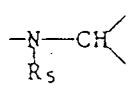 , 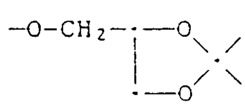 ou 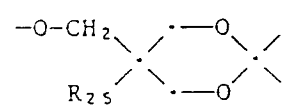 ,

où $R_{25}$ représente H ou un alkyle en $C_1$-$C_4$,
$R_{17}$ représente H, un alkyle en $C_1$-$C_4$ qui est éventuellement substitué par un - OH, ou il représente-$CH_2-CH(OH)-CH_2-OH$, un alcoxy en $C_1$-$C_8$, -OH, -CO-alkyle en $C_1$-$C_4$, $-CO-CH=CH_2$, l'allyle, le benzyle ou un groupe

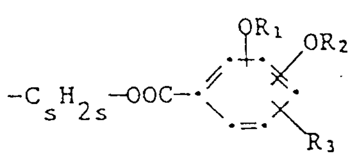

où s représente le nombre 2 ou 3,
t représente un nombre de 0 à 2, et
$R_{21}$ et $R_{22}$, indépendamment l'un de l'autre, représentent H, un alkyle en $C_1$-$C_4$ ou le phényle.

2. Encre selon la revendication 1, comportant comme stabilisant au moins un composé de formule I'

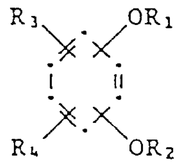

(I'),

où

$R_1$ et $R_2$ indépendamment l'un de l'autre représentent un alkyle en $C_1$-$C_4$, qui est substitué éventuellement par 1 ou 2 -OH, $-COO^-M^+$ et/ou $-SO_3^-M^+$, ou ils représentent $-CH_2CH(OH)-CH_2-SO_3^-M^+$, -CO-alkyle en $C_1$-$C_4$, $M^+$ représente $H^+$, un cation métallique mono- di- ou trivalent, ou un groupe $(R_{12}')N^+(R_{12}'')(R_{13}')(R_{14}')$,

$R_5$ et $R_6$ indépendamment l'un de l'autre représentent H ou un alkyle en $C_1$-$C_4$, qui est substitué éventuellement par un OH,

$R_3$ et $R_4$ indépendamment l'un de l'autre représentent H, un halogène - $OR_7$, $-COOR^o$, $-OOC-R_5$, $-COO^-M^+$, $-CO-N(R_5)(R_6)$, $-(R_5)N-CO-R_6$, $-CO-R_5$, $-SO_3^-M^+$, $-SO_2N(R_5)(R_6)$, $-P(OR_5)_3$, $-(O)P-(-OR^o)_2$, $-(O)P-(O^-M^+)_2$, un alkyle en $C_1$-$C_8$ qui éventuellement est substitué par de 1 à 7 $-OR_5$ ou $-OOC-R_5$, avec 1 ou 2 $-COOR^o$, $-COO^-M^+$, $-CO-N(R5)(R_6)$ ou avec un $-SO_3^-M^+$, $-SO_2N(R_5)(R_6)$, $-(O)P-(-OR^o)_2$, ou $-(O)P-(O^-M^+)_2$ où

$R^o$ représente un alkyle en $C_1$-$C_4$ éventuellement substitué avec un - OH et, $R_7$ représente un alkyle en $C_1$-$C_4$ éventuellement substitué par 1 ou 2 - OH, ou $R_3$ et $R_4$ indépendamment l'un de l'autre représentent un groupe de formule II

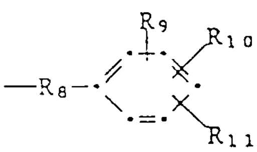

, (II)

où

$R_8$ représente une liaison directe ou le méthylène, $R_9$ représente H, un alkyle en $C_1$-$C_8$, $-COO^-M^+$ ou $-SO_3^-M^+$, où $M^+$ a la signification précédente, et $R_1$ et $R_2$ ont la signification précitée.

3. Encre selon la revendication 1, où $R_1$ et $R_2$, indépendamment l'un de l'autre, représentent un alkyle en $C_1$-$C_4$, l'allyle, un hydroxyalkyle en $C_2$-$C_4$, -alkyle en $C_1$-$C_4$-$COO^-M^+$, $-CH_2CH(OH)CH_2-SO_3^-M^+$ ou -CO-alkyle en $C_1$-$C_4$, ou dans laquelle $R_1$ est un groupe de formule

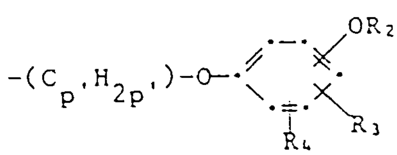

4. Encre selon la revendication 3, où $R_1$ et $R_2$, indépendamment l'un de l'autre, représentent le méthyle, l'éthyle, l'allyle, $-CH_2CH_2-OH$, $-CH_2-COO^-M^+$, $-CH_2CH(OH)CH_3$ ou $-CH_2CH(OH)CH_2(OH)$.

5. Encre selon la revendication 1, où $R_3$ et $R_4$, indépendamment l'un de l'autre, représentent H, un halogène, $-OR_7$, $-COOR^o$, $-COO^-M^+$, $-CO-N(R_5)(R_6)$, - $SO_3^-M^+$, $-SO_2N(R_5)(R_6)$, un alkyle en $C_1$-$C_8$, qui est éventuellement substitué avec 1 ou 2 $-COOR^o$ ou $-COO^-M^+$, ou ils représentent l'allyle.

6. Encre selon la revendication 1, où $R_3$ et/ou $R_4$ représentent le radical - $OR_7$, où $R_7$ représente un alkyle en $C_1$-$C_4$, un hydroxyalkyle en $C_2$-$C_4$ ou $-CH_2-CH(OH)CH_2-OH$.

**7.** Encre selon la revendication 1, où $R_1$ et $R_2$, indépendamment l'un de l'autre, représentent un alkyle en $C_1$-$C_4$ ou un alkyle en $C_1$-$C_4$ substitué par le carboxy, ou $R_1$ et $R_2$ représentent ensemble un alkylène en $C_1$-$C_4$, $R_3$ et $R_4$ indépendamment l'un de l'autre représentent H, , $COO^-M^+$,$-COOR^o$, un alcoxy en $C_1$-$C_4$, alkyle en $C_1$-$C_4$-$COO^-M^+$, alkyle en $C_1$-$C_4$-$COOR^o$, un alkyle en $C_1$-$C_4$ ou $-SO_3^-M^+$ et $R_3$' et $R_4$' indépendamment l'un de l'autre représentent H ou un alcoxy en $C_1$-$C_4$.

**8.** Encre selon la revendication 1, où $R_1$ et $R_2$ représentent $CH_3$, $R_3$ représente $-COO^-M^+$ ou $-COOR^o$ et $R_4$ est le méthoxy.

**9.** Utilisation des composés de formule I°

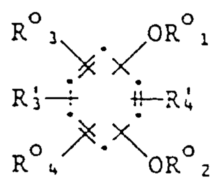

(I°),

dans laquelle

$R^o_1$ et $R^o_2$, indépendamment l'un de l'autre, représentent un alkyle en $C_1$-$C_4$ qui est éventuellement substitué par 1 ou 2 -OH, $-COO^-M^+$ et/ou $-SO_3^-M^+$, ou ils représentent un alcényle en $C_3$-$C_5$, un alcynyle en $C_3$-$C_5$,

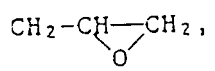

$-CH_2CH(OH)CH_2$-$SO_3^{\ominus}M^{\oplus}$ ou

-CO-alkyle en $C_1$-$C_4$, qui est éventuellement substitué avec $-COOR_5$ ou - CO-N($R_5$)($R_6$), ou, si $OR^o_1$ et $OR^o_2$ sont en position ortho l'un par rapport à l'autre, $R^o_1$ et $R^o_2$ ensemble représentent un alkylène en $C_1$-$C_6$, où

$M^+$ représente $H^+$, un ion de métal alcalin, ou un groupe

($R_{12'}$)$N^+$($R_{12}$'')($R_{13}$')($R_{14}$'), où $R_{12}$', $R_{12}$'', $R_{13}$' et $R_{14}$', indépendamment les uns des autres, représentent H, un alkyle en $C_1$-$C_1$, qui est éventuellement substitué avec de 1 à 3 OH, ou qui est interrompu par un O, ou ils représentent l'allyle, le cyclopentyle, le cyclohexyle, le phényle, le benzyle ou le tolyle, ou $R^o_1$ peut aussi représenter un groupe

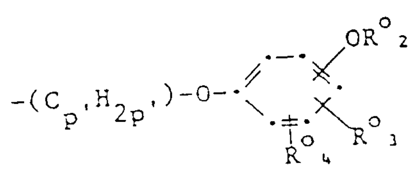

où p' est un nombre de 2 à 6,

$R_5$ et $R_6$, indépendamment l'un de l'autre, représentent H ou un alkyle en $C_1$-$C_4$, qui est éventuellement substitué avec un OH, $COOR^o$, $COO^-M^+$, $SO_3^-M^+$, $P(O)(OR^o)_2$ ou $-P(O)-(O^-M^+)_2$,

$R_3$' et $R_4$', indépendamment l'un de l'autre, représentent H, un alkyle en $C_1$-$C_4$, OH ou un alcoxy en $C_1$-$C_4$,

$R^o_3$ et $R^o_4$, indépendamment l'un de l'autre, représentent H, un halogène, $-OR_7$, $-COOR^o$, $-COO^{\ominus}M^{\oplus}$, OOC-$R_5$, -CO-N($R_5$)($R_6$), -($R_5$)N-CO-$R_6$, -CO-$R_5$, $-SO_3^{\ominus}M^{\oplus}$,$-SO_2N(R_5)(R_6)$, $-P(OR_5)_3$, $-(O)P(OR^o)_2$, -(O)-P-$(O^{\ominus}M^{\oplus})_2$,

un alkyle en $C_1$-$C_8$, qui est éventuellement substitué avec de 1 à 7 $-OR_5$ ou -OOC-$R_5$, avec 1 ou 2 $-COOR^o$, $-COO^-M^+$, CO-N($R_5$)($R_6$) ou avec un $-SO_3^-M^+$, $-SO_2N(R_5)(R_6)$, $-(O)P-(-OR^o)_2$ ou -(O)P-$(O^-M^+)_2$, ou ils représentent l'allyle, un cycloalkyle en $C_5$-$C_6$, où $M^+$, $R_5$ et $R_6$ ont la signification donnée et $R^o$ représente un alkyle en $C_1$-$C_4$, éventuellement substitué avec un -OH, et $R_7$ est un alkyle

en $C_1$-$C_4$, éventuellement substitué avec 1 ou 2 OH, ou il représente -CO-alkyle en $C_1$-$C_4$, ou $R^o_3$ et $R^o_4$, indépendamment l'un de l'autre, représentent un des groupes des formules II à IV

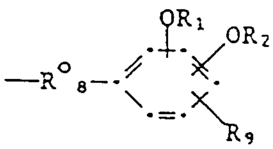 ,

(II°)

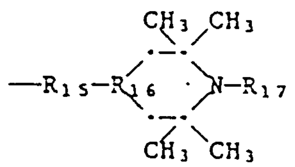 ,

(III)

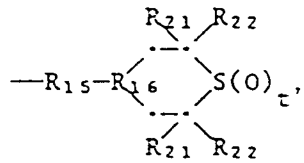

(IV)

où

$R^o_8$ est une liaison directe, ou le méthylène,

$R_9$ représente l'hydrogéne, un alkyle en $C_1$-$C_8$, $-COO^-M^+$, ou $-SO_3^-M^+$,

$R_{15}$ représente -CO-,

$$-(O)_g-C_pH_{2p}-CO-,$$

$-OOC-C_pH_{2p}-$, $-COO-C_pH_{2p}-$, $-O-C_pH_{2p}-$, $-O-CH_2-CH(OH)-CH_2-$ou

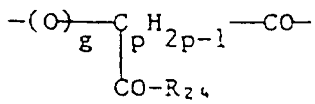 ,

où g représente 0 ou 1 et p représente de 1 à 6,

$R_{24}$ représente $-OR_5$, $-N(R_5)(R_6)$ ou un groupe

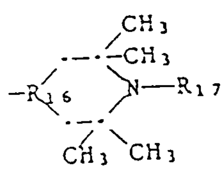

$R_{16}$ représente un des radicaux suivants :

-O-CH< ,

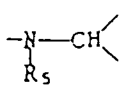 , 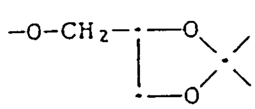 ou 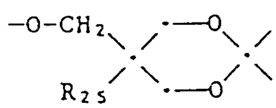 ,

où $R_{25}$ représente H ou un alkyle en $C_1$-$C_4$,

$R_{17}$ représente H, un alkyle en $C_1$-$C_4$ qui est éventuellement substitué avec un - OH, ou il représente $-CH_2-CH(OH)-CH_2-OH$, un alcoxy en $C_1$-$C_8$, -OH, -CO-alkyle en $C_1$-$C_4$, $-CO-CH=CH_2$, l'allyle, le benzyle ou un groupe

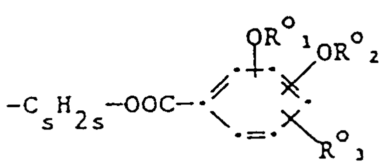

où s représente le nombre 2 ou 3,
t représente un nombre de 0 à 2, et
$R_{21}$ et $R_{22}$, indépendamment l'un de l'autre, représentent H, un alkyle en $C_1$-$C_4$ ou le phényle,
à l'exception des composés suivants :

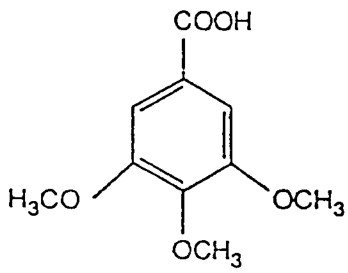 ; 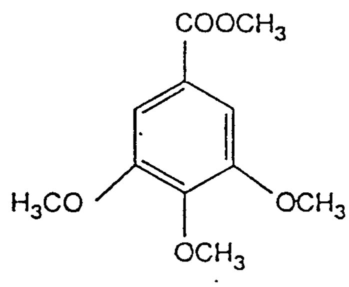 ; 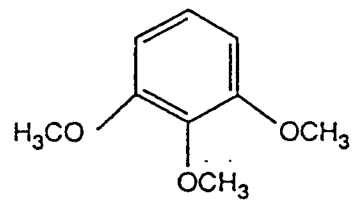 ;

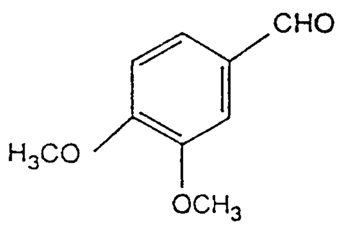 ; 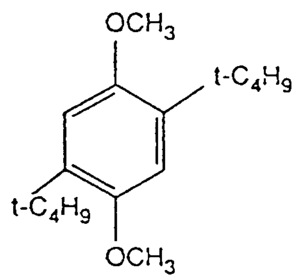 ; 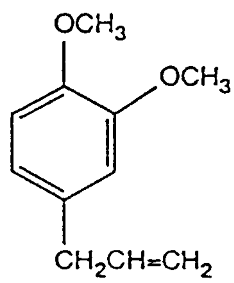 ;

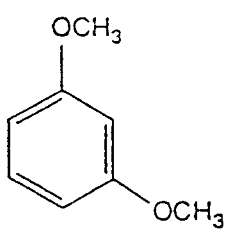 ; 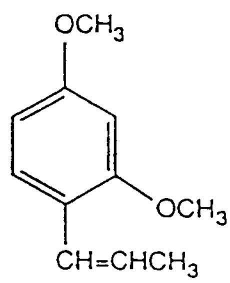 ; 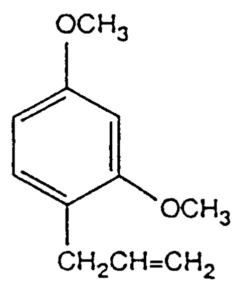 ;

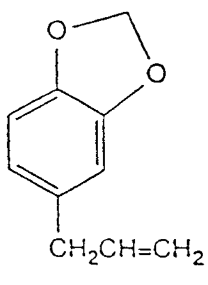 ; 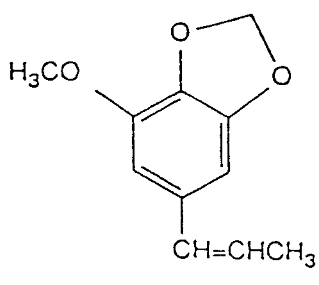 ; 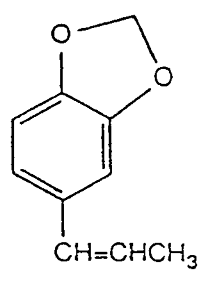 ;

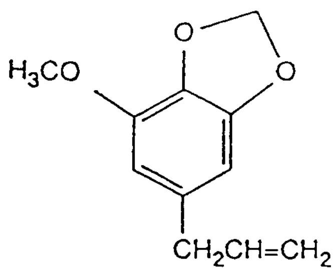

;

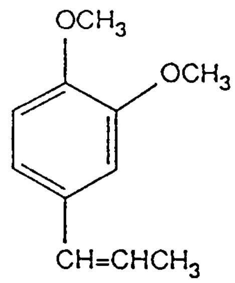

;

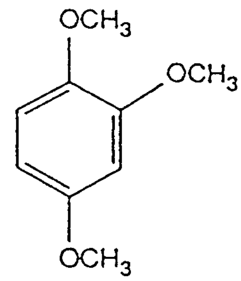

;

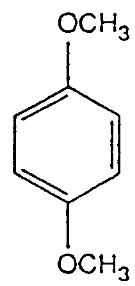

;

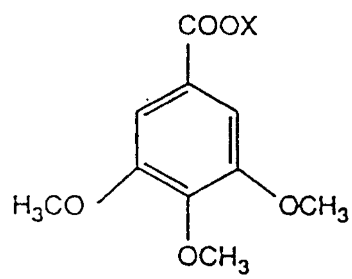

,

où X est Na, K $NH_4$, Ca ou $HN(C_2H_5)_3$

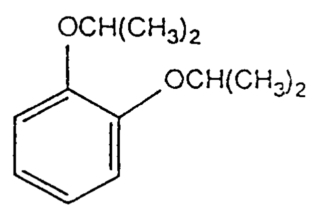

.

comme stabilisants pour les encres.

**10.** Utilisation des composés de formule I comme stabilisants pour les encres.

**11.** Utilisation selon la revendication 10, où l'encre est utilisée pour l'impression par jet d'encre.

**12.** Utilisation selon la revendication 10, où l'encre est utilisée pour l'impression par jet d'encre et comporte en outre au moins un colorant azoïque soluble dans l'eau.

**13.** Utilisation selon la revendication 9 comme stabilisant pour les matières photographiques à couches de gélatine, qui comportent un colorant ou un précurseur de colorant.